(19) Europäisches Patentamt
European Patent Office
Office européen des brevets

(11) EP 3 522 243 A1

(12) **EUROPEAN PATENT APPLICATION**
published in accordance with Art. 153(4) EPC

(43) Date of publication:
**07.08.2019 Bulletin 2019/32**

(21) Application number: **17855777.3**

(22) Date of filing: **15.09.2017**

(51) Int Cl.:
***H01L 51/30*** *(2006.01)* ***H01L 21/336*** *(2006.01)*
***H01L 29/786*** *(2006.01)* ***H01L 51/05*** *(2006.01)*
***H01L 51/40*** *(2006.01)*

(86) International application number:
**PCT/JP2017/033409**

(87) International publication number:
**WO 2018/061821 (05.04.2018 Gazette 2018/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.09.2016 JP 2016191915**

(71) Applicant: **FUJIFILM Corporation**
**Tokyo 106-8620 (JP)**

(72) Inventors:
- **KARIYA, Toshihiro**
**Ashigarakami-gun**
**Kanagawa 258-8577 (JP)**
- **FUKUZAKI, Eiji**
**Ashigarakami-gun**
**Kanagawa 258-8577 (JP)**
- **GOTO, Takashi**
**Ashigarakami-gun**
**Kanagawa 258-8577 (JP)**
- **WATANABE, Tetsuya**
**Ashigarakami-gun**
**Kanagawa 258-8577 (JP)**

(74) Representative: **Hoffmann Eitle**
**Patent- und Rechtsanwälte PartmbB**
**Arabellastraße 30**
**81925 München (DE)**

(54) **COMPOSITION FOR FORMING ORGANIC SEMICONDUCTOR FILM, ORGANIC SEMICONDUCTOR FILM AND METHOD FOR PRODUCING SAME, AND ORGANIC SEMICONDUCTOR ELEMENT**

(57) Provided are an organic semiconductor film-forming composition, an organic semiconductor film formed of the organic semiconductor film-forming composition, an organic semiconductor element, and a method of forming an organic semiconductor film. The organic semiconductor film-forming composition includes at least two organic semiconductors, in which one organic semiconductor is a low molecular weight organic semiconductor compound having a molecular weight of 5000 or lower, and another organic semiconductor is a fused heterocyclic compound represented by the specific Formula (1) that has a chemical structure different from that of the low molecular weight organic semiconductor compound and has a molecular weight of 3000 or lower.

EP 3 522 243 A1

FIG. 1

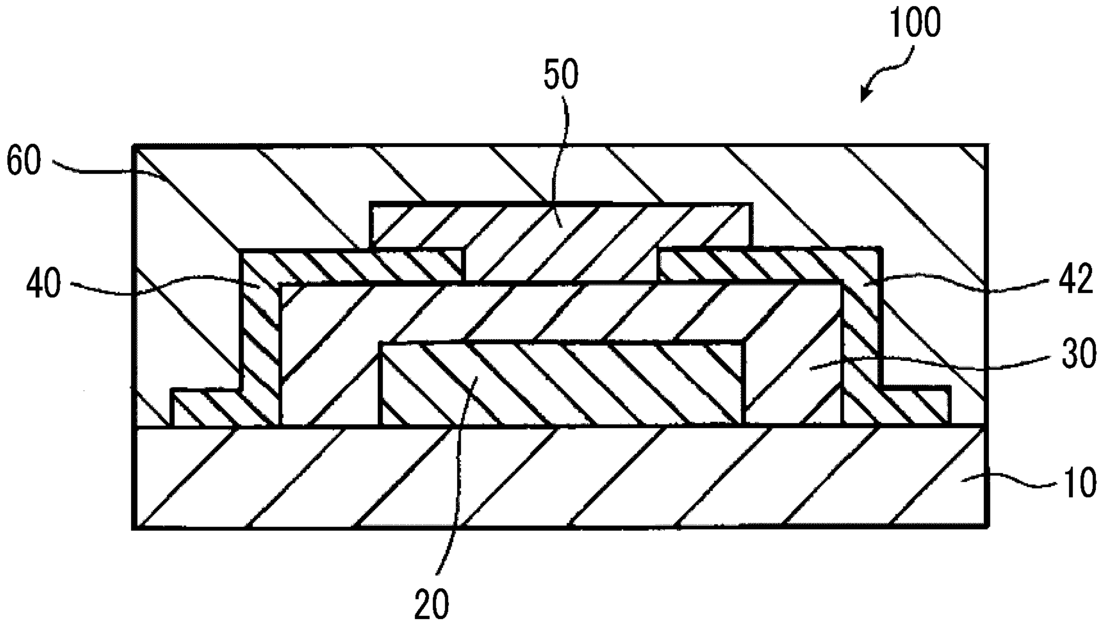

## Description

## BACKGROUND OF THE INVENTION

### 1. Field of the Invention

The present invention relates to an organic semiconductor film-forming composition, an organic semiconductor film, and a method of forming an organic semiconductor film, and an organic semiconductor element.

### 2. Description of the Background Art

In a display such as a liquid crystal display or an organic electroluminescence display, a device using a logical circuit such as a radio frequency identifier (RFID: RF tag) or a memory, a solar cell, or the like, an organic semiconductor element such as an organic thin film transistor is used. In particular, an organic semiconductor element including an organic semiconductor layer (also referred to as "organic semiconductor film") can be reduced in weight, and a printing method can be used for manufacturing the organic semiconductor element. Therefore, this organic semiconductor element can realize cost reduction and also has excellent flexibility. Thus, the organic semiconductor element has attracted attention as the next-generation semiconductor element that is an alternative to an inorganic semiconductor element including a silicon semiconductor layer, and development thereof has progressed.

In order to improve the performance of the organic thin film transistor, the improvement of carrier mobility is an important factor. Therefore, in order to improve the carrier mobility, an organic semiconductor layer or an organic semiconductor including an organic semiconductor layer has been considered.

For example, JP2015-195361A describes an organic transistor including an organic semiconductor layer that includes a compound having a thienobenzothiophene structure. In addition, JP2015-195362A describes an organic transistor including an organic semiconductor that includes a specific compound having a fused ring structure including 5 rings. Further, JP5089986B describes an organic transistor that includes an organic semiconductor layer including: an organic binder resin having a dielectric constant of 3.3 or lower at 1000 Hz; and a polyacene represented by specific Formula A. JP4545373B describes an organic semiconductor element including an organic semiconductor thin layer in which at least one of a conjugated polymer compound or an amorphous low molecular weight compound as an organic semiconductor component is interposed between crystal grains of a crystalline low molecular weight organic semiconductor compound.

## SUMMARY OF THE INVENTION

However, recently, further improvement of carrier mobility has been required along with an increase in the resolution of a display or an increase in the speed of a logical circuit.

As described above, typically, the organic semiconductor layer of the organic semiconductor element can be formed using a printing method. However, as a result of investigation, the present inventors found that it is difficult to form the organic semiconductor layer including the organic binder resin or the conjugated polymer compound using a printing method, in particular, an ink jet method and, even in a case where the organic semiconductor layer is formed using a printing method, a sufficient carrier mobility is not exhibited. Further, the present inventors also found that the organic binder resin or the conjugated polymer compound is likely to enclose a solvent used for forming the organic semiconductor layer (is likely to remain in the organic semiconductor layer), and hysteresis of a threshold voltage increases in an organic semiconductor element including the organic semiconductor layer that encloses the solvent.

An object of the present invention is to provide an organic semiconductor film-forming composition that is used for forming an organic semiconductor film of an organic semiconductor element such that a carrier mobility of the formed organic semiconductor element can be improved and hysteresis can also be reduced. In addition, another object of the present invention is to provide an organic semiconductor film formed of the organic semiconductor film-forming composition and a method of forming the organic semiconductor film. Further, still another object of the present invention is to provide an organic semiconductor element having a high carrier mobility and a small hysteresis.

The present inventors repeatedly conducted a thorough investigation in consideration of the above-described objects and found that, in a case where an organic semiconductor film of an organic semiconductor element is formed with a printing method by using a low molecular weight organic semiconductor compound having a molecular weight of 5000 or lower and a fused heterocyclic compound represented by the following Formula (1) that has a chemical structure different from that of the low molecular weight organic semiconductor compound and has a molecular weight of 3000 or lower in combination, the carrier mobility of the obtained organic semiconductor element can be improved, and hysteresis can be reduced. The present invention has been completed based on the above findings as a result of repeated investigation.

The object of the present invention is achieved by the following means.

<1> An organic semiconductor film-forming composition comprising:

at least two organic semiconductors, in which one organic semiconductor is a low molecular weight organic semiconductor compound having a molecular weight of 5000 or lower, and another organic semiconductor is a fused heterocyclic compound represented by the following Formula (1) that has a chemical structure different from that of the low molecular weight organic semiconductor compound and has a molecular weight of 3000 or lower,

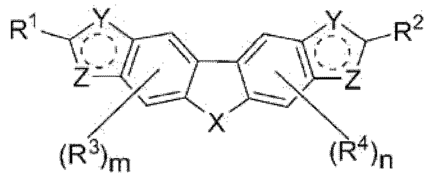

Formula (1)

in Formula (1),

X represents an oxygen atom, a sulfur atom, a selenium atom, a tellurium atom, or $NR^5$,
Y and Z each independently represent $CR^6$, an oxygen atom, a sulfur atom, a selenium atom, a nitrogen atom, or $NR^7$,
a 5-membered ring including Y and Z is an aromatic heterocycle,
$R^1$ and $R^2$ in Formula (1) are bonded to a ring-constituting atom of the 5-membered ring including Y and Z directly or indirectly through a divalent group A,
$R^3$ and $R^4$ in Formula (1) are bonded to a ring-constituting atom of a benzene ring directly or indirectly through the divalent group A,
the divalent group A is a group selected from -O-, -S-, $-NR^8-$, -CO-, -SO-, or $-SO_2-$ or is a group in which two or more selected from -O-, -S-, $-NR^8-$, -CO-, -SO-, or $-SO_2-$ are linked to each other,
$R^1$, $R^2$, $R^5$, $R^6$, $R^7$, and $R^8$ each independently represent a hydrogen atom, an alkyl group, an alkenyl group, an alkynyl group, an aryl group, or a heteroaryl group,
$R^3$ and $R^4$ each independently represent a halogen atom, an alkyl group, an alkenyl group, an alkynyl group, an aryl group, or a heteroaryl group, and
m and n each independently represent an integer of 0 to 2.

<2> The organic semiconductor film-forming composition according to <1>,
in which the 5-membered ring including Y and Z is a ring selected from a thiophene ring, a furan ring, a selenophene ring, a pyrrole ring, a thiazole ring, a selenazole ring, an imidazole ring, or an oxazole ring.
<3> The organic semiconductor film-forming composition according to <1> or <2>,
in which the number of carbon atoms in each of $R^1$, $R^2$, $R^3$, and $R^4$ is 30 or less.
<4> The organic semiconductor film-forming composition according to any one of <1> to <3>,
in which both m and n represent 0.
<5> The organic semiconductor film-forming composition according to any one of <1> to <4>,
in which $R^1$ and $R^2$ each independently represent an alkyl group having 20 or less carbon atoms, an aryl group having 20 or less carbon atoms, or a heteroaryl group having 20 or less carbon atoms.
<6> The organic semiconductor film-forming composition according to any one of <1> to <5>,
in which $R^1$ and $R^2$ are the same as each other,
$R^3$ and $R^4$ are the same as each other, and
m and n are the same as each other.
<7> The organic semiconductor film-forming composition according to <1>,
wherein the compound represented by Formula (1) is a compound represented by the following Formula (2) or (3),

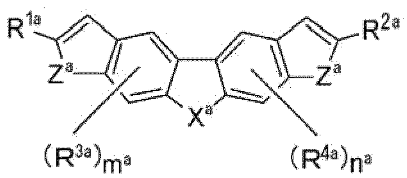

Formula (2)

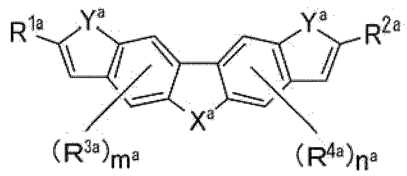

Formula (3)

in Formulae (2) and (3),

$X^a$ represents an oxygen atom, a sulfur atom, or a selenium atom,
$Y^a$ and $Z^a$ each independently represent an oxygen atom, a sulfur atom, a selenium atom, or $NR^{7a}$,
$R^{7a}$ has the same definition as $R^7$ in Formula (1),
$R^{1a}$, $R^{2a}$, $R^{3a}$, $R^{4a}$, $m^a$, and $n^a$ have the same definitions as $R^1$, $R^2$, $R^3$, $R^4$, m, and n in Formula (1), respectively, and
binding forms of $R^{1a}$, $R^{2a}$, $R^{3a}$, and $R^{4a}$ to a ring-constituting atom are also the same as binding forms of $R^1$, $R^2$, $R^3$, and $R^4$ in Formula (1) to a ring-constituting atom, respectively.

<8> The organic semiconductor film-forming composition according to <7>,
in which the compound represented by Formula (2) is represented by the following Formula (4), and
the compound represented by Formula (3) is represented by the following Formula (5),

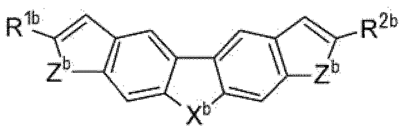

Formula (4)

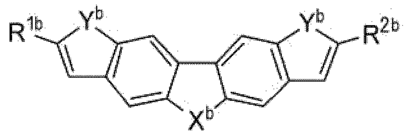

Formula (5)

in Formulae (4) and (5),

$X^b$, $Y^b$, and $Z^b$ each independently represent an oxygen atom, a sulfur atom, or a selenium atom,
$R^{1b}$ and $R^{2b}$ have the same definitions as $R^{1a}$ and $R^{2a}$ in Formula (2), respectively, and
binding forms of $R^{1b}$ and $R^{2b}$ to a ring-constituting atom are also the same as binding forms of $R^{1a}$ and $R^{2a}$ in Formula (2) to a ring-constituting atom, respectively.

<9> The organic semiconductor film-forming composition according to <8>,
in which $R^{1b}$ and $R^{2b}$ have an aliphatic hydrocarbon group.
<10> The organic semiconductor film-forming composition according to <8> or <9>,
in which $R^{1b}$ and $R^{2b}$ each independently represent an aryl group having a linear aliphatic hydrocarbon group or a heteroaryl group having a linear aliphatic hydrocarbon group.
<11> The organic semiconductor film-forming composition according to any one of claims 1 to 10,
wherein the low molecular weight organic semiconductor compound is a compound having the same mother nucleus as the fused heterocyclic compound and having a substituent different from that of the fused heterocyclic compound.
<12> The organic semiconductor film-forming composition according to <11>,
in which a total molecular weight of the substituent included in the low molecular weight organic semiconductor compound is lower than a total molecular weight of the substituent included in the fused heterocyclic compound.
<13> The organic semiconductor film-forming composition according to any one of <1> to <12>,
in which a content of the fused heterocyclic compound is 20 mass% or lower with respect to a total mass of the organic semiconductors in the organic semiconductor film-forming composition.
<14> The organic semiconductor film-forming composition according to any one of <1> to <13>, further comprising: at least one solvent having a boiling point of 180°C or higher and a solubility parameter of 17.0 to 23.0 $MPa^{1/2}$.
<15> The organic semiconductor film-forming composition according to <14>,

in which the solvent has a naphthalene skeleton.

<16> The organic semiconductor film-forming composition according to <15>,

in which a content of the solvent having a naphthalene skeleton is 50 mass% or higher.

<17> A method of forming an organic semiconductor film comprising:

a step of applying the organic semiconductor film-forming composition according to any one of <1> to <16> to a substrate.

<18> An organic semiconductor film comprising:

at least two organic semiconductors,

in which one organic semiconductor is a low molecular weight organic semiconductor compound having a molecular weight of 5000 or lower, and

another organic semiconductor is a fused heterocyclic compound represented by the following Formula (1) that has a chemical structure different from that of the low molecular weight organic semiconductor compound and has a molecular weight of 3000 or lower,

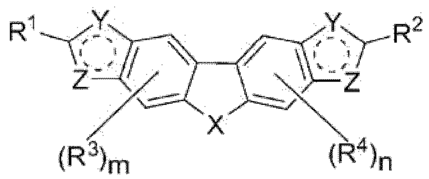

Formula (1)

in Formula (1),

X represents an oxygen atom, a sulfur atom, a selenium atom, a tellurium atom, or $NR^5$,

Y and Z each independently represent $CR^6$, an oxygen atom, a sulfur atom, a selenium atom, a nitrogen atom, or $NR^7$,

a 5-membered ring including Y and Z is an aromatic heterocycle,

$R^1$ and $R^2$ in Formula (1) are bonded to a ring-constituting atom of the 5-membered ring including Y and Z directly or indirectly through a divalent group A,

$R^3$ and $R^4$ in Formula (1) are bonded to a ring-constituting atom of a benzene ring directly or indirectly through the divalent group A,

the divalent group A is a group selected from -O-, -S-, $-NR^8-$, -CO-, -SO-, or $-SO_2-$ or is a group in which two or more selected from -O-, -S-, $-NR^8-$, -CO-, -SO-, or $-SO_2-$ are linked to each other,

$R^1$, $R^2$, $R^5$, $R^6$, $R^7$, and $R^8$ each independently represent a hydrogen atom, an alkyl group, an alkenyl group, an alkynyl group, an aryl group, or a heteroaryl group,

$R^3$ and $R^4$ each independently represent a halogen atom, an alkyl group, an alkenyl group, an alkynyl group, an aryl group, or a heteroaryl group, and

m and n each independently represent an integer of 0 to 2.

<19> An organic semiconductor element comprising:

the organic semiconductor film according to <18>.

By using the organic semiconductor film-forming composition according to the present invention for forming an organic semiconductor film of an organic semiconductor element, a carrier mobility of the formed organic semiconductor element can be improved and hysteresis can also be reduced. In addition, by using the organic semiconductor film according to the present invention as an organic semiconductor film of an organic semiconductor element, a carrier mobility of the formed organic semiconductor element can be improved and hysteresis can also be reduced. Further, with the method of forming an organic semiconductor film according to the present invention, an organic semiconductor film having the above-described excellent properties can be formed. The organic semiconductor element according to the present invention exhibits a high carrier mobility and a small hysteresis.

The above-described and other characteristics and advantageous effects of the present invention will be clarified from the following description appropriately with reference to the accompanying drawings.

## BRIEF DESCRIPTION OF THE DRAWINGS

Fig. 1 is a schematic cross-sectional view showing one aspect of a bottom gate-bottom contact type organic thin film transistor as an example of an organic semiconductor element according to the present invention.
Fig. 2 is a schematic cross-sectional view showing one aspect of a bottom gate-top contact type organic thin film transistor as an example of the organic semiconductor element according to the present invention.
Fig. 3 is a schematic plan view showing a formation pattern of openings of a mask used for forming a source electrode and a drain electrode using an ink jet printing method in Examples.

## DESCRIPTION OF THE PREFERRED EMBODIMENTS

In this specification, numerical ranges represented by "to" include numerical values before and after "to" as lower limit values and upper limit values.

The meaning of compounds described in this specification include not only the compounds themselves but also salts and ions thereof. In addition, within a range where a desired effect does not deteriorate, a part of the structure may be changed. In addition, in a case where it is not clearly described that a compound is substituted or unsubstituted, this compound has any substituent within a range where a desired effect does not deteriorate. The same shall be applied to a substituent, a linking group, or the like (hereinafter, referred to as "substituent or the like").

In this specification, in a case where a plurality of substituents or the like represented by a specific reference numeral are present or a plurality of substituents or the like are simultaneously defined, the respective substituents or the like may be the same as or different from each other unless specified otherwise. The same shall be applied to definition of the number of substituents or the like. In addition, in a case where a plurality of substituents or the like are close to (in particular, adjacent to) each other, the substituents or the like may be linked to each other to form a ring unless specified otherwise.

In addition, in a case where the number of carbon atoms in a group is limited, the number of carbon atoms in this group represents the total number of carbon atoms including substituents unless specified otherwise.

In the present invention, in a case where a group can form an acyclic skeleton and a cyclic skeleton, this group includes a group having an acyclic skeleton and a group having a cyclic skeleton unless specified otherwise. For example, an alkyl group includes a linear alkyl group, a branched alkyl group, and a cycloalkyl group. In a case where a group can form a cyclic skeleton, the lower limit of the number of atoms of the group forming a cyclic skeleton is not limited to the lower limit of the number of atoms specifically described regarding this group, and is 3 or more and preferably 5 or more.

Hereinafter, a preferred embodiment of the present invention will be described.

[Organic Semiconductor Film-Forming Composition]

An organic semiconductor film-forming composition according to an embodiment of the present invention (hereinafter, also simply referred to as "composition according to the embodiment of the present invention") comprises at least two organic semiconductors.

At least one organic semiconductor among the two organic semiconductors is a low molecular weight organic semiconductor compound having a molecular weight of 5000 or lower. Another one organic semiconductor among the two organic semiconductors is a fused heterocyclic compound represented by the following Formula (1) that has a chemical structure different from that of the low molecular weight organic semiconductor compound and has a molecular weight of 3000 or lower.

In addition to the low molecular weight organic semiconductor compound and the fused heterocyclic compound, the composition according to the embodiment of the present invention may include, for example, an organic semiconductor other than the low molecular weight organic semiconductor compound and the fused heterocyclic compound, a solvent, or an additive.

The components will be described in order.

<Low Molecular Weight Organic Semiconductor Compound>

The low molecular weight organic semiconductor compound is not particularly limited as long as it is an organic semiconductor compound having a molecular weight of 5000 or lower, and examples thereof include an organic semiconductor that can be used in an organic semiconductor element.

Since the molecular weight of the low molecular weight organic semiconductor compound is 5000 or lower, the solubility of the low molecular weight organic semiconductor compound in a solvent described below can be improved. From the viewpoint of solubility, the molecular weight of the low molecular weight organic semiconductor compound is preferably 3000 or lower, more preferably 1500 or lower, still more preferably 1000 or lower, and even still more preferably 800 or lower. The molecular weight of the low molecular weight organic semiconductor compound is not particularly limited, but from the viewpoints of the film quality stability of the organic semiconductor film, is preferably 400 or higher,

more preferably 450 or higher, and still more preferably 500 or higher.

The low molecular weight organic semiconductor compound is not particularly limited as long as it satisfies the molecular weight, and examples thereof include an aromatic hydrocarbon compound or a heteroaromatic compound that has a fused ring structure including 3 to 9 rings, a fullerene compound, a phthalocyanine compound, and a semiconductor polymer.

The aromatic hydrocarbon compound or the heteroaromatic compound that has a fused ring structure including 3 to 9 rings is not particularly limited as long as it is a compound having a fused ring structure in which 3 to 9 aromatic rings (aromatic hydrocarbon rings and aromatic heterocycles) are fused. Examples of the fused ring structure include a fused ring structure represented by T of Formula (A-1) described below. Examples of the aromatic hydrocarbon compound or the heteroaromatic compound include a compound represented by Formula (A-1) described below, preferably, a fused heterocyclic compound represented by Formula (1) described below. The aromatic hydrocarbon compound or the heteroaromatic compound may have a substituent or may be unsubstituted.

The fullerene compound is not particularly limited, and examples thereof include C60 or phenyl-$C_{61}$-butyric acid methyl ester (PCBM).

The phthalocyanine compound is not particularly limited, and examples thereof include copper phthalocyanine and fluorinated copper phthalocyanine.

The semiconductor polymer is not particularly limited, and examples thereof include a polythiophene such as polyquarterthiophene (PQT) or poly(3-hexyl thiophene) (P3HT), and a polythienothiophene such as poly[2,5-bis(3-dodecylthiophene-2-yl)thieno[3,2-b]thiophene] (PBTTT).

In particular, for example, from the viewpoint of improving the performance of the organic semiconductor element such as carrier mobility, an aromatic hydrocarbon compound or a heteroaromatic compound that has a fused ring structure including 3 to 9 rings is preferable, and a compound represented by the following Formula (A-1) is more preferable.

Formula (A-1) $\mathbf{R^{a1}\text{-}L^{a1}\text{-}T\text{-}L^{a2}\text{-}R^{a2}}$

In Formula (A-1), T represents an aromatic hydrocarbon group or an aromatic heterocyclic group that has a fused ring structure including 3 to 9 rings.

$L_{a1}$ and $L_{a2}$ each independently represent a single bond or a divalent linking group.

$R^{a1}$ and $R^{a2}$ each independently represent a hydrogen atom or a substituent.

T represents a group obtained by removing two hydrogen atoms from the fused ring structure in which 3 to 9 aromatic rings are fused (hereinafter, also referred to as "fused polycyclic aromatic group"). Examples of the aromatic ring include an aromatic hydrocarbon ring (for example, a benzene ring) and an aromatic heterocycle (for example, a thiophene ring, a furan ring, a pyrrole ring, a selenophene ring, an imidazole ring, a tellurophene ring, an oxazole ring, a thiazole ring, a selenazole ring, a pyridine ring, or a pyrimidine ring).

The fused ring structure of T is preferably a fused ring structure including 3 to 7 aromatic rings, more preferably a fused ring structure including 4 to 6 aromatic rings, and still more preferably a fused ring structure including 5 or 6 aromatic rings.

It is preferable that T represents an aromatic heterocyclic group. In this case, it is preferable that at least one aromatic ring in the fused ring structure of T is an aromatic heterocycle, and from the viewpoint of carrier mobility, it is more preferable that 2 to 7 aromatic rings are the aromatic heterocycles, and it is still more preferable that 3 to 5 aromatic rings are the aromatic heterocycles. As a ring-constituting heteroatom of the aromatic heterocycle, a ring at least one kind of atoms selected from the group consisting of a sulfur atom, a nitrogen atom, a selenium atom, an oxygen atom, and a tellurium atom is preferable, and a ring at least one kind of atoms selected from the group consisting of a sulfur atom, a nitrogen atom, a selenium atom, and an oxygen atom is more preferable. Examples of the aromatic heterocycle include the above-described groups. In particular, a 5-membered ring including Y and Z represented by Formula (1) described below is preferable.

In addition, from the viewpoint of carrier mobility, regarding a ring-constituting heteroatom of the aromatic heterocycle, it is preferable that one ring has one heteroatom.

From the viewpoint of carrier mobility, it is preferable that T has at least one ring selected from the group consisting of a thiophene ring, a furan ring, a selenophene ring, a pyrrole ring, a thiazole ring, a selenazole ring, an imidazole ring, and an oxazole ring, it is more preferable that T has at least one ring selected from the group consisting of a furan ring, a thiophene ring, and a selenophene ring, it is still more preferable that T has at least a thiophene ring and/or a selenophene ring, it is still more preferable that T has at least a thiophene ring, and it is still more preferable that all the aromatic heterocycles in T are thiophene rings.

It is preferable that the compound represented by Formula (A-1) includes a group represented by T or the group T is included as a main skeleton. Here, the main skeleton represents that the proportion of the molecular weight of the group represented by T is 30% or higher and preferably 40% or higher with respect to the total molecular weight of the

compound represented by Formula (A-1). The upper limit is not particularly limited and is preferably 80% or lower from the viewpoint of solubility.

It is preferable that the fused ring structure of T is a structure in which aromatic heterocycles and/or benzene rings are fused in a linear shape (including a straight-line shape or a zigzag shape), and it is more preferable that the fused ring structure of T includes an acene structure, a phenacene structure, or a heteroacene structure that has a fused ring structure including 3 to 7 rings.

Here, the acene structure is a structure in which benzene rings are linearly fused such that an angle between the benzene rings is 180°, and specific examples thereof include a fused ring structure such as naphthalene, anthracene, tetracene, pentacene, hexacene, or heptacene.

In addition, the phenacene structure is a structure in which benzene rings are fused in a zigzag shape, and specific examples thereof include a fused ring structure such as phenanthrene, chrysene, or picene.

Further, the heteroacene structure represents that some of benzene rings that form an acene structure or a phene structure are substituted with an aromatic heterocycle (for example the above-described rings). The phene structure is a structure in which benzene rings are fused in patterns including a zigzag shape, for example, a phenacene structure in which all the benzene rings are fused in a zigzag shape. Specific examples of a fused ring structure that is the phene structure but is not the phenacene structure include a fused ring structure such as benzo[a]anthracene, benzo[c]phenanthrene, dibenzo[a,h]anthracene, dibenzo[a,j]anthracene, dibenzo[c,g]phenanthrene, or pentaphene.

In the compound represented by Formula (A-1), it is preferable that T includes a heteroacene skeleton that is a structure in which aromatic heterocycles and/or benzene rings are linearly fused, it is more preferable that T includes a heteroacene skeleton that is a skeleton in which 5-membered ring including Y and Z represented by Formula (1) described below as aromatic heterocycles and/or benzene rings are linearly fused, it is still more preferable that T includes a thienoaceneskeleton that is a structure in which thiophene rings and/or benzene rings are linearly fused, and it is still more preferable T includes a thienoacene structure in which 3 to 7 rings are fused. According to the above-described aspects, an organic semiconductor film having a higher carrier mobility can be obtained.

From the viewpoint of carrier mobility, the number of thiophene rings in the fused polycyclic aromatic group T is preferably 2 to 7, more preferably 3 to 7, and still more preferably 3 to 5.

Specific examples of the fused ring structure of T are not particularly limited include the following fused ring structures, preferably a fused ring structure represented by Formula (1) described below. As described below, these fused ring structures include Ra1 and Ra2 described below bonded to any ring-constituting atom and may further have a substituent bonded to another ring-constituting atom.

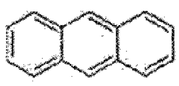
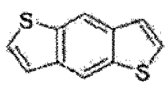
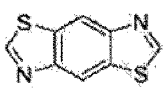
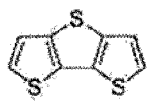
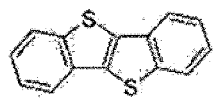

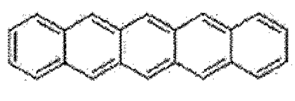
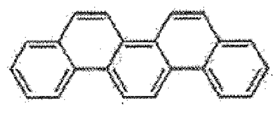
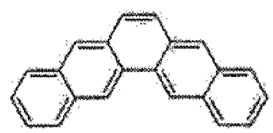

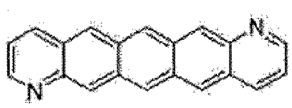
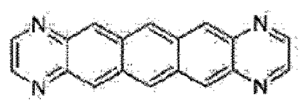
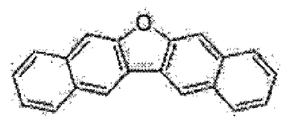

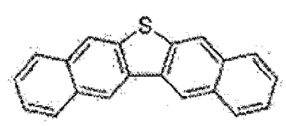
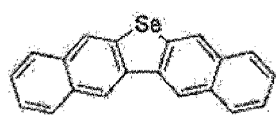
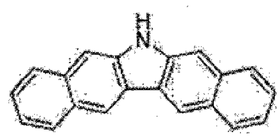

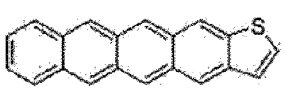
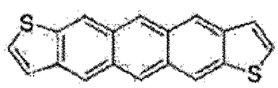
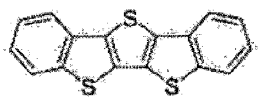
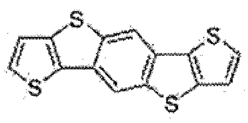
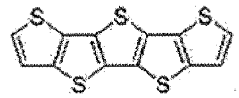
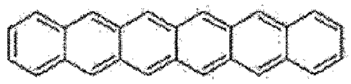
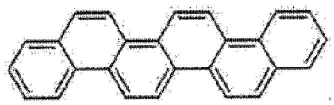
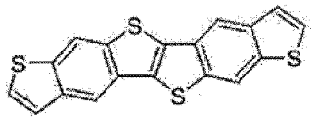
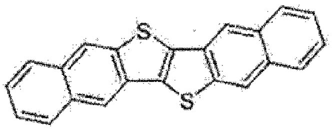
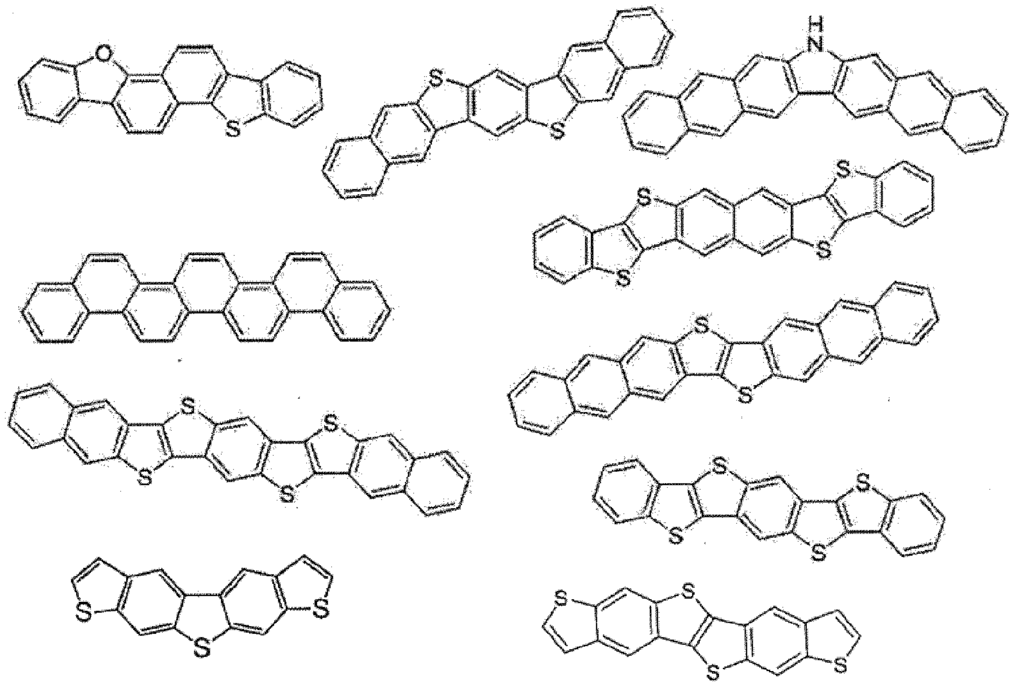
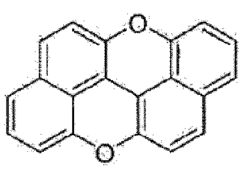
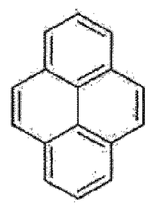
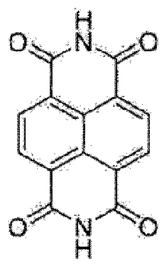
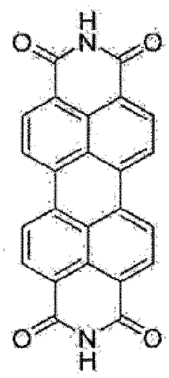
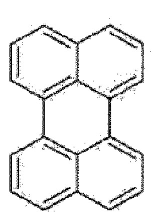
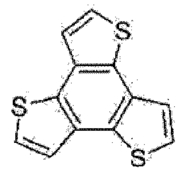

Among the above-described fused ring structures, a fused ring structure such as dinaphthothienothiophene or

benzothienobenzothiophene is preferable.

In Formula (A-1), $L^{a1}$ and $L^{a2}$ each independently represent a single bond or a divalent linking group and preferably a single bond.

The divalent linking group is not particularly limited and has the same definition and the same preferable configurations as each group of the divalent group A in Formula (1) described below.

In Formula (A-1), $R^{a1}$ and $R^{a2}$ each independently represent a hydrogen atom or a substituent and preferably a substituent.

The substituent which may be used as $R^{a1}$ and $R^{a2}$ is not particularly limited and has the same definition and the same preferable configurations as the group which may be used as $R^1$ in Formula (1) described below. In particular, an alkyl group, an aryl group, or a heteroaryl group is more preferable.

The alkyl group which may be used as $R^{a1}$ and $R^{a2}$ has the same definition and the same preferable configurations as the alkyl group which may be used as $R^1$ in Formula (1). In this case, from the viewpoint of improving solubility to improve carrier mobility, the number of carbon atoms in each of the two alkyl groups which may be used as $R^{a1}$ and $R^{a2}$ in the compound represented by Formula (A-1) is preferably 1 to 20, more preferably 2 to 16, and still more preferably 3 to 12. It is preferable that the two alkyl groups which may be used as $R^{a1}$ and $R^{a2}$ have different numbers of carbon atoms.

The aryl group which may be used as $R^{a1}$ and $R^{a2}$ has the same definition and the same preferable configurations as the aryl group which may be used as $R^1$ in Formula (1). The substituent which may be included in the aryl group is not particularly limited and has the same definition and the same preferable configurations as the substituent which may be included in the aryl group used as $R^1$ in Formula (1). In particular, an alkyl group is preferable. The alkyl group which may be included in the aryl group used as $R^{a1}$ and $R^{a2}$ has the same definition and the same preferable configurations as the alkyl group which may be used as $R^{a1}$ and $R^{a2}$.

A position where the substituent is bonded to the aryl group which may be used as $R^{a1}$ and $R^{a2}$ is not particularly limited. In a case where the aryl group is a phenyl group, the position is a para position with respect to a carbon atom bonded to T.

The heteroaryl group which may be used as $R^{a1}$ and $R^{a2}$ has the same definition and the same preferable configurations as the heteroaryl group which may be used as $R^1$ in Formula (1). The substituent which may be included in the heteroaryl group is not particularly limited and has the same definition and the same preferable configurations as the substituent which may be included in the heteroaryl group used as $R^1$ in Formula (1). In particular, an alkyl group is preferable. The alkyl group which may be included in the heteroaryl group used as $R^{a1}$ and $R^{a2}$ has the same definition and the same preferable configurations as the alkyl group which may be used as $R^{a1}$ and $R^{a2}$.

A position where the substituent is bonded to the heteroaryl group which may be used as $R^{a1}$ and $R^{a2}$ is not particularly limited. In a case where the heteroaryl group is a thienyl group, it is preferable that T and the substituent are bonded in the 2-position or 5-position.

In addition to $R^{a1}$ and $R^{a2}$, the compound represented by Formula (A-1) may further have a substituent. The substituent is not particularly limited, and examples thereof include the same group as $-L^{a1}-R^{a1}$ and a substituent included in the alkyl group which may be used as $R^1$ or $R^2$ described below. In addition, the substituent may be further substituted with another substituent.

As the other substituent which may be further included in the substituent, a halogen atom, an alkyl group, an alkenyl group, an alkoxy group, an alkylthio group, or an aryl group is preferable, a fluorine atom, a substituted or unsubstituted alkyl group having 1 to 3 carbon atoms, a substituted or unsubstituted alkoxy group having 1 or 2 carbon atoms, a substituted or unsubstituted methylthio group, or a phenyl group is more preferable, and a fluorine atom, a substituted or unsubstituted alkyl group having 1 to 3 carbon atoms, a substituted or unsubstituted alkoxy group having 1 or 2 carbon atoms, or a substituted or unsubstituted methylthio group is still more preferable.

The number of substituents which may be included in the compound represented by Formula (A-1) is not particularly limited as long as it is (Number of Hydrogen Atoms which may be included in Fused Ring Structure of T-2) or less and is preferably 0 to 2.

In the compound represented by Formula (A-1), binding sites of $-L^{a1}-R^{a1}$ and $-L^{a2}-R^{a2}$ to the fused ring structure of T are not particularly limited. The binding sites are preferably a pair of ring-constituting atoms point-symmetric, line-symmetric, or rotation-symmetric to the fused ring structure, and more preferably a pair of ring-constituting atoms that form rings positioned at opposite terminals of the fused ring structure.

Among the compounds represented by Formula (A-1), a fused heterocyclic compound represented by Formula (1) described below is preferable. The details of the fused heterocyclic compound represented by Formula (1) will be described below.

The compound represented by Formula (A-1) and specific examples thereof will be shown below, but the present invention is not limited to these compounds.

Examples include a pentacene compound such as 6,13-bis(triisopropylsilylethynyl)pentacene (TIPS-PEN), tetramethyl pentacene, or perfluoropentacene, an anthradithiophene compound (ADT) such as 5,11-bis(triethylsilylethynyl)anthradithiophene (TES-ADT) or 2,8-difluoro-5,11-bis(triethylsilylethynyl)anthradithiophene (diF-TES-ADT), a benzothienobenzothiophene compound (BTBT) such as diphenyl benzothienobenzothiophene (DPh-BTBT) or dialkyl benzothienobenzothiophene (Cn-BTBT), a dinaphthothienothiophene compound (DNTT) such as dialkyl dinaphthothienothiophene (Cn-DNTT), a dioxaanthanthrene compound such as peri-xanthenoxanthene, and a rubrene compound.

Among the following exemplary compounds, compounds A-19 to A-21 are also specific examples of the fused heterocyclic compound represented by Formula (1).

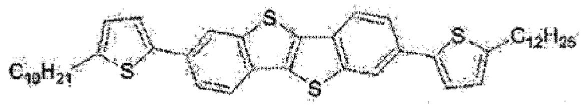

Compound A-1

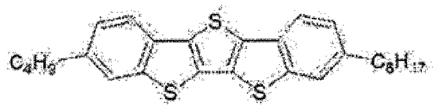

Compound A-2

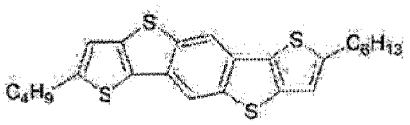

Compound A-3

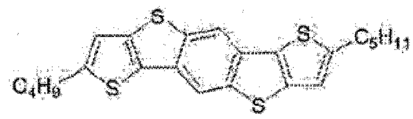

Compound A-4

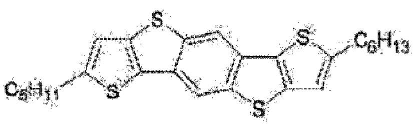

Compound A-5

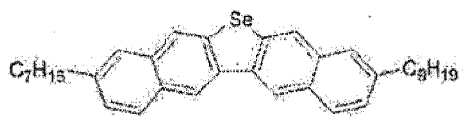

Compound A-6

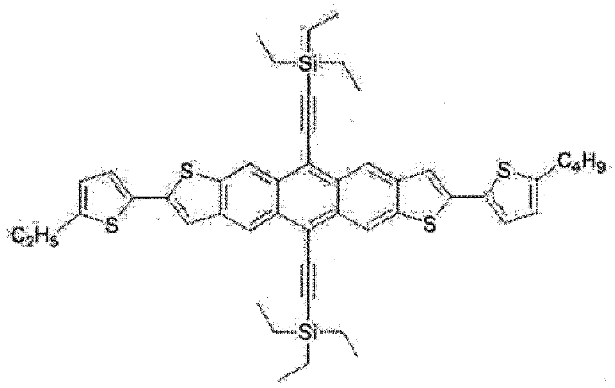

Compound A-7

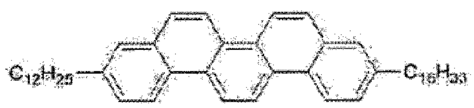

Compound A-8

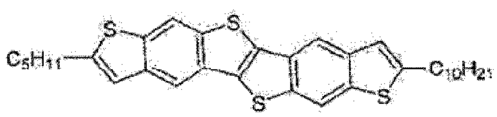

Compound A-9

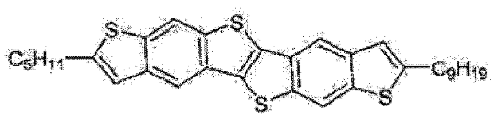

Compound A-10

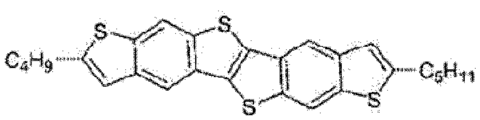

Compound A-11

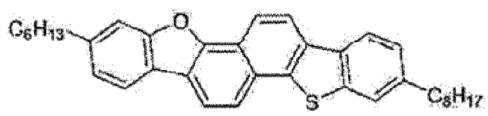

Compound A-12

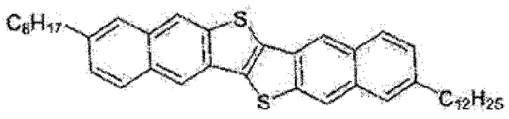

Compound A-13

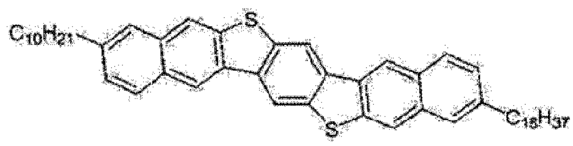

Compound A-14

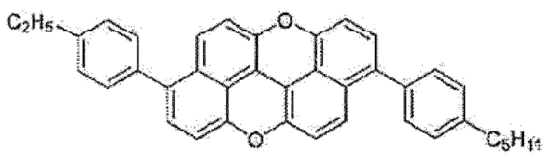

Compound A-15

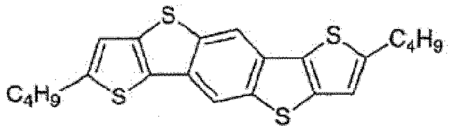

Compound A-16

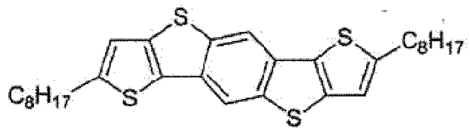

Compound A-17

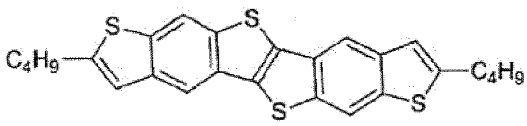

Compound A-18

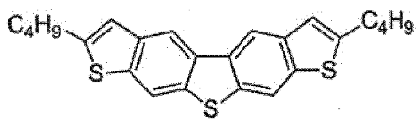

Compound A-19

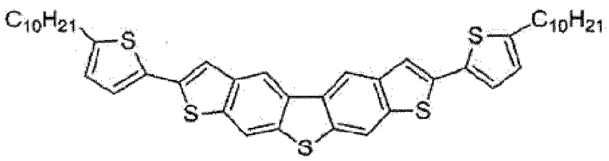

Compound A-20

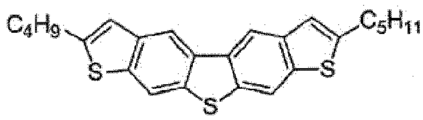

Compound A-21

As the low molecular weight organic semiconductor compound included in the composition according to the embodiment of the present invention, one kind may be used alone, or two or mote kinds may be used.

The low molecular weight organic semiconductor compound can be synthesized using a typical method, and a commercially available product thereof is also available.

For example, the compound represented by Formula (A-1) can be synthesized using a typical method. Specific examples of a method of manufacturing the compound represented by Formula (A-1) include various methods described in JP2011-032268A, JP2009-054810A, JP2011-526588A, JP2012-209329A, Scientific Report 2014, 4, p. 5048, JP2013-540697A, JP2009-218333A, US2008/0142792A, WO2014/156773A, WO2010/098372A, Adv. Mater., 2014, 26, p. 4546, and JP2010-006794A.

The content of the low molecular weight organic semiconductor compound (preferably the compound represented by Formula (A-1)) in the composition according to the embodiment of the present invention (in a case where the composition according to the embodiment of the present invention includes two or more low molecular weight organic semiconductor compounds, the total content thereof; hereinafter, the same shall be applied) is preferably 0.3 to 15 mass% and more preferably 0.5 to 10 mass% with respect to the total mass of the composition according to the embodiment of the present invention. In a case where the content is 0.3 mass% or higher, an organic semiconductor element having a high carrier mobility can be obtained. On the other hand, in a case where the content is lower than 15 mass%,

the composition according to the embodiment of the present invention can be suitably applied to a printing method, in particular, an ink jet printing or flexographic printing method.

The content of the low molecular weight organic semiconductor compound (preferably the compound represented by Formula (A-1)) in the composition according to the embodiment of the present invention is preferably 5 to 98 mass%, more preferably 10 to 95 mass%, and still more preferably 20 to 80 mass% with respect to the total mass of the solid content.

In addition, the content of the low molecular weight organic semiconductor compound (preferably the compound represented by Formula (A-1)) is preferably 50 to 99 mass% and more preferably 85 to 98 mass% with respect to the total mass of the solid content excluding a binder polymer described below.

Further, the content of the low molecular weight organic semiconductor compound with respect to the total mass of the organic semiconductors included in the composition according to the embodiment of the present invention is appropriately set in a range satisfying the above-described content. The content of the low molecular weight organic semiconductor compound is preferably 80 to 99 mass% and more preferably 85 to 98 mass%.

In a case where the composition according to the embodiment of the present invention includes an organic semiconductor other than the low molecular weight organic semiconductor compound and the fused heterocyclic compound described below, the content of the low molecular weight organic semiconductor compound (preferably the compound represented by Formula (A-1)) is preferably 50 mass% or higher, more preferably 70 mass% or higher, and still more preferably 90 mass% or higher with respect to the total mass of the organic semiconductors.

<Fused Heterocyclic Compound>

The fused heterocyclic compound is included in the composition according to the embodiment of the present invention as one organic semiconductor. The fused heterocyclic compound is represented by the following Formula (1) and has a chemical structure different from that of the low molecular weight organic semiconductor compound. In addition, the molecular weight of the fused heterocyclic compound is 3000 or lower.

In a case where the composition according to the embodiment of the present invention includes the fused heterocyclic compound in addition to the low molecular weight organic semiconductor compound, the composition is more stably printed (jetted) using a printing method, in particular, an ink jet printing method as compared to a composition including a polymer compound, for example, the organic binder resin or the conjugated polymer compound. Therefore, the composition according to the embodiment of the present invention can be applied using a printing method, in particular, an ink jet printing method that is suitable for the method of manufacturing the organic semiconductor element from the viewpoint of cost or productivity. The organic semiconductor film that is formed using a printing method exhibits a high carrier mobility and can effectively reduce the occurrence of hysteresis.

First, the fused heterocyclic compound will be described.

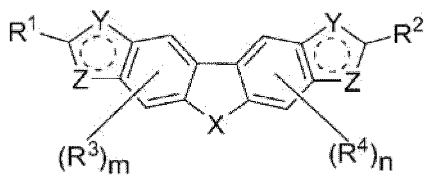

Formula (1)

In Formula (1), X represents an oxygen atom, a sulfur atom, a selenium atom, a tellurium atom, or $NR^5$. From the viewpoint of carrier mobility during application to an organic semiconductor layer, it is preferable that X represents an oxygen atom, a sulfur atom, or a selenium atom. The details of $R^5$ will be described below.

Y and Z each independently represent $CR^6$, an oxygen atom, a sulfur atom, a selenium atom, a nitrogen atom, or $NR^7$, and a 5-membered ring including Y and Z is an aromatic heterocycle. The details of $R^6$ and $R^7$ will be described below.

Y represents preferably $CR^6$, an oxygen atom, or a sulfur atom and more preferably $CR^6$ or a sulfur atom. In addition, Z represents preferably $CR^6$, an oxygen atom, a sulfur atom, or $NR^7$, more preferably $CR^6$, an oxygen atom, or a sulfur atom, and still more preferably $CR^6$ or a sulfur atom.

In a case where Y represents $CR^6$, Z represents preferably an oxygen atom, a sulfur atom, a selenium atom, or $NR^{7a}$, more preferably an oxygen atom, a sulfur atom, or a selenium atom, still more preferably an oxygen atom or a sulfur atom, and still more preferably a sulfur atom.

In addition, in a case where Y represents an oxygen atom, Z represents preferably $CR^6$, an oxygen atom, or a sulfur atom, more preferably CR6, or a sulfur atom, and still more preferably $CR^6$.

In a case where Y represents a sulfur atom, Z represents preferably $CR^6$, an oxygen atom, a sulfur atom, or a nitrogen atom, more preferably $CR^6$ or a nitrogen atom, and still more preferably $CR^6$.

In a case where Z represents $CR^6$, Y represents preferably an oxygen atom, a sulfur atom, a selenium atom, or $NR^7$, and more preferably an oxygen atom, a sulfur atom, or a selenium atom.

In Formula (1), the aromatic heterocycle in the 5-membered ring including Y and Z is preferably a ring selected from a thiophene ring, a furan ring, a selenophene ring, a pyrrole ring, a thiazole ring, a selenazole ring, an imidazole ring, or an oxazole ring, more preferably a ring selected from a thiophene ring, a furan ring, a selenophene ring, a pyrrole ring, a thiazole ring, or an oxazole ring, still more preferably a thiophene ring, a furan ring, a selenophene ring, or a pyrrole ring, and still more preferably a thiophene ring or a selenophene ring.

In Formula (1), $R^1$ and $R^2$ each independently represent a hydrogen atom, an alkyl group, an alkenyl group, an alkynyl group, an aryl group, or a heteroaryl group. $R^1$ and $R^2$ each independently represent preferably an alkyl group, an alkenyl group, an alkynyl group, an aryl group, or a heteroaryl group, and more preferably an alkyl group, an aryl group, or a heteroaryl group.

The number of carbon atoms in the alkyl group which may be used as $R^1$ and $R^2$ is preferably 30 or less and more preferably 20 or less. More specifically, the number of carbon atoms in the alkyl group which may be used as $R^1$ and $R^2$ is preferably 1 to 30, more preferably 1 to 20, still more preferably 1 to 15, and still more preferably 3 to 11. By adjusting the number of carbon atoms in the alkyl group to be in the preferable range, the linearity of molecules can be improved, and the carrier mobility can be further improved during application to an organic semiconductor layer.

The alkyl group which may be used as $R^1$ and $R^2$ may be linear, branched, or cyclic. From the viewpoint of carrier mobility, a linear alkyl group is preferable.

In a case where $R^1$ or $R^2$ represents an alkyl group having a substituent, the substituent in the alkyl group is not particularly limited, and examples thereof include: a halogen atom (for example, a fluorine atom, a chlorine atom, a bromine atom, or an iodine atom); a cycloalkyl group (a cycloalkyl group preferably having 3 to 20 carbon atoms and more preferably 4 to 15 carbon atoms; the cycloalkyl group is preferably a 5-membered ring or a 6-membered ring); an aryl group (an aryl group having preferably 6 to 20 carbon atoms, more preferably 6 to 18 carbon atoms, and still more preferably 6 to 15 carbon atoms; for example, a phenyl group, a naphthyl group, a p-pentylphenyl group, a 3,4-dipentylphenyl group, a p-heptoxyphenyl group, or a 3,4-diheptoxyphenyl group); a heterocyclic group (preferably a 3 to 8-membered ring and more preferably a 5- or 6-membered ring; it is preferable that as a ring-constituting atom, an oxygen atom, a sulfur atom, and/or a nitrogen atom is included; for example, a 2-hexylfuranyl group); a cyano group; a hydroxy group; a nitro group; an acyl group (an acyl group having preferably from 2 to 20 carbon atoms, more preferably from 2 to 15 carbon atoms, and still more preferably from 2 to 10 carbon atoms; for example, a hexanoyl group or a benzoyl group); an alkoxy group (an alkoxy group having preferably 1 to 20 carbon atoms, more preferably 1 to 15 carbon atoms, and still more preferably 1 to 10 carbon atoms; for example, a butoxy group); an aryloxy group (an aryloxy group having preferably 6 to 20 carbon atoms, more preferably 6 to 18 carbon atoms, and still more preferably 6 to 15 carbon atoms); a silyloxy group (a silyloxy group having preferably 0 to 18 carbon atoms, more preferably 0 to 15 carbon atoms, and still more preferably 0 to 12 carbon atoms); a heterocyclic oxy group (a heterocyclic oxy group including preferably 3 to 8 rings and more preferably 5 or 6 rings); an acyloxy group (an acyloxy group having preferably 2 to 20 carbon atoms, more preferably 2 to 15 carbon atoms, and still more preferably 2 to 10 carbon atoms); a carbamoyloxy group (a carbamoyloxy group having preferably 1 to 20 carbon atoms, more preferably 1 to 15 carbon atoms, and still more preferably 1 to 10 carbon atoms); an amino group (an amino group having preferably 0 to 20 carbon atoms, more preferably 0 to 15 carbon atoms, and still more preferably 0 to 10 carbon atoms and including an anilino group); an acylamino group (an acylamino group having preferably 2 to 20 carbon atoms, more preferably 2 to 15 carbon atoms, and still more preferably 2 to 10 carbon atoms); an aminocarbonylamino group (an aminocarbonylamino group having preferably 1 to 20 carbon atoms, more preferably 1 to 15 carbon atoms, and still more preferably 1 to 10 carbon atoms); an alkoxycarbonylamino group (an alkoxycarbonylamino group having preferably 2 to 20 carbon atoms, more preferably 2 to 15 carbon atoms, and still more preferably 2 to 10 carbon atoms); an aryloxycarbonylamino group (an aryloxycarbonylamino group having preferably 7 to 20 carbon atoms, more preferably 7 to 18 carbon atoms, and still more preferably 7 to 15 carbon atoms); an alkylsulfonylamino group (an alkylsulfonylamino group having preferably 1 to 20 carbon atoms, more preferably 1 to 15 carbon atoms, and still more preferably 1 to 10 carbon atoms); an arylsulfonylamino group (an arylsulfonylamino group having preferably 6 to 20 carbon atoms, more preferably 6 to 18 carbon atoms, and still more preferably 6 to 15 carbon atoms); a mercapto group; an alkylthio group (an alkylthio group having preferably 1 to 20 carbon atoms, more preferably from 1 to 15 carbon atoms, and still more preferably from 1 to 10 carbon atoms; for example, a methylthio group or an octylthio group); an arylthio group (an arylthio group having preferably 6 to 20 carbon atoms, more preferably 6 to 18 carbon atoms, and still more preferably 6 to 15 carbon atoms); a heterocyclic thio group (a heterocyclic thio group including preferably 3 to 8 rings and more preferably 5 or 6 rings); a sulfamoyl group (a sulfamoyl group having preferably 0 to 20 carbon atoms, more preferably 0 to 15 carbon atoms, and still more preferably 0 to 10 carbon atoms); a sulfo group; an alkylsulfinyl group (an alkylsulfinyl group having preferably 1 to 20 carbon atoms, more preferably 1 to 15 carbon atoms, and still more preferably 1 to 10 carbon atoms); an arylsulfinyl group (an arylsulfinyl group having preferably 6 to 20 carbon atoms, more preferably 6 to 18 carbon atoms, and still more preferably 6 to 15 carbon atoms); an alkylsulfonyl group (an alkylsulfonyl group having preferably 1 to 20 carbon atoms, more

preferably 1 to 15 carbon atoms, and still more preferably 1 to 10 carbon atoms); an arylsulfonyl group (an arylsulfonyl group having preferably 6 to 20 carbon atoms, more preferably 6 to 18 carbon atoms, and still more preferably 6 to 15 carbon atoms); an alkyloxycarbonyl group (an alkyloxycarbonyl group having preferably 2 to 20 carbon atoms, more preferably 2 to 15 carbon atoms, and still more preferably 2 to 10 carbon atoms); an aryloxycarbonyl group (an aryloxycarbonyl group having preferably 7 to 20 carbon atoms, more preferably 7 to 18 carbon atoms, and still more preferably 7 to 15 carbon atoms); a carbamoyl group (a carbamoyl group having preferably 1 to 20 carbon atoms, more preferably 1 to 15 carbon atoms, and still more preferably 1 to 10 carbon atoms); an aryl azo group (an aryl azo group having preferably 6 to 20 carbon atoms, more preferably 6 to 18 carbon atoms, and still more preferably 6 to 15 carbon atoms); a heterocyclic azo group (a heterocyclic azo group including preferably 3 to 8 rings and more preferably 5 or 6 rings); a phosphino group; a phosphinyl group; a phosphinyloxy group; a phosphinylamino group;a phosphono group; a silyl group (a silyl group having preferably 0 to 18 carbon atoms, more preferably 0 to 15 carbon atoms, and still more preferably 0 to 12 carbon atoms; for example, a di-trimethylsiloxy methyl butoxy group); a hydrazino group; a ureido group; a boronic acid group ($-B(OH)_2$); a phosphate group ($-OPO(OH)_2$); a sulfate group ($-OSO_3H$); and other well-known substituents. In addition, in a case where the alkyl group which may be used as $R^1$ and $R^2$ is a cycloalkyl group, this cycloalkyl group may include, as a substituent, an alkyl group, an alkenyl group, or an alkynyl group which may be included in the aryl group used as $R^1$ and $R^2$.

In particular, it is preferable that $R^1$ or $R^2$ represents an unsubstituted alkyl group.

The number of carbon atoms in the alkenyl group which may be used as $R^1$ and $R^2$ is preferably 30 or less and more preferably 20 or less. More specifically, the number of carbon atoms in the alkenyl group which may be used as $R^1$ and $R^2$ is preferably 2 to 30, more preferably 2 to 20, still more preferably 2 to 15, still more preferably 2 to 10, and still more preferably 2 to 4.

A substituent which may be included in the alkenyl group is not particularly limited. For example, the alkenyl group which may be used as $R^1$ and $R^2$ may have the substituent which may be included in the alkyl group used as $R^1$ and $R^2$.

The number of carbon atoms in the alkynyl group which may be used as $R^1$ and $R^2$ is preferably 30 or less and more preferably 20 or less. More specifically, the number of carbon atoms in the alkenyl group which may be used as $R^1$ and $R^2$ is preferably 2 to 30, more preferably 2 to 20, still more preferably 2 to 15, still more preferably 2 to 10, still more preferably 2 to 4, and still more preferably 2.

A substituent which may be included in the alkynyl group is not particularly limited. For example, the alkynyl group which may be used as $R^1$ and $R^2$ may have the substituent which may be included in the alkyl group used as $R^1$ and $R^2$. In particular, as the substituent which may be included in the alkynyl group, a group selected from a silyl group or an aryl group is preferable, a group selected from a trialkylsilyl group or a phenyl group is more preferable, and a trialkylsilyl group is still more preferable.

The number of carbon atoms in the aryl group which may be used as $R^1$ and $R^2$ is preferably 30 or less and more preferably 20 or less. More specifically, the number of carbon atoms in the aryl group which may be used as $R^1$ and $R^2$ is preferably 6 to 30, more preferably 6 to 20, still more preferably 6 to 18, and still more preferably 6 to 12.

A substituent which may be included in the aryl group is not particularly limited. For example, the aryl group which may be used as $R^1$ and $R^2$ may have the substituent which may be included in the alkyl group used as $R^1$ and $R^2$. In addition, the substituent which may be included in the aryl group used as $R^1$ and $R^2$ is preferably an alkyl group (a linear, branched, or cyclic alkyl group having preferably 1 to 20 carbon atoms, more preferably 1 to 15 carbon atoms, and still more preferably 1 to 10 carbon atoms), and may be an alkenyl group (a linear or branched alkenyl group having preferably 2 to 20 carbon atoms, more preferably 2 to 15 carbon atoms, and still more preferably 2 to 10 carbon atoms) or an alkynyl group (a linear or branched alkynyl group having preferably 2 to 20 carbon atoms, more preferably 2 to 15 carbon atoms, and still more preferably 2 to 10 carbon atoms).

In a case where the aryl group which may be used as $R^1$ and $R^2$ has a substituent, the number of substituents in the aryl group is preferably 1 to 3, more preferably 1 or 2, and still more preferably 1.

The number of carbon atoms in the heteroaryl group which may be used as $R^1$ and $R^2$ is preferably 30 or less and more preferably 20 or less. More specifically, the number of carbon atoms in the heteroaryl group which may be used as $R^1$ and $R^2$ is preferably 3 to 30, more preferably 4 to 20, still more preferably 4 to 10, and still more preferably 4. As a ring-constituting heteroatom in the heteroaryl group, a ring at least one kind of atoms selected from the group consisting of a sulfur atom, a nitrogen atom, a selenium atom, an oxygen atom, and a tellurium atom is preferable. In addition, this heteroaryl group is preferably a 3- to 8-membered ring and more preferably 5-or 6-membered ring.

Specific preferable examples of the heteroaryl group which may be used as $R^1$ and $R^2$ include a furanyl group, a pyrrolyl group, a pyrazolyl group, an imidazolyl group, a thienyl group, a thiazolyl group, a thienothienyl group, a benzothienyl group, a thienophenyl group, a pyridyl group, a pyrimidinyl group, a pyridazinyl group, and a pyrazinyl group. In particular, a thienyl group or a furyl group is more preferable, and a thienyl group is still more preferable.

A substituent which may be included in the heteroaryl group is not particularly limited. For example, the heteroaryl group may have the substituent which may be included in the aryl group used as $R^1$ and $R^2$. In a case where the heteroaryl

group which may be used as $R^1$ and $R^2$ has a substituent, it is preferable that this substituent is an alkyl group.

From the viewpoint of further improving carrier mobility during application to an organic semiconductor layer, it is preferable that the structure of $R^1$ and $R^2$ has an aliphatic hydrocarbon group. In particular, it is preferable that $R^1$ and $R^2$ each independently represent an aryl group or a heteroaryl group and the aryl group or the heteroaryl group has an aliphatic hydrocarbon group as a substituent.

In the present invention, the aliphatic hydrocarbon group refers to a linear, branched, or cyclic nonaromatic hydrocarbon group, and examples thereof include an alkyl group, an alkenyl group, and an alkynyl group. Among these, an alkyl group is preferable.

In Formula (1), $R^1$ and $R^2$ in Formula (1) are bonded to a ring-constituting atom of the 5-membered ring including Y and Z directly or indirectly through a divalent group A. That is, the configuration of Formula (1) defined in the present invention also includes a configuration in which $R^1$ and/or $R^2$ is bonded to a ring-constituting atom of the 5-membered ring including Y and Z through the divalent group A. The divalent group A is a group selected from -O-, -S-, $-NR^8-$, -CO-, -SO-, or $-SO_2-$ or is a group in which two or more selected from -O-, -S-, $-NR^8-$, -CO-, -SO-, or $-SO_2-$ are linked to each other. In a case where the divalent group A is a group in which two or more selected from -O-, -S-, $-NR^8-$, -CO-, -SO-, or $-SO_2-$ are linked to each other, the number of the two or more groups linked to each other is preferably 2 to 4 and more preferably 2 or 3.

In particular, the divalent group A is preferably a group selected from -O-, -S-, $-NR^8-$-CO-, -SO-, or $-SO_2-$, more preferably -O-, -S-, or -CO-, and still more preferably -O-.

It is preferable that $R^1$ and $R^2$ have the same structure in a case where the divalent group A is also taken into consideration.

In Formula (1), $R^3$ and $R^4$ each independently represent a halogen atom, an alkyl group, an alkenyl group, an alkynyl group, an aryl group, or a heteroaryl group. The halogen atom which may be used as $R^3$ and $R^4$ is preferably a fluorine atom, a chlorine atom, a bromine atom, or an iodine atom and more preferably a fluorine atom or a chlorine atom.

The alkyl group, the alkenyl group, the alkynyl group, the aryl group, and the heteroaryl group which may be used as $R^3$ and $R^4$ have the same preferable configurations as the alkyl group, the alkenyl group, the alkynyl group, the aryl group, and the heteroaryl group which may be used as $R^1$. Among these configurations, a configuration in which the alkyl group, the alkenyl group, the alkynyl group, the aryl group, and the heteroaryl group which may be used as $R^3$ and $R^4$ have a halogen atom as a substituent is also preferable.

In addition, $R^3$ and $R^4$ are bonded to a ring-constituting atom of a benzene ring in Formula (1) directly or indirectly through the divalent group A, That is, the configuration of Formula (1) defined in the present invention also includes a configuration in which $R^3$ and/or $R^4$ is bonded to a ring-constituting atom of the benzene ring through the divalent group A.

It is preferable that $R^3$ and $R^4$ have the same structure in a case where the divalent group A is also taken into consideration.

m and n representing the numbers of $R^3$ and $R^4$ each independently represent an integer of 0 to 2. m and n each independently represent preferably 0 or 1 and more preferably 0. It is preferable that m and n have the same value.

In Formula (1), $R^5$ in $NR^5$ which may be used as X, $R^6$ in $CR^6$ and $R^7$ in $NR^7$ which may be used as Y and Z, and $R^8$ in $NR^8$ which may be used as the divalent group A each independently represent a hydrogen atom, an alkyl group, an alkenyl group, an alkynyl group, an aryl group, or a heteroaryl group. Examples of preferable configurations of the alkyl group, the alkenyl group, the alkynyl group, the aryl group, and the heteroaryl group which may be used as $R^5$, $R^6$, $R^7$, and $R^8$ include the preferable configurations of the alkyl group, the alkenyl group, the alkynyl group, the aryl group, and the heteroaryl group which may be used as $R^1$.

In particular, $R^5$ represents preferably an alkyl group having 1 to 11 carbon atoms and more preferably an alkyl group having 1 to 5 carbon atoms. In addition, $R^6$ represents preferably a hydrogen atom or an alkyl group and more preferably a hydrogen atom. In addition, $R^7$ represents preferably an alkyl group or an aryl group and more preferably an alkyl group. In addition, $R^8$ represents preferably an alkyl group or an aryl group.

From the viewpoint of improving molecular symmetry to further improve carrier mobility, in Formula (1), it is preferable that $R^1$ and $R^2$ are the same, $R^3$ and $R^4$ are the same, and m and n are the same.

In the present invention, examples of the compound represented by Formula (1) include all the compounds represented by the formula. However, in one aspect of the compound represented by Formula (1), the compound represented by Formula (1) does not include a configuration in which X represents an oxygen atom or a sulfur atom and the 5-membered ring including Y and Z is an imidazole ring (including a configuration in which a ring-constituting atom of the imidazole ring has a substituent). In addition, in another aspect, the compound represented by Formula (1) does not also include a configuration in which X represents a sulfur atom, Y represents CH, Z represents a sulfur atom, both $R^1$ and $R^2$ represent a hydrogen atom, and both m and n represent 0. In a case where the compound represented by Formula (1) according to any one of the aspects is applied to the organic semiconductor layer, a desired carrier mobility can be obtained.

it is preferable that the compound represented by Formula (1) is a compound represented by the following

Formula (2) or (3).

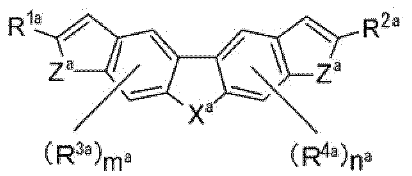

Formula (2)

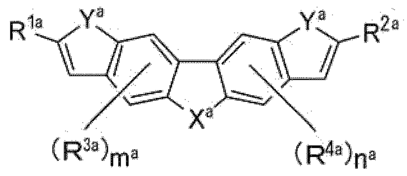

Formula (3)

In Formulae (2) and (3), $X^a$ represents an oxygen atom, a sulfur atom, or a selenium atom.

$Y^a$ and $Z^a$ each independently represent an oxygen atom, a sulfur atom, a selenium atom, or $NR^{7a}$. $R^{7a}$ has the same definition as $R^7$ in Formula (1).

$R^{1a}$, $R^{2a}$, $R^{3a}$, $R^{4a}$, $m^a$, and $n^a$ have the same definitions and the same preferable configurations as $R^1$, $R^2$, $R^3$, $R^4$, m, and n in Formula (1), respectively.

In addition, binding forms of $R^{1a}$, $R^{2a}$, $R^{3a}$, and $R^{4a}$ to a ring-constituting atom are also the same as binding forms of $R^1$, $R^2$, $R^3$, and $R^4$ in Formula (1) to a ring-constituting atom, and preferable binding forms thereof are also the same. That is, $R^{1a}$, $R^{2a}$, $R^{3a}$, and $R^{4a}$ may be bonded to a ring-constituting atom directly or indirectly through the divalent group A. In the present invention, the configuration in which $R^{1a}$, $R^{2a}$, $R^{3a}$, and/or $R^{4a}$ is bonded to a ring-constituting atom through the divalent group A is also included in the structure of Formula (2) or (3).

In the present invention, in one aspect of the compound represented by Formula (2), a configuration in which, in Formula (2), $X^a$ represents a sulfur atom, $Z^a$ represents a sulfur atom, both $R^{1a}$ and $R^{2a}$ represent a hydrogen atom, and both $m^a$ and $n^a$ represent 0 is excluded from the compound represented by Formula (2).

It is more preferable that the compound represented by the formula (2) is a compound represented by the following Formula (4). In addition, it is more preferable that the compound represented by the formula (3) is a compound represented by the following Formula (5).

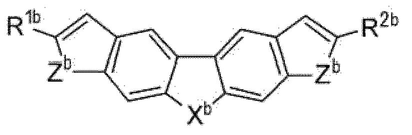

Formula (4)

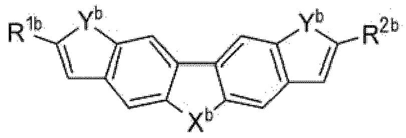

Formula (5)

In Formulae (4) and (5), $X^b$, $Y^b$, and $Z^b$ represent an oxygen atom, a sulfur atom, or a selenium atom.

$R^{1b}$ and $R^{2b}$ have the same definitions and the same preferable configurations as $R^{1a}$ and $R^{2a}$ in Formula (2), respectively. Binding forms of $R^{1b}$ and $R^{2b}$ to a ring-constituting atom are also the same as binding forms of $R^{1a}$ and $R^{2a}$ in Formula (2) to a ring-constituting atom, respectively, and preferable binding forms thereof are also the same. That is, $R^{1b}$ and $R^{2b}$ may be bonded to a ring-constituting atom directly or indirectly through the divalent group A. In the present invention, the configuration in which $R^{1b}$ and/or $R^{2b}$ is bonded to a ring-constituting atom through the divalent group A is also included in the structure of Formula (4) or (5).

In the present invention, in one aspect of the compound represented by Formula (4), a configuration in which, in Formula (4), $X^b$ represents a sulfur atom, $Z^b$ represents a sulfur atom, and both $R^{1b}$ and $R^{2b}$ represent a hydrogen atom is excluded from the compound represented by Formula (2).

In Formulae (4) and (5), it is preferable that $R^{1b}$ and $R^{2b}$ have an aliphatic hydrocarbon group. It is preferable that the aliphatic hydrocarbon group is a linear aliphatic hydrocarbon group. It is preferable that $R^{1b}$ and $R^{2b}$ represent an aryl group having a linear aliphatic hydrocarbon group or a heteroaryl group having a linear aliphatic hydrocarbon group.

The fused heterocyclic compound has the chemical structure represented by Formula (1), but this chemical structure is partially or wholly different from the chemical structure of the low molecular weight organic semiconductor compound. That is, the fused heterocyclic compound and the low molecular weight organic semiconductor compound are different from each other as the organic semiconductor compounds.

Examples of an aspect in which the fused heterocyclic compound and the low molecular weight organic semiconductor compound have different chemical structures include an aspect the fused heterocyclic compound and the

low molecular weight organic semiconductor compound have different mother nuclei (the substituents are the same), an aspect in which the substituents of the fused heterocyclic compound and the low molecular weight organic semiconductor compound are different from each other in the kind, the number of carbon atoms, the number, or the substitution site (the mother nuclei are the same), and an aspect in which the substituents of the fused heterocyclic compound and the low molecular weight organic semiconductor compound have different mother nuclei and different substituents. Here, in the fused heterocyclic compound, the mother nucleus refers to a fused ring structure in which five rings in Formula (1) described below are fused and which does not have a substituent. In addition, in the low molecular weight organic semiconductor compound, the mother nucleus refers to the above-described fused ring structure or a ring structure that includes a repeating unit of the low molecular weight organic semiconductor compound and does not have a substituent. The substituent refers to an atom other than a hydrogen atom or a group that is bonded to the mother nucleus. For example, in the compound represented by Formula (A-1), examples of the substituent include $-L^{a1}-R^{a1}$ and $-L^{a2}-R^{a2}$. For example, in the compound represented by Formula (1), examples of the substituent include $R^1$ to $R^8$.

Among the above-described aspects, from the viewpoints of carrier mobility and hysteresis, an aspect in which the fused heterocyclic compound and the low molecular weight organic semiconductor compound have the same mother nucleus and have different substituents is preferable. That is, it is preferable that the low molecular weight organic semiconductor compound is a compound having the same mother nucleus as the fused heterocyclic compound and having a substituent different from that of the fused heterocyclic compound. In this aspect, the fused heterocyclic compound and the low molecular weight organic semiconductor compound are selected from fused heterocyclic compounds represented by Formula (1) described below. In the aspect, regarding the difference between the substituents, it is preferable that the substituents are the same in the number and the substitution site and are different in the kind and the number of carbon atoms, it is more preferable that the molecular weight (total molecular weight) of the substituent in the low molecular weight organic semiconductor compound is lower than that in the fused heterocyclic compound, and it is still more preferable that the substituents are the same in the kind, the number, and the substitution site but are different in the number (total number) of carbon atoms in the substituent of the low molecular weight organic semiconductor compound is less than and that of the fused heterocyclic compound and the molecular weight (total molecular weight) thereof is lower than that of the fused heterocyclic compound).

Specific examples of the compound represented by Formula (1) will be shown below and in Examples, but the present invention is not limited thereto.

Examples of the compound represented by Formula (1) include specific examples 1 to 458 shown in paragraphs "0053" to "0075" and specific examples 535 to 686 shown in paragraphs "0079" to "0087" of JP2015-195362A.

In addition, examples shown in tables below as the compound represented by Formula (1) can also be used. In the tables below, in a case where $R^1$ and $R^2$ are bonded to a ring-constituting atom through the divalent group A, the columns of $R^1$ and $R^2$ show structures including the divalent group A. A wave line (linked to a straight line) in $R^1$ to $R^4$ represents a binding site. For example, in a specific example 1, both $R^1$ and $R^2$ represent a methyl group. In addition, in a specific example 8, $R^1$ represents an n-hexylcarbonyl group, and $R^2$ represents a dibutylamino group. In the tables below, iPr represents isopropyl, Bu represents butyl, and amyl represents an amyl group.

In the tables below, a case where at least one of m or n represents 1 represents both of an aspect where a carbon atom bonded to $R^3$ or $R^4$ is a carbon atom on the X side (between X and Z) in Formula (1) and an aspect where a carbon atom bonded to $R^3$ or $R^4$ is a carbon atom on the Y side in Formula (1).

| Specific Example | X | Y | Z | m | n | $R^1$ | $R^2$ | $R^3$ | $R^4$ |
|---|---|---|---|---|---|---|---|---|---|
| 1 | O | CH | Se | 0 | 0 | $CH_3$ | $CH_3$ | - | - |
| 2 | O | CH | Se | 0 | 0 | $n-C_{14}H_{29}$ | $n-C_{14}H_{29}$ | - | - |
| 3 | O | CH | Se | 0 | 0 | 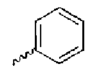 | 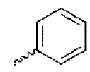 | - | - |
| 4 | O | CH | Se | 0 | 0 | 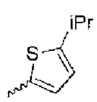 | 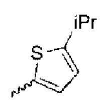 | - | - |
| 5 | O | CH | Se | 1 | 1 | $CH_3$ | $CH_3$ | $CH_3$ | $CH_3$ |
| 6 | O | $C(n-C_8H_{17})$ | Se | 0 | 0 | H | H | - | - |
| 7 | O | $C(n-C_6H_{13})$ | Se | 0 | 0 | $CH_3$ | $CH_3$ | - | - |

(continued)

| Specific Example | X | Y | Z | m | n | $R^1$ | $R^2$ | $R^3$ | $R^4$ |
|---|---|---|---|---|---|---|---|---|---|
| 8 | O | CH | Se | 1 | 1 | 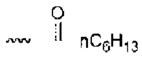 | $N(C_4H_9)_2$ | $OCH_3$ | $OCH_3$ |
| 9 | O | CH | Se | 0 | 0 | 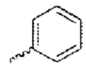 | $n\text{-}C_5H_{11}$ | - | - |
| 10 | O | CH | Se | 2 | 2 | $CH_3$ | $CH_3$ | $CH_3$ | $CH_3$ |

| Specific Example | X | Y | Z | m | n | $R^1$ | $R^2$ | $R^3$ | $R^4$ |
|---|---|---|---|---|---|---|---|---|---|
| 11 | O | O | N | 0 | 0 | $CH_3$ | $CH_3$ | - | - |
| 12 | O | O | N | 0 | 0 | $n\text{-}C_{14}H_{29}$ | $n\text{-}C_{14}H_{29}$ | - | - |
| 13 | O | O | N | 0 | 0 | 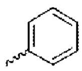 | 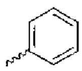 | - | - |
| 14 | O | O | N | 0 | 0 | 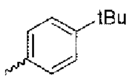 | 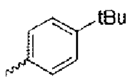 | - | - |
| 15 | O | O | N | 1 | 1 | $CH_3$ | $CH_3$ | $CH_3$ | $CH_3$ |
| 16 | O | O | N | 0 | 0 | 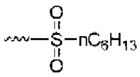 | 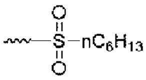 | - | - |
| 17 | O | O | N | 0 | 0 | $CH_3$ | $CH_3$ | - | - |
| 18 | O | O | N | 1 | 1 | 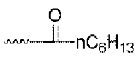 | $\equiv nC_7H_{15}$ | $SCH_3$ | $SCH_3$ |
| 19 | O | O | N | 0 | 0 | 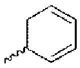 | $n\text{-}C_5H_{11}$ | - | - |
| 20 | O | O | N | 2 | 2 | $CH_3$ | $CH_3$ | $CH_3$ | $CH_3$ |

| Specific Example | X | Y | Z | m | n | $R^1$ | $R^2$ | $R^3$ | $R^4$ |
|---|---|---|---|---|---|---|---|---|---|
| 21 | O | S | N | 0 | 0 | $CH_3$ | $CH_3$ | - | - |
| 22 | O | S | N | 0 | 0 | $n\text{-}C_{14}H_{29}$ | $n\text{-}C_{14}H_{29}$ | - | - |
| 23 | O | S | N | 0 | 0 | 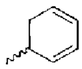 | 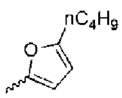 | - | - |
| 24 | O | S | N | 0 | 0 | 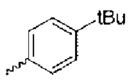 | 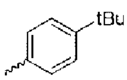 | - | - |
| 25 | O | s | N | 1 | 1 | $CH_3$ | $CH_3$ | $CH_3$ | $CH_3$ |
| 26 | O | S | N | 0 | 0 | $S\text{-}nC_6H_{13}$ | $S\text{-}nC_6H_{13}$ | - | - |

(continued)

| Specific Example | X | Y | Z | m | n | $R^1$ | $R^2$ | $R^3$ | $R^4$ |
|---|---|---|---|---|---|---|---|---|---|
| 27 | O | S | N | 0 | 0 | $CH_3$ | $CH_3$ | - | - |
| 28 | O | S | N | 1 | 1 | 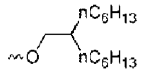 | 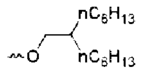 | $SCH_3$ | $SCH_3$ |
| 29 | O | S | N | 0 | 0 | 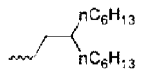 | $n\text{-}C_5H_{11}$ | - | - |
| 30 | O | S | N | 2 | 2 | $CH_3$ | $CH_3$ | $CH_3$ | $CH_3$ |

| Specific Example | X | Y | Z | m | n | $R^1$ | $R^2$ | $R^3$ | $R^4$ |
|---|---|---|---|---|---|---|---|---|---|
| 31 | O | Se | CH | 0 | 0 | $CH_3$ | $CH_3$ | - | - |
| 32 | O | Se | CH | 0 | 0 | $n\text{-}C_{14}H_{29}$ | $n\text{-}C_{14}H_{29}$ | - | - |
| 33 | O | Se | CH | 0 | 0 | 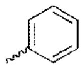 | 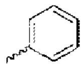 | - | - |
| 34 | O | Se | CH | 0 | 0 | 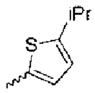 | 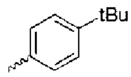 | - | - |
| 35 | O | Se | CH | 1 | 1 | H | H | $n\text{-}C_{14}H_{29}$ | $n\text{-}C_{14}H_{29}$ |
| 36 | O | Se | $C(n\text{-}C_9H_{19})$ | 0 | 0 | 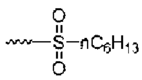 | 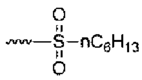 | - | - |
| 37 | O | Se | $C(n\text{-}C_6H_{13})$ | 0 | 0 | $CH_3$ | $CH_3$ | - | - |
| 38 | O | Se | CH | 1 | 1 | 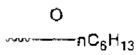 | $N(C_4H_9)_2$ | $OCH_3$ | $OCH_3$ |
| 39 | O | Se | CH | 0 | 0 | 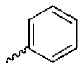 | $n\text{-}C_5H_{11}$ | - | - |
| 40 | O | Se | CH | 2 | 2 | $CH_3$ | $CH_3$ | $CH_3$ | $CH_3$ |

| Specific Example | X | Y | Z | m | n | $R^1$ | $R^2$ | $R^3$ | $R^4$ |
|---|---|---|---|---|---|---|---|---|---|
| 41 | O | Se | N | 0 | 0 | $CH_3$ | $CH_3$ | - | - |
| 42 | O | Se | N | 0 | 0 | $n\text{-}C_{14}H_{29}$ | $n\text{-}C_{14}H_{29}$ | - | - |
| 43 | O | Se | N | 0 | 0 | 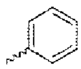 | 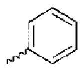 | - | - |
| 44 | O | Se | N | 0 | 0 | 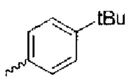 | 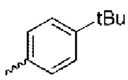 | - | - |
| 45 | O | Se | N | 1 | 1 | $CH_3$ | $CH_3$ | $CH_3$ | $CH_3$ |

(continued)

| Specific Example | X | Y | Z | m | n | $R^1$ | $R^2$ | $R^3$ | $R^4$ |
|---|---|---|---|---|---|---|---|---|---|
| 46 | O | Se | N | 0 | 0 | 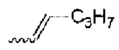 | 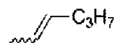 | - | - |
| 47 | O | Se | N | 0 | 0 | $CH_3$ | $CH_3$ | - | - |
| 48 | O | Se | N | 1 | 1 | 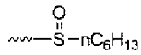 | 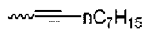 | $SCH_3$ | $SCH_3$ |
| 49 | O | Se | N | 0 | 0 | 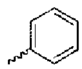 | $n-C_5H_{11}$ | - | - |
| 50 | O | Se | N | 2 | 2 | $CH_3$ | $CH_3$ | $CH_3$ | $CH_3$ |

| Specific Example | X | Y | Z | m | n | $R^1$ | $R^2$ | $R^3$ | $R^4$ |
|---|---|---|---|---|---|---|---|---|---|
| 51 | O | CH | S | 0 | 0 | $CH_3$ | $CH_3$ | - | - |
| 52 | O | CH | S | 0 | 0 | $n-C_{14}H_{29}$ | $n-C_{14}H_{29}$ | - | - |
| 53 | O | CH | S | 0 | 0 | 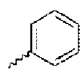 | 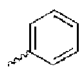 | - | - |
| 54 | O | CH | S | 0 | 0 | 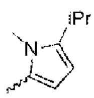 | 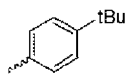 | - | - |
| 55 | O | CH | S | 1 | 1 | H | H | $n-C_{14}H_{29}$ | $n-C_{14}H_{29}$ |
| 56 | O | $C(n-C_9H_{19})$ | S | 0 | 0 | 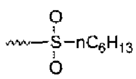 | 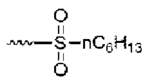 | - | - |
| 57 | O | $C(n-C_6H_{13})$ | $N(CH_3)$ | 0 | 0 | $CH_3$ | $CH_3$ | - | - |
| 58 | O | CH | $N(CH_3)$ | 1 | 1 | 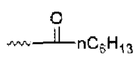 | 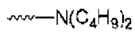 | $OCH_3$ | $OCH_3$ |
| 59 | O | CH | $N(n-C_9H_{19})$ | 0 | 0 | 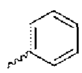 | $n-C_5H_{11}$ | - | - |
| 60 | O | CH | $N(n-C_9H_{19})$ | 2 | 2 | $CH_3$ | $CH_3$ | $CH_3$ | $CH_3$ |

| Specific Example | X | Y | Z | m | n | $R^1$ | $R^2$ | $R^3$ | $R^4$ |
|---|---|---|---|---|---|---|---|---|---|
| 61 | O | O | CH | 0 | 0 | $CH_3$ | $CH_3$ | - | - |
| 62 | O | O | $C(n-C_9H_{19})$ | 0 | 0 | $n-C_{14}H_{29}$ | $n-C_{14}H_{29}$ | - | - |

(continued)

| Specific Example | X | Y | Z | m | n | $R^1$ | $R^2$ | $R^3$ | $R^4$ |
|---|---|---|---|---|---|---|---|---|---|
| 63 | O | O | $C(n\text{-}C_6H_{13})$ | 0 | 0 | 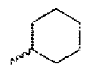 | 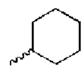 | - | - |
| 64 | O | O | CH | 0 | 0 | 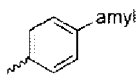 | 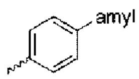 | - | - |
| 65 | O | O | CH | 1 | 1 | $CH_3$ | $CH_3$ | $CH_3$ | $CH_3$ |
| 66 | O | O | $C(n\text{-}C_9H_{19})$ | 0 | 0 | 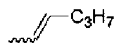 | 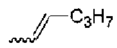 | - | - |
| 67 | O | S | $C(n\text{-}C_6H_{13})$ | 0 | 0 | $CH_3$ | $CH_3$ | - | - |
| 68 | O | S | CH | 1 | 1 | 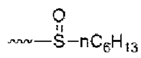 | 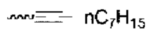 | $SCH_3$ | $SCH_3$ |
| 69 | O | S | CH | 0 | 0 | 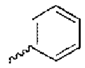 | $n\text{-}C_5H_{11}$ | - | - |
| 70 | O | S | CH | 2 | 2 | $CH_3$ | $CH_3$ | $CH_3$ | $CH_3$ |

| Specific Example | X | Y | Z | m | n | $R^1$ | $R^2$ | $R^3$ | $R^4$ |
|---|---|---|---|---|---|---|---|---|---|
| 71 | O | N | O | 0 | 0 | $CH_3$ | $CH_3$ | - | - |
| 72 | O | N | O | 0 | 0 | $n\text{-}C_{14}H_{29}$ | $n\text{-}C_{14}H_{29}$ | - | - |
| 73 | O | N | O | 0 | 0 | 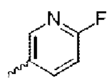 | 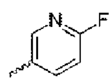 | - | - |
| 74 | O | N | O | 0 | 0 | 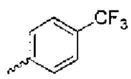 | 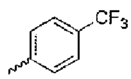 | - | - |
| 75 | O | N | O | 1 | 1 | $CH_3$ | $CH_3$ | $CH_3$ | $CH_3$ |
| 76 | O | N | O | 0 | 0 | 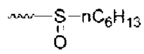 | 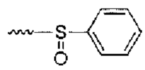 | - | - |
| 77 | O | N | O | 0 | 0 | $CH_3$ | $CH_3$ | - | - |
| 78 | O | N | O | 1 | 1 | 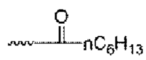 | 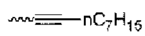 | $SCH_3$ | $SCH_3$ |
| 79 | O | N | O | 0 | 0 | 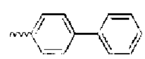 | $n\text{-}C_5H_{11}$ | - | - |
| 80 | O | N | O | 2 | 2 | $CH_3$ | $CH_3$ | $CH_3$ | $CH_3$ |

| Specific Example | X | Y | Z | m | n | $R^1$ | $R^2$ | $R^3$ | $R^4$ |
|---|---|---|---|---|---|---|---|---|---|
| 81 | O | N | S | 0 | 0 | $CH_3$ | $CH_3$ | - | - |
| 82 | O | N | S | 0 | 0 | $n\text{-}C_9H_{19}$ | $n\text{-}C_9H_{19}$ | - | - |

(continued)

| Specific Example | X | Y | Z | m | n | $R^1$ | $R^2$ | $R^3$ | $R^4$ |
|---|---|---|---|---|---|---|---|---|---|
| 83 | O | N | S | 0 | 0 | 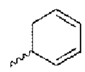 | 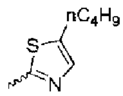 | - | - |
| 84 | O | N | S | 0 | 0 | 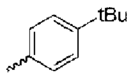 | 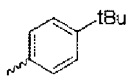 | - | - |
| 85 | O | N | S | 1 | 1 | $CH_3$ | $CH_3$ | $CH_3$ | $CH_3$ |
| 86 | O | N | S | 0 | 0 | $-S-nC_6H_{13}$ | $S\ nC_6H_{13}$ | - | - |
| 87 | O | N | S | 0 | 0 | $CH_3$ | $CH_3$ | - | - |
| 88 | O | N | S | 1 | 2 | 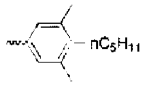 | 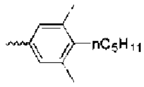 | $SCH_3$ | $SCH_3$ |
| 89 | O | N | S | 0 | 0 | 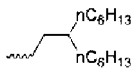 | $n-C_5H_{11}$ | - | - |
| 90 | O | N | Se | 0 | 0 | $CH_3$ | $CH_3$ | - | - |

| Specific Example | X | Y | Z | m | n | $R^1$ | $R^2$ | $R^3$ | $R^4$ |
|---|---|---|---|---|---|---|---|---|---|
| 91 | O | N | Se | 0 | 0 | $n-C_{14}H_{29}$ | $n-C_{14}H_{29}$ | - | - |
| 92 | O | N | Se | 0 | 0 | 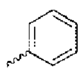 | 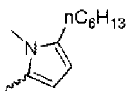 | - | - |
| 93 | O | N | Se | 0 | 0 | 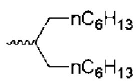 | 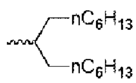 | - | - |
| 94 | O | N | Se | 1 | 1 | H | H | $n-C_{14}H_{29}$ | $n-C_{14}H_{29}$ |
| 95 | O | N | Se | 0 | 0 | 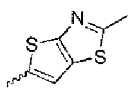 | 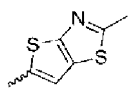 | - | - |
| 96 | O | N | Se | 0 | 0 | $CH_3$ | $CH_3$ | - | - |
| 97 | O | N | Se | 1 | 1 | 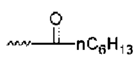 | $-N(C_4H_9)_2$ | $OCH_3$ | $OCH_3$ |
| 98 | O | N | Se | 0 | 0 | 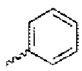 | $n-C_5H_{11}$ | - | - |
| 99 | O | $N(CH_3)$ | CH | 0 | 0 | $CH_3$ | $CH_3$ | - | - |
| 100 | O | NH | CH | 0 | 0 | $n-C_{14}H_{29}$ | $n-C_{14}H_{29}$ | - | - |

| Specific Example | X | Y | Z | m | n | $R^1$ | $R^2$ | $R^3$ | $R^4$ |
|---|---|---|---|---|---|---|---|---|---|
| 101 | O | $N(CH_3)$ | CH | 0 | 0 | 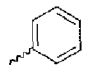 | 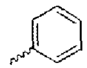 | - | - |
| 102 | O | $N(CH_3)$ | CH | 0 | 0 | 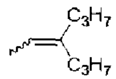 | 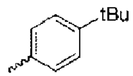 | - | - |
| 103 | O | $N(CH_3)$ | CH | 1 | 1 | H | H | $n\text{-}C_{14}H_{29}$ | $n\text{-}C_{14}H_{29}$ |
| 104 | O | $N(CH_3)$ | $C(n\text{-}C_9H_{19})$ | 0 | 0 | $-O-nC_6H_{13}$ | $-O-nC_6H_{13}$ | - | - |
| 105 | O | $N(n\text{-}C_9H_{19})$ | $C(n\text{-}C_6H_{13})$ | 0 | 0 | $CH_3$ | $CH_3$ | - | - |
| 106 | O | $N(CH_3)$ | CH | 1 | 1 | 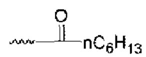 | $-N(C_4H_9)_2$ | $OCH_3$ | $OCH_3$ |
| 107 | O | N(i-Pr) | CH | 0 | 0 | 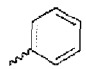 | $n\text{-}C_5H_{11}$ | - | - |
| 108 | O | $N(CH_3)$ | CH | 2 | 2 | $CH_3$ | $CH_3$ | $CH_3$ | $CH_3$ |
| 109 | S | CH | Se | 0 | 0 | $CH_3$ | $CH_3$ | - | - |
| 110 | S | CH | Se | 0 | 0 | $n\text{-}C_{14}H_{29}$ | $n\text{-}C_{14}H_{29}$ | - | - |

| Specific Example | X | Y | Z | m | n | R1 | R2 | R3 | R4 |
|---|---|---|---|---|---|---|---|---|---|
| 111 | S | CH | Se | 0 | 0 | 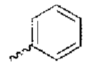 | 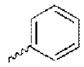 | - | - |
| 112 | S | CH | Se | 0 | 0 | 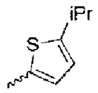 | 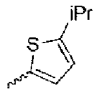 | - | - |
| 113 | S | CH | Se | 1 | 1 | $CH_3$ | $CH_3$ | $CH_3$ | $CH_3$ |
| 114 | S | $C(n\text{-}C_8H_{17})$ | Se | 0 | 0 | H | H | - | - |
| 115 | S | $C(n\text{-}C_6H_{13})$ | Se | 0 | 0 | $CH_3$ | $CH_3$ | - | - |
| 116 | S | CH | Se | 1 | 1 | 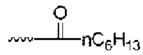 | $N(C_4H_9)_2$ | $OCH_3$ | $OCH_3$ |
| 117 | S | CH | Se | 0 | 0 | 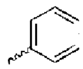 | $n\text{-}C_5H_{11}$ | - | - |
| 118 | S | CH | $N(CH_3)$ | 2 | 2 | $CH_3$ | $CH_3$ | $CH_3$ | $CH_3$ |
| 119 | S | O | N | 0 | 0 | $CH_3$ | $CH_3$ | - | - |
| 120 | S | O | N | 0 | 0 | $n\text{-}C_{14}H_{29}$ | $n\text{-}C_{14}H_{29}$ | - | - |

| Specific Example | X | Y | Z | m | n | R1 | R2 | R3 | R4 |
|---|---|---|---|---|---|---|---|---|---|
| 121 | S | O | N | 0 | 0 | 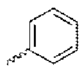 | 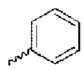 | - | - |
| 122 | S | O | N | 0 | 0 | 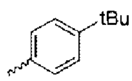 | 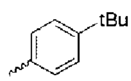 | - | - |
| 123 | S | O | N | 1 | 1 | $CH_3$ | $CH_3$ | $CH_3$ | $CH_3$ |
| 124 | S | O | N | 0 | 0 | 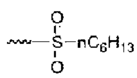 | 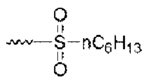 | - | - |
| 125 | S | O | N | 0 | 0 | $CH_3$ | $CH_3$ | - | - |
| 126 | S | O | N | 1 | 1 | 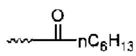 | 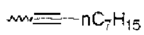 | $SCH_3$ | $SCH_3$ |
| 127 | S | O | N | 0 | 0 | 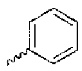 | $n\text{-}C_5H_{11}$ | - | - |
| 128 | S | O | N | 2 | 2 | $CH_3$ | $CH_3$ | $CH_3$ | $CH_3$ |
| 129 | S | S | N | 0 | 0 | $CH_3$ | $CH_3$ | - | - |
| 130 | S | S | N | 0 | 0 | $n\text{-}C_9H_{19}$ | $n\text{-}C_9H_{19}$ | - | - |

| Specific Example | X | Y | Z | m | n | $R^1$ | $R^2$ | $R^3$ | $R^4$ |
|---|---|---|---|---|---|---|---|---|---|
| 131 | S | S | N | 0 | 0 | 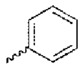 | 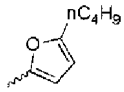 | - | - |
| 132 | S | S | N | 0 | 0 | 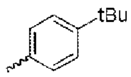 | 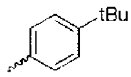 | - | - |
| 133 | S | S | N | 1 | 1 | $CH_3$ | $CH_3$ | $CH_3$ | $CH_3$ |
| 134 | S | S | N | 0 | 0 | $-S-nC_6H_{13}$ | $-S-nC_6H_{13}$ | - | - |
| 135 | S | S | N | 0 | 0 | $CH_3$ | $CH_3$ | - | - |
| 136 | S | S | N | 1 | 1 | 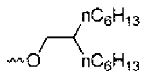 | 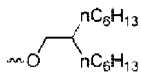 | $SCH_3$ | $SCH_3$ |
| 137 | S | S | N | 0 | 0 | 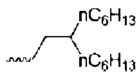 | $n-C_5H_{11}$ | - | - |
| 138 | S | S | N | 2 | 2 | $CH_3$ | $CH_3$ | $CH_3$ | $CH_3$ |
| 139 | S | Se | CH | 0 | 0 | $CH_3$ | $CH_3$ | - | - |
| 140 | S | Se | CH | 0 | 0 | $n-C_{14}H_{29}$ | $n-C_{14}H_{29}$ | - | - |

| Specific Example | X | Y | Z | m | n | $R^1$ | $R^2$ | $R^3$ | $R^4$ |
|---|---|---|---|---|---|---|---|---|---|
| 141 | S | Se | CH | 0 | 0 | 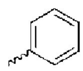 | 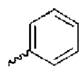 | - | - |
| 142 | S | Se | CH | 0 | 0 | 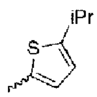 | 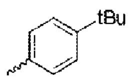 | - | - |
| 143 | S | Se | CH | 1 | 1 | H | H | $n-C_{14}H_{29}$ | $n-C_{14}H_{29}$ |
| 144 | S | Se | $C(n-C_9H_{19})$ | 0 | 0 | 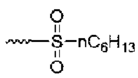 | 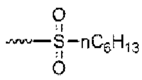 | - | - |
| 145 | S | Se | $C(n-C_6H_{13})$ | 0 | 0 | $CH_3$ | $CH_3$ | - | - |
| 146 | S | Se | CH | 1 | 1 | 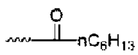 | $-N(C_4H_9)_2$ | $OCH_3$ | $OCH_3$ |
| 147 | S | Se | CH | 0 | 0 | 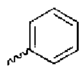 | $n-C_5H_{11}$ | - | - |
| 148 | S | Se | CH | 2 | 2 | $CH_3$ | $CH_3$ | $CH_3$ | $CH_3$ |
| 149 | S | Se | N | 0 | 0 | $CH_3$ | $CH_3$ | - | - |
| 150 | S | Se | N | 0 | 0 | $n-C_{14}H_{29}$ | $n-C_{14}H_{29}$ | - | - |

| Specific Example | X | Y | Z | m | n | $R^1$ | $R^2$ | $R^3$ | $R^4$ |
|---|---|---|---|---|---|---|---|---|---|
| 151 | S | Se | N | 0 | 0 | 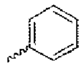 | 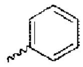 | - | - |
| 152 | S | Se | N | 0 | 0 | 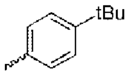 | 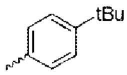 | - | - |
| 153 | S | Se | N | 1 | 1 | $CH_3$ | $CH_3$ | $CH_3$ | $CH_3$ |
| 154 | S | Se | N | 0 | 0 | 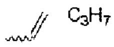 | 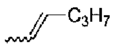 | - | - |
| 155 | S | Se | N | 0 | 0 | $CH_3$ | $CH_3$ | - | - |
| 156 | S | Se | N | 1 | 1 | 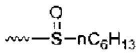 | 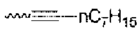 | $SCH_3$ | $SCH_3$ |
| 157 | S | Se | N | 0 | 0 | 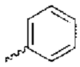 | n-$C_5H_{11}$ | - | - |
| 158 | S | Se | N | 2 | 2 | $CH_3$ | $CH_3$ | $CH_3$ | $CH_3$ |
| 159 | S | N | O | 0 | 0 | $CH_3$ | $CH_3$ | - | - |
| 160 | S | N | O | 0 | 0 | n-$C_{14}H_{29}$ | n-$C_{14}H_{29}$ | - | - |

| Specific Example | X | Y | Z | m | n | $R^1$ | $R^2$ | $R^3$ | $R^4$ |
|---|---|---|---|---|---|---|---|---|---|
| 161 | S | N | O | 0 | 0 | 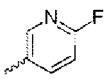 | 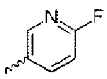 | - | - |
| 162 | S | N | O | 0 | 0 | 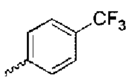 | 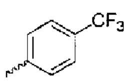 | - | - |
| 163 | S | N | O | 1 | 1 | $CH_3$ | $CH_3$ | $CH_3$ | $CH_3$ |
| 164 | S | N | O | 0 | 0 | 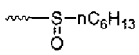 | 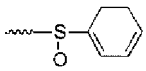 | - | - |
| 165 | S | N | O | 0 | 0 | $CH_3$ | $CH_3$ | - | - |
| 166 | S | N | O | 1 | 1 | 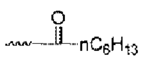 | 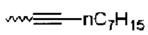 | $SCH_3$ | $SCH_3$ |
| 167 | S | N | O | 0 | 0 | 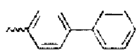 | n-$C_5H_{11}$ | - | - |
| 168 | S | N | O | 2 | 2 | $CH_3$ | $CH_3$ | $CH_3$ | $CH_3$ |
| 169 | S | N | S | 0 | 0 | $CH_3$ | $CH_3$ | - | - |
| 170 | S | N | S | 0 | 0 | n-$C_9H_{19}$ | n-$C_9H_{19}$ | - | - |

| Specific Example | X | Y | Z | m | n | $R^1$ | $R^2$ | $R^3$ | $R^4$ |
|---|---|---|---|---|---|---|---|---|---|
| 171 | S | N | S | 0 | 0 | 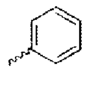 | 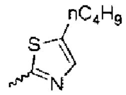 | - | - |
| 172 | S | N | S | 0 | 0 | 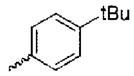 | 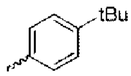 | - | - |
| 173 | S | N | S | 1 | 1 | $CH_3$ | $CH_3$ | $CH_3$ | $CH_3$ |
| 174 | S | N | S | 0 | 0 | $S-nC_6H_{13}$ | $S-nC_6H_{13}$ | - | - |
| 175 | S | N | S | 0 | 0 | $CH_3$ | $CH_3$ | - | - |
| 176 | S | N | S | 1 | 2 | 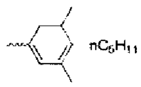 | 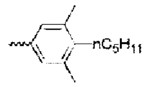 | $SCH_3$ | $SCH_3$ |
| 177 | S | N | S | 0 | 0 | 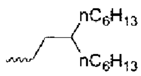 | $n-C_5H_{11}$ | - | - |
| 178 | S | N | Se | 0 | 0 | $CH_3$ | $CH_3$ | - | - |
| 179 | S | N | Se | 0 | 0 | $n-C_{14}H_{29}$ | $n-C_{14}H_{29}$ | - | - |
| 180 | S | N | Se | 0 | 0 | 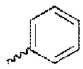 | 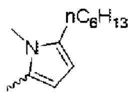 | - | - |

| Specific Example | X | Y | z | m | n | $R^1$ | $R^2$ | $R^3$ | $R^4$ |
|---|---|---|---|---|---|---|---|---|---|
| 181 | S | N | Se | 0 | 0 | 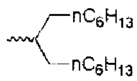 | 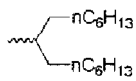 | - | - |
| 182 | S | N | Se | 1 | 1 | H | H | $n-C_{14}H_{29}$ | $n-C_{14}H_{29}$ |
| 183 | S | N | Se | 0 | 0 | 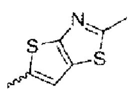 | 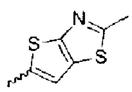 | - | - |
| 184 | S | N | Se | 0 | 0 | $CH_3$ | $CH_3$ | - | - |
| 185 | S | N | Se | 1 | 1 | 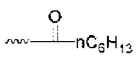 | $N(C_4H_9)_2$ | $OCH_3$ | $OCH_3$ |
| 186 | S | N | Se | 0 | 0 | 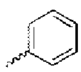 | $n-C_5H_{11}$ | - | - |
| 187 | S | $N(CH_3)$ | CH | 0 | 0 | $CH_3$ | $CH_3$ | - | - |

(continued)

| Specific Example | X | Y | z | m | n | $R^1$ | $R^2$ | $R^3$ | $R^4$ |
|---|---|---|---|---|---|---|---|---|---|
| 188 | S | NH | CH | 0 | 0 | 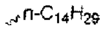 | 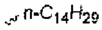 | - | - |
| 189 | S | $N(CH_3)$ | CH | 0 | 0 | 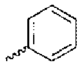 | 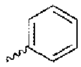 | - | - |
| 190 | S | $N(CH_3)$ | CH | 0 | 0 | 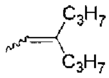 | 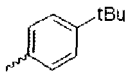 | - | - |

| Specific Example | X | Y | Z | m | n | $R^1$ | $R^2$ | $R^3$ | $R^4$ |
|---|---|---|---|---|---|---|---|---|---|
| 191 | S | $N(CH_3)$ | CH | 1 | 1 | H | H | $n-C_{14}H_{29}$ | $n-C_{14}H_{29}$ |
| 192 | S | $N(CH_3)$ | $C[n-C_9H_{19})$ | 0 | 0 | $-O-nC_6H_{13}$ | $-O-nC_6H_{13}$ | - | - |
| 193 | S | $N(n-C_9H_{19})$ | $C(n-C_6H_{13})$ | 0 | 0 | $CH_3$ | $CH_3$ | - | - |
| 194 | S | $N(CH_3)$ | CH | 1 | 1 | 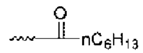 | $-N(C_4H_9)_2$ | $OCH_3$ | $OCH_3$ |
| 195 | S | N(i-Pr) | CH | 0 | 0 | 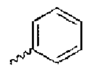 | $n-C_5H_{11}$ | - | - |
| 196 | S | $N(CH_3)$ | CH | 2 | 2 | $CH_3$ | $CH_3$ | $CH_3$ | $CH_3$ |
| 197 | Se | CH | Se | 0 | 0 | $CH_3$ | $CH_3$ | - | - |
| 198 | Se | CH | Se | 0 | 0 | $n-C_{14}H_{29}$ | $n-C_{14}H_{29}$ | - | - |
| 199 | Se | CH | Se | 0 | 0 | 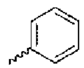 | 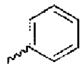 | - | - |
| 200 | Se | CH | Se | 0 | 0 | 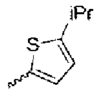 | 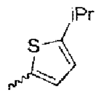 | - | - |

| Specific Example | X | Y | Z | m | n | $R^1$ | $R^2$ | $R^3$ | $R^4$ |
|---|---|---|---|---|---|---|---|---|---|
| 201 | Se | CH | Se | 1 | 1 | $CH_3$ | $CH_3$ | $CH_3$ | $CH_3$ |
| 202 | Se | C(n-$C_8H_{17}$) | Se | 0 | 0 | H | H | - | - |
| 203 | Se | C(n-$C_6H_{13}$) | Se | 0 | 0 | $CH_3$ | $CH_3$ | - | - |
| 204 | Se | CH | Se | 1 | 1 | 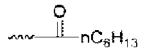 | —$N(C_4H_9)_2$ | $OCH_3$ | $OCH_3$ |
| 205 | Se | CH | Se | 0 | 0 | 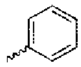 | n-$C_5H_{11}$ | - | - |
| 206 | Se | CH | Se | 2 | 2 | $CH_3$ | $CH_3$ | $CH_3$ | $CH_3$ |
| 207 | Se | O | N | 0 | 0 | $CH_3$ | $CH_3$ | - | - |
| 208 | Se | O | N | 0 | 0 | n-$C_{14}H_{29}$ | n-$C_{14}H_{29}$ | - | - |
| 209 | Se | O | N | 0 | 0 | 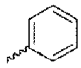 | 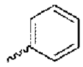 | - | - |
| 210 | Se | O | N | 0 | 0 | 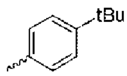 | 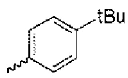 | - | - |

| Specific Example | X | Y | Z | m | n | $R^1$ | $R^2$ | $R^3$ | $R^4$ |
|---|---|---|---|---|---|---|---|---|---|
| 211 | Se | O | N | 1 | 1 | $CH_3$ | $CH_3$ | $CH_3$ | $CH_3$ |
| 212 | Se | O | N | 0 | 0 | 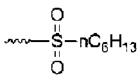 | 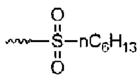 | - | - |
| 213 | Se | O | N | 0 | 0 | $CH_3$ | $CH_3$ | - | - |
| 214 | Se | O | N | 1 | 1 | 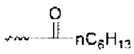 | 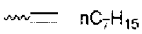 | $SCH_3$ | $SCH_3$ |
| 215 | Se | O | N | 0 | 0 | 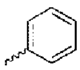 | n-$C_5H_{11}$ | - | - |
| 216 | Se | O | N | 2 | 2 | $CH_3$ | $CH_3$ | $CH_3$ | $CH_3$ |
| 217 | Se | S | N | 0 | 0 | $CH_3$ | $CH_3$ | - | - |
| 218 | Se | S | N | 0 | 0 | n-$C_9H_{19}$ | n-$C_9H_{19}$ | - | - |
| 219 | Se | S | N | 0 | 0 | 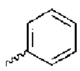 | 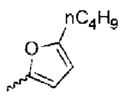 | - | - |
| 220 | Se | S | N | 0 | 0 | 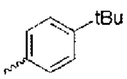 | 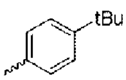 | - | - |

| Specific Example | X | Y | Z | m | n | $R^1$ | $R^2$ | $R^3$ | $R^4$ |
|---|---|---|---|---|---|---|---|---|---|
| 221 | Se | S | N | 1 | 1 | $CH_3$ | $CH_3$ | $CH_3$ | $CH_3$ |
| 222 | Se | S | N | 0 | 0 | $-S-nC_6H_{13}$ | $-S-nC_6H_{13}$ | - | - |
| 223 | Se | S | N | 0 | 0 | $CH_3$ | $CH_3$ | - | - |
| 224 | Se | S | N | 1 | 1 | 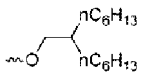 | 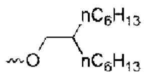 | $SCH_3$ | $SCH_3$ |
| 225 | Se | S | N | 0 | 0 | 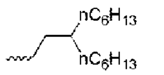 | $n-C_5H_{11}$ | - | - |
| 226 | Se | S | N | 2 | 2 | $CH_3$ | $CH_3$ | $CH_3$ | $CH_3$ |
| 227 | Se | Se | CH | 0 | 0 | $CH_3$ | $CH_3$ | - | - |
| 228 | Se | Se | CH | 0 | 0 | $n-C_{14}H_{29}$ | $n-C_{14}H_{29}$ | - | - |
| 229 | Se | Se | CH | 0 | 0 | 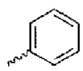 | 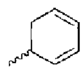 | - | - |
| 230 | Se | Se | CH | 0 | 0 | 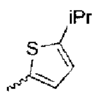 | 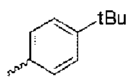 | - | - |

| Specific Example | x | Y | Z | m | n | $R^1$ | $R^2$ | $R^3$ | $R^4$ |
|---|---|---|---|---|---|---|---|---|---|
| 231 | Se | Se | CH | 1 | 1 | H | H | $n-C_{14}H_{29}$ | $n-C_{14}H_{29}$ |
| 232 | Se | Se | $C(n-C_9H_{19})$ | 0 | 0 | 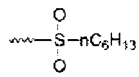 | 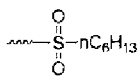 | - | - |
| 233 | Se | Se | $C(n-C_6H_{13})$ | 0 | 0 | $CH_3$ | $CH_3$ | - | - |
| 234 | Se | Se | CH | 1 | 1 | 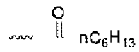 | $N(C_4H_9)_2$ | $OCH_3$ | $OCH_3$ |
| 235 | Se | Se | CH | 0 | 0 | 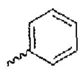 | $n-C_5H_{11}$ | - | - |
| 236 | Se | Se | CH | 2 | 2 | $CH_3$ | $CH_3$ | $CH_3$ | $CH_3$ |
| 237 | Se | Se | N | 0 | 0 | $CH_3$ | $CH_3$ | - | - |
| 238 | Se | Se | N | 0 | 0 | $n-C_{14}H_{29}$ | $n-C_{14}H_{29}$ | - | - |
| 239 | Se | Se | N | 0 | 0 | 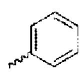 | 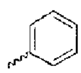 | - | - |
| 240 | Se | Se | N | 0 | 0 | 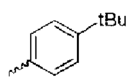 | 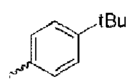 | - | - |

| Specific Example | X | Y | Z | m | n | $R^1$ | $R^2$ | $R^3$ | $R^4$ |
|---|---|---|---|---|---|---|---|---|---|
| 241 | Se | Se | N | 1 | 1 | $CH_3$ | $CH_3$ | $CH_3$ | $CH_3$ |
| 242 | Se | Se | N | 0 | 0 | 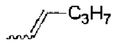 | 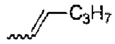 | - | - |
| 243 | Se | Se | N | 0 | 0 | $CH_3$ | $CH_3$ | - | - |
| 244 | Se | Se | N | 1 | 1 | 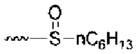 | 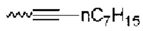 | $SCH_3$ | $SCH_3$ |
| 245 | Se | Se | N | 0 | 0 | 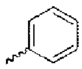 | $n\text{-}C_5H_{11}$ | - | - |
| 246 | Se | Se | N | 2 | 2 | $CH_3$ | $CH_3$ | $CH_3$ | $CH_3$ |
| 247 | Se | CH | O | 0 | 0 | $CH_3$ | $CH_3$ | - | - |
| 248 | Se | CH | O | 0 | 0 | $n\text{-}C_{14}H_{29}$ | $n\text{-}C_{14}H_{29}$ | - | - |
| 249 | Se | CH | O | 0 | 0 | 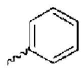 | 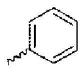 | - | - |
| 250 | Se | CH | O | 0 | 0 | 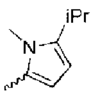 | 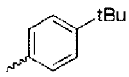 | - | - |

| Specific Example | X | Y | Z | m | n | $R^1$ | $R^2$ | $R^3$ | $R^4$ |
|---|---|---|---|---|---|---|---|---|---|
| 251 | Se | CH | O | 1 | 1 | H | H | $n\text{-}C_{14}H_{29}$ | $n\text{-}C_{14}H_{29}$ |
| 252 | Se | $C(n\text{-}C_9H_{19})$ | O | 0 | 0 | 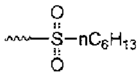 | 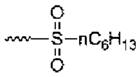 | - | - |
| 253 | Se | $C(n\text{-}C_6H_{13})$ | O | 0 | 0 | $CH_3$ | $CH_3$ | - | - |
| 254 | Se | CH | O | 1 | 1 | 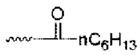 | $N(C_4H_9)_2$ | $OCH_3$ | $OCH_3$ |
| 255 | Se | CH | O | 0 | 0 | 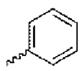 | $n\text{-}C_5H_{11}$ | - | - |
| 256 | Se | CH | O | 2 | 2 | $CH_3$ | $CH_3$ | $CH_3$ | $CH_3$ |
| 257 | Se | O | CH | 0 | 0 | $CH_3$ | $CH_3$ | - | - |
| 258 | Se | O | CH | 0 | 0 | $n\text{-}C_{14}H_{29}$ | $n\text{-}C_{14}H_{29}$ | - | - |
| 259 | Se | O | CH | 0 | 0 | 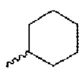 | 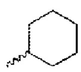 | - | - |
| 260 | Se | O | $C(n\text{-}C_9H_{19})$ | 0 | 0 | 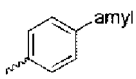 | 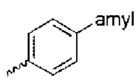 | - | - |

| Specific Example | X | Y | Z | m | n | $R^1$ | $R^2$ | $R^3$ | $R^4$ |
|---|---|---|---|---|---|---|---|---|---|
| 261 | Se | S | $C(n-C_6H_{13})$ | 1 | 1 | 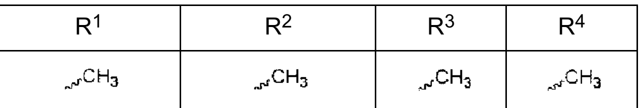 | | | |
| 262 | Se | S | CH | 0 | 0 | 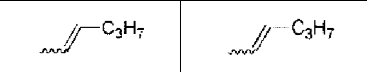 | | - | - |
| 263 | Se | S | CH | 0 | 0 | 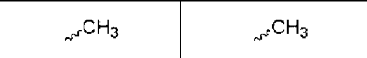 | | - | - |
| 264 | Se | S | $C(n-C_9H_{19})$ | 1 | 1 | 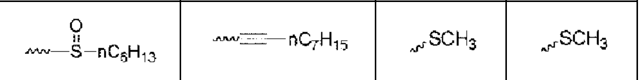 | | | |
| 265 | Se | S | $C(CH_3)$ | 0 | 0 | 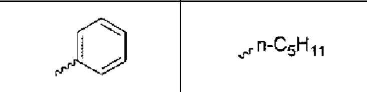 | | - | - |
| 266 | Se | S | $C(n-C_6H_{13})$ | 2 | 2 | 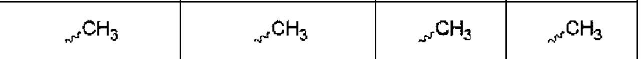 | | | |
| 267 | Se | N | O | 0 | 0 | 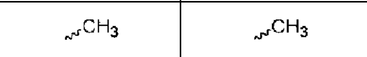 | | - | - |
| 268 | Se | N | O | 0 | 0 | 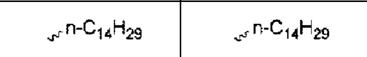 | | - | - |
| 269 | Se | N | O | 0 | 0 | 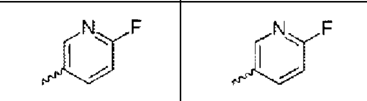 | | - | - |
| 270 | Se | N | O | 0 | 0 | 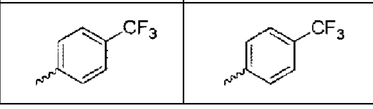 | | - | - |

| Specific Example | X | Y | Z | m | n | $R^1$ | $R^2$ | $R^3$ | $R^4$ |
|---|---|---|---|---|---|---|---|---|---|
| 271 | Se | N | O | 1 | 1 | 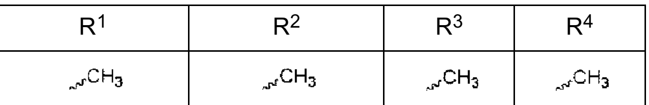 | | | |
| 272 | Se | N | O | 0 | 0 | 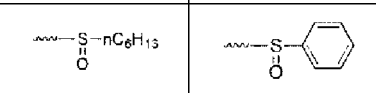 | | - | - |
| 273 | Se | N | O | 0 | 0 | 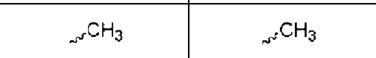 | | - | - |
| 274 | Se | N | O | 1 | 1 | 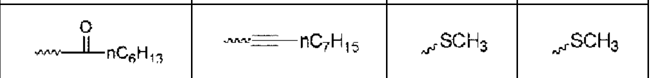 | | | |
| 275 | Se | N | O | 0 | 0 | 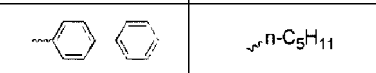 | | - | - |
| 276 | Se | N | O | 2 | 2 | 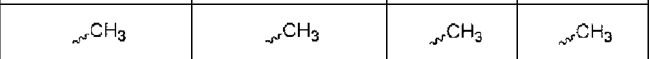 | | | |
| 277 | Se | N | S | 0 | 0 | 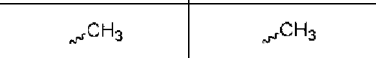 | | - | - |
| 278 | Se | N | S | 0 | 0 | 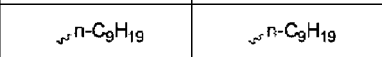 | | - | - |
| 279 | Se | N | S | 0 | 0 | 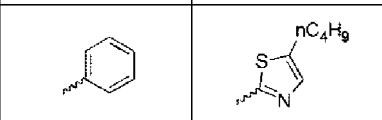 | | - | - |
| 280 | Se | N | S | 0 | 0 | 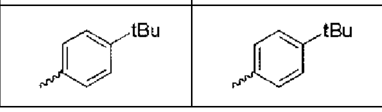 | | - | - |

| Specific Example | X | Y | Z | m | n | R1 | R2 | R3 | R4 |
|---|---|---|---|---|---|---|---|---|---|
| 281 | Se | N | S | 1 | 1 | $CH_3$ | $CH_3$ | $CH_3$ | $CH_3$ |
| 282 | Se | N | S | 0 | 0 | $-S-nC_6H_{13}$ | $-S-nC_6H_{13}$ | - | - |
| 283 | Se | N | S | 0 | 0 | $CH_3$ | $CH_3$ | - | - |
| 284 | Se | N | S | 1 | 2 | 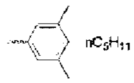 | 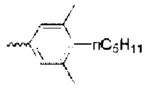 | $SCH_3$ | $SCH_3$ |
| 285 | Se | N | S | 0 | 0 | 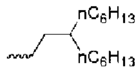 | $n\text{-}C_5H_{11}$ | - | - |
| 286 | Se | N | Se | 0 | 0 | $CH_3$ | $CH_3$ | - | - |
| 287 | Se | N | Se | 0 | 0 | $n\text{-}C_{14}H_{29}$ | $n\text{-}C_{14}H_{29}$ | - | - |
| 288 | Se | N | Se | 0 | 0 | 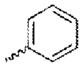 | 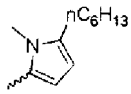 | - | - |
| 289 | Se | N | Se | 0 | 0 | 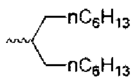 | 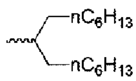 | - | - |
| 290 | Se | N | Se | 1 | 1 | H | H | $n\text{-}C_{14}H_{29}$ | $n\text{-}C_{14}H_{29}$ |

| Specific Example | X | Y | Z | m | n | R1 | R2 | R3 | R4 |
|---|---|---|---|---|---|---|---|---|---|
| 291 | Se | N | Se | 0 | 0 | 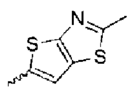 | 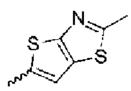 | - | - |
| 292 | Se | N | Se | 0 | 0 | $CH_3$ | $CH_3$ | - | - |
| 293 | Se | N | Se | 1 | 1 | 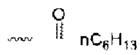 | $-N(C_4H_9)_2$ | $OCH_3$ | $OCH_3$ |
| 294 | Se | N | Se | 0 | 0 | 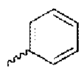 | $n\text{-}C_5H_{11}$ | - | - |
| 295 | Se | N | $N(CH_3)$ | 2 | 2 | $CH_3$ | $CH_3$ | $CH_3$ | $CH_3$ |
| 296 | Se | N | NH | 0 | 0 | $C_2H_5$ | $C_2H_5$ | - | - |
| 297 | Se | N | $N(CH_3)$ | 0 | 0 | $n\text{-}C_{14}H_{29}$ | $n\text{-}C_{14}H_{29}$ | - | - |
| 298 | Se | N | $N(CH_3)$ | 0 | 0 | 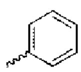 | 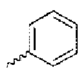 | - | - |
| 299 | Se | N | $N(CH_3)$ | 0 | 0 | 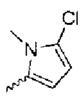 | 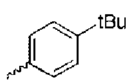 | - | - |
| 300 | Se | N | $N(CH_3)$ | 1 | 1 | H | H | $n\text{-}C_{14}H_{29}$ | $n\text{-}C_{14}H_{29}$ |

| Specific Example | X | Y | Z | m | n | $R^1$ | $R^2$ | $R^3$ | $R^4$ |
|---|---|---|---|---|---|---|---|---|---|
| 301 | Se | N | $N(n\text{-}C_9H_{19})$ | 0 | 0 | 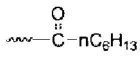 | 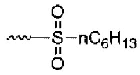 | - | - |
| 302 | Se | N | $N(CH_3)$ | 0 | 0 | $CH_3$ | $CH_3$ | - | - |
| 303 | Se | N | N(i-Pr) | 1 | 1 | 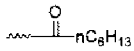 | $N(C_4H_9)_2$ | $OCH_3$ | $OCH_3$ |
| 304 | Se | N | $N(CH_3)$ | 0 | 0 | 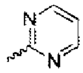 | $n\text{-}C_5H_{11}$ | - | - |
| 305 | Se | N | $N(CH_3)$ | 2 | 2 | $CH_3$ | $CH_3$ | $CH_3$ | $CH_3$ |
| 306 | Se | $N(CH_3)$ | N | 0 | 0 | $CH_3$ | $CH_3$ | - | - |
| 307 | Se | NH | N | 0 | 0 | $n\text{-}C_{14}H_{29}$ | $n\text{-}C_{14}H_{29}$ | - | - |
| 308 | Se | $N(CH_3)$ | N | 0 | 0 | 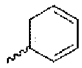 | 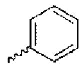 | - | - |
| 309 | Se | $N(CH_3)$ | N | 0 | 0 | 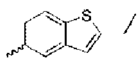 | 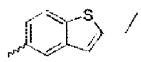 | - | - |
| 310 | Se | $N(CH_3)$ | N | 1 | 1 | $CH_3$ | $CH_3$ | $CH_3$ | $CH_3$ |

| Specific Example | X | Y | Z | m | n | $R^1$ | $R^2$ | $R^3$ | $R^4$ |
|---|---|---|---|---|---|---|---|---|---|
| 311 | Se | $N(CH_3)$ | N | 0 | 0 | 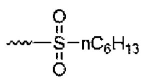 | 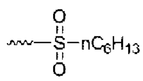 | - | - |
| 312 | Se | $N(n\text{-}C_9H_{19})$ | N | 0 | 0 | $CH_3$ | $CH_3$ | - | - |
| 313 | Se | $N(CH_3)$ | N | 1 | 1 | 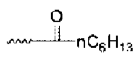 | 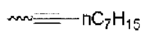 | 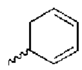 | 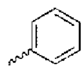 |
| 314 | Se | N(i-Pr) | N | 0 | 0 | 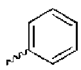 | $n\text{-}C_5H_{11}$ | - | - |
| 315 | Se | $N(CH_3)$ | N | 2 | 2 | $CH_3$ | $CH_3$ | $CH_3$ | $CH_3$ |
| 316 | Se | $N(CH_3)$ | CH | 0 | 0 | $CH_3$ | $CH_3$ | - | - |
| 317 | Se | NH | CH | 0 | 0 | $n\text{-}C_{14}H_{29}$ | $n\text{-}C_{14}H_{29}$ | - | - |
| 318 | Se | $N(CH_3)$ | CH | 0 | 0 | 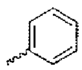 | 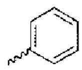 | - | - |
| 319 | Se | $N(CH_3)$ | CH | 0 | 0 | 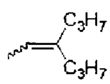 | 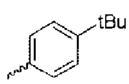 | - | - |
| 320 | Se | $N(CH_3)$ | CH | 1 | 1 | H | H | $n\text{-}C_{14}H_{29}$ | $n\text{-}C_{14}H_{29}$ |

| Specific Example | X | Y | Z | m | n | $R^1$ | $R^2$ | $R^3$ | $R^4$ |
|---|---|---|---|---|---|---|---|---|---|
| 321 | Se | $N(CH_3)$ | $C(n-C_9H_{19})$ | 0 | 0 | $-O-nC_6H_{13}$ | $-O-nC_6H_{13}$ | - | - |
| 322 | Se | $N(n-C_9H_{19})$ | $C(n-C_6H_{13})$ | 0 | 0 | $CH_3$ | $CH_3$ | - | - |
| 323 | Se | $N(CH_3)$ | CH | 1 | 1 | 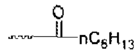 | $-N(C_4H_9)_2$ | $OCH_3$ | $OCH_3$ |
| 324 | Se | N(i-Pr) | CH | 0 | 0 | 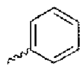 | $n-C_5H_{11}$ | - | - |
| 325 | Se | $N(CH_3)$ | CH | 2 | 2 | $CH_3$ | $CH_3$ | $CH_3$ | $CH_3$ |
| 326 | Te | CH | Se | 0 | 0 | $CH_3$ | $CH_3$ | - | - |
| 327 | Te | CH | Se | 0 | 0 | $n-C_{14}H_{29}$ | $n-C_{14}H_{29}$ | - | - |
| 328 | Te | CH | Se | 0 | 0 | 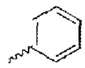 | 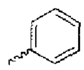 | - | - |
| 329 | Te | CH | Se | 0 | 0 | 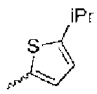 | 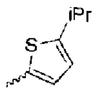 | - | - |
| 330 | Te | CH | Se | 1 | 1 | $CH_3$ | $CH_3$ | $CH_3$ | $CH_3$ |

| Specific Example | X | Y | Z | m | n | $R^1$ | $R^2$ | $R^3$ | $R^4$ |
|---|---|---|---|---|---|---|---|---|---|
| 331 | Te | $C(n-C_8H_{17})$ | Se | 0 | 0 | H | H | - | - |
| 332 | Te | $C(n-C_6H_{13})$ | Se | 0 | 0 | $CH_3$ | $CH_3$ | - | - |
| 333 | Te | CH | Se | 1 | 1 | 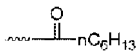 | $-N(C_4H_9)_2$ | $OCH_3$ | $OCH_3$ |
| 334 | Te | CH | Se | 0 | 0 | 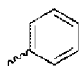 | $n-C_5H_{11}$ | - | - |
| 335 | Te | CH | Se | 2 | 2 | $CH_3$ | $CH_3$ | $CH_3$ | $CH_3$ |
| 336 | Te | O | N | 0 | 0 | $CH_3$ | $CH_3$ | - | - |
| 337 | Te | O | N | 0 | 0 | $n-C_{14}H_{29}$ | $n-C_{14}H_{29}$ | - | - |
| 338 | Te | O | N | 0 | 0 | 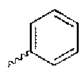 | 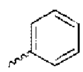 | - | - |
| 339 | Te | O | N | 0 | 0 | 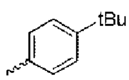 | 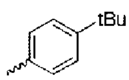 | - | - |
| 340 | Te | O | N | 1 | 1 | $CH_3$ | $CH_3$ | $CH_3$ | $CH_3$ |

| Specific Example | X | Y | Z | m | n | $R^1$ | $R^2$ | $R^3$ | $R^4$ |
|---|---|---|---|---|---|---|---|---|---|
| 341 | Te | O | N | 0 | 0 | 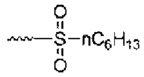 | 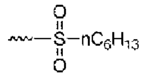 | - | - |
| 342 | Te | O | N | 0 | 0 | $CH_3$ | $CH_3$ | - | - |
| 343 | Te | O | N | 1 | 1 | 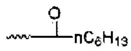 | 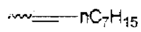 | $SCH_3$ | $SCH_3$ |
| 344 | Te | O | N | 0 | 0 | 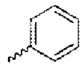 | $n-C_5H_{11}$ | - | - |
| 345 | Te | O | N | 2 | 2 | $CH_3$ | $CH_3$ | $CH_3$ | $CH_3$ |
| 346 | Te | S | N | 0 | 0 | $CH_3$ | $CH_3$ | - | - |
| 347 | Te | S | N | 0 | 0 | $n-C_9H_{19}$ | $n-C_9H_{19}$ | - | - |
| 348 | Te | S | N | 0 | 0 | 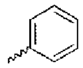 | 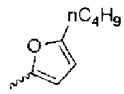 | - | - |
| 349 | Te | S | N | 0 | 0 | 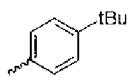 | 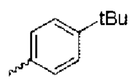 | - | - |
| 350 | Te | S | N | 1 | 1 | $CH_3$ | $CH_3$ | $CH_3$ | $CH_3$ |

| Specific Example | X | Y | Z | m | n | $R^1$ | $R^2$ | $R^3$ | $R^4$ |
|---|---|---|---|---|---|---|---|---|---|
| 351 | Te | S | N | 0 | 0 | $-S-nC_6H_{13}$ | $-S-nC_6H_{13}$ | - | - |
| 352 | Te | S | N | 0 | 0 | $CH_3$ | $CH_3$ | - | - |
| 353 | Te | S | N | 1 | 1 | 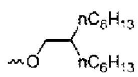 | 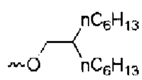 | $SCH_3$ | $SCH_3$ |
| 354 | Te | S | N | 0 | 0 | 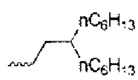 | $n-C_5H_{11}$ | - | - |
| 355 | Te | S | N | 2 | 2 | $CH_3$ | $CH_3$ | $CH_3$ | $CH_3$ |
| 356 | Te | Se | CH | 0 | 0 | $CH_3$ | $CH_3$ | - | - |
| 357 | Te | Se | CH | 0 | 0 | $n-C_{14}H_{29}$ | $n-C_{14}H_{29}$ | - | - |
| 358 | Te | Se | CH | 0 | 0 | 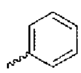 | 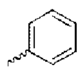 | - | - |
| 359 | Te | Se | CH | 0 | 0 | 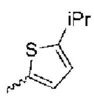 | 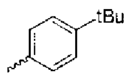 | - | - |

(continued)

| Specific Example | X | Y | Z | m | n | $R^1$ | $R^2$ | $R^3$ | $R^4$ |
|---|---|---|---|---|---|---|---|---|---|
| 360 | Te | Se | CH | 1 | 1 | H | H | n-$C_{14}H_{29}$ | n-$C_{14}H_{29}$ |

| Specific Example | X | Y | Z | m | n | $R^1$ | $R^2$ | $R^3$ | $R^4$ |
|---|---|---|---|---|---|---|---|---|---|
| 361 | Te | Se | C(n-$C_9H_{19}$) | 0 | 0 | 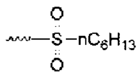 | 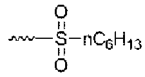 | - | - |
| 362 | Te | Se | C(n-$C_6H_{13}$) | 0 | 0 | $CH_3$ | $CH_3$ | - | - |
| 363 | Te | Se | CH | 1 | 1 | 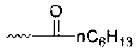 | $N(C_4H_9)_2$ | $OCH_3$ | $OCH_3$ |
| 364 | Te | Se | CH | 0 | 0 | 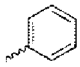 | n-$C_5H_{11}$ | - | - |
| 365 | Te | Se | CH | 2 | 2 | $CH_3$ | $CH_3$ | $CH_3$ | $CH_3$ |
| 366 | Te | Se | N | 0 | 0 | $CH_3$ | $CH_3$ | - | - |
| 367 | Te | Se | N | 0 | 0 | n-$C_{14}H_{29}$ | n-$C_{14}H_{29}$ | - | - |
| 368 | Te | Se | N | 0 | 0 | 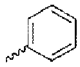 | 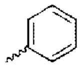 | - | - |
| 369 | Te | Se | N | 0 | 0 | 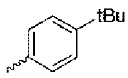 | 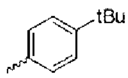 | - | - |
| 370 | Te | Se | N | 1 | 1 | $CH_3$ | $CH_3$ | $CH_3$ | $CH_3$ |

| Specific Example | X | Y | Z | m | n | $R^1$ | $R^2$ | $R^3$ | $R^4$ |
|---|---|---|---|---|---|---|---|---|---|
| 371 | Te | Se | N | 0 | 0 | 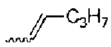 | 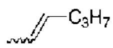 | - | - |
| 372 | Te | Se | N | 0 | 0 | $CH_3$ | $CH_3$ | - | - |
| 373 | Te | Se | N | 1 | 1 | 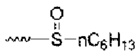 | 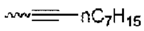 | $SCH_3$ | $SCH_3$ |
| 374 | Te | Se | N | 0 | 0 | 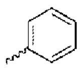 | n-$C_5H_{11}$ |  | - |
| 375 | Te | Se | N | 2 | 2 | $CH_3$ | $CH_3$ | $CH_3$ | $CH_3$ |
| 376 | Te | CH | O | 0 | 0 | $CH_3$ | $CH_3$ | - | - |
| 377 | Te | CH | O | 0 | 0 | n-$C_{14}H_{29}$ | n-$C_{14}H_{29}$ | - | - |
| 378 | Te | CH | O | 0 | 0 | 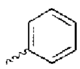 | 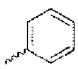 | - | - |

(continued)

| Specific Example | X | Y | Z | m | n | $R^1$ | $R^2$ | $R^3$ | $R^4$ |
|---|---|---|---|---|---|---|---|---|---|
| 379 | Te | CH | O | 0 | 0 | 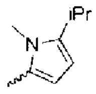 | 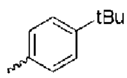 | - | - |
| 380 | Te | CH | S | 1 | 1 | H | H | $n\text{-}C_{14}H_{29}$ | $n\text{-}C_{14}H_{29}$ |

| Specific Example | X | Y | Z | m | n | $R^1$ | $R^2$ | $R^3$ | $R^4$ |
|---|---|---|---|---|---|---|---|---|---|
| 381 | Te | $C(n\text{-}C_9H_{19})$ | S | 0 | 0 | 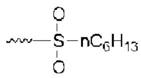 | 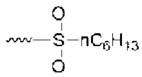 | - | - |
| 382 | Te | $C(n\text{-}C_6H_{13})$ | S | 0 | 0 | $CH_3$ | $CH_3$ | - | - |
| 383 | Te | CH | S | 1 | 1 | 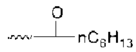 | $N(C_4H_9)_2$ | $OCH_3$ | $OCH_3$ |
| 384 | Te | CH | $N(CH_3)$ | 0 | 0 | 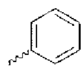 | $n\text{-}C_5H_{11}$ | - | - |
| 385 | Te | CH | $N(n\text{-}C_3H_7)$ | 2 | 2 | $CH_3$ | $CH_3$ | $CH_3$ | $CH_3$ |
| 386 | Te | O | CH | 0 | 0 | $CH_3$ | $CH_3$ | - | - |
| 387 | Te | O | CH | 0 | 0 | $n\text{-}C_{14}H_{29}$ | $n\text{-}C_{14}H_{29}$ | - | - |
| 388 | Te | O | $C(n\text{-}C_9H_{19})$ | 0 | 0 | 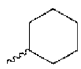 | 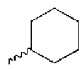 | - | - |
| 389 | Te | O | $C(n\text{-}C_6H_{13})$ | 0 | 0 | 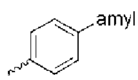 | 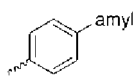 | - | - |
| 390 | Te | O | CH | 1 | 1 | $CH_3$ | $CH_3$ | $CH_3$ | $CH_3$ |

| Specific Example | X | Y | Z | m | n | $R^1$ | $R^2$ | $R^3$ | $R^4$ |
|---|---|---|---|---|---|---|---|---|---|
| 391 | Te | O | CH | 0 | 0 | 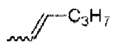 | 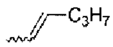 | - | - |
| 392 | Te | S | $C(n\text{-}C_9H_{19})$ | 0 | 0 | $CH_3$ | $CH_3$ | - | - |
| 393 | Te | S | $C(n\text{-}C_6H_{13})$ | 1 | 1 | 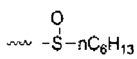 | 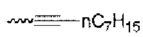 | $SCH_3$ | $SCH_3$ |
| 394 | Te | S | CH | 0 | 0 | 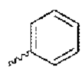 | $n\text{-}C_5H_{11}$ | - | - |
| 395 | Te | S | CH | 2 | 2 | $CH_3$ | $CH_3$ | $CH_3$ | $CH_3$ |
| 396 | Te | N | O | 0 | 0 | $CH_3$ | $CH_3$ | - | - |
| 397 | Te | N | O | 0 | 0 | $n\text{-}C_{14}H_{29}$ | $n\text{-}C_{14}H_{29}$ | - | - |
| 398 | Te | N | O | 0 | 0 | 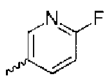 | 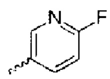 | - | - |

(continued)

| Specific Example | X | Y | Z | m | n | $R^1$ | $R^2$ | $R^3$ | $R^4$ |
|---|---|---|---|---|---|---|---|---|---|
| 399 | Te | N | O | 0 | 0 | 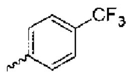 | 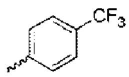 | - | - |
| 400 | Te | N | O | 1 | 1 | $CH_3$ | $CH_3$ | $CH_3$ | $CH_3$ |

| Specific Example | X | Y | Z | m | n | $R^1$ | $R^2$ | $R^3$ | $R^4$ |
|---|---|---|---|---|---|---|---|---|---|
| 401 | Te | N | O | 0 | 0 | 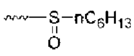 | 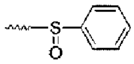 | - | - |
| 402 | Te | N | O | 0 | 0 | $CH_3$ | $CH_3$ | - | - |
| 403 | Te | N | O | 1 | 1 | 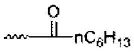 | 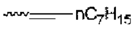 | $SCH_3$ | $SCH_3$ |
| 404 | Te | N | O | 0 | 0 | 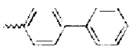 | $n\text{-}C_5H_{11}$ | - | - |
| 405 | Te | N | O | 2 | 2 | $CH_3$ | $CH_3$ | $CH_3$ | $CH_3$ |
| 406 | Te | N | S | 0 | 0 | $CH_3$ | $CH_3$ | - | - |
| 407 | Te | N | S | 0 | 0 | $n\text{-}C_9H_{19}$ | $n\text{-}C_9H_{19}$ | - | - |
| 408 | Te | N | S | 0 | 0 | 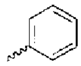 | 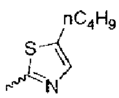 | - | - |
| 409 | Te | N | S | 0 | 0 | 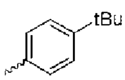 | 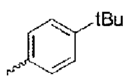 | - | - |
| 410 | Te | N | S | 1 | 1 | $CH_3$ | $CH_3$ | $CH_3$ | $CH_3$ |

| Specific Example | X | Y | Z | m | n | $R^1$ | $R^2$ | $R^3$ | $R^4$ |
|---|---|---|---|---|---|---|---|---|---|
| 411 | Te | N | S | 0 | 0 | $S-nC_6H_{13}$ | $S-nC_6H_{13}$ | - | - |
| 412 | Te | N | S | 0 | 0 | $CH_3$ | $CH_3$ | - | - |
| 413 | Te | N | S | 1 | 2 | 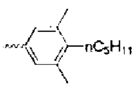 | 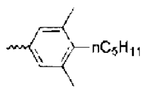 | $SCH_3$ | $SCH_3$ |
| 414 | Te | N | S | 0 | 0 | 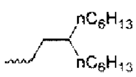 | $n\text{-}C_5H_{11}$ | - | - |
| 415 | Te | N | Se | 0 | 0 | $CH_3$ | $CH_3$ | - | - |
| 416 | Te | N | Se | 0 | 0 | $n\text{-}C_{14}H_{29}$ | $n\text{-}C_{14}H_{29}$ | - | - |

(continued)

| Specific Example | X | Y | Z | m | n | $R^1$ | $R^2$ | $R^3$ | $R^4$ |
|---|---|---|---|---|---|---|---|---|---|
| 417 | Te | N | Se | 0 | 0 | 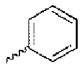 | 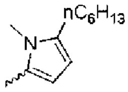 | - | - |
| 418 | Te | N | Se | 0 | 0 | 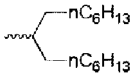 | 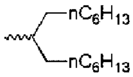 | - | - |
| 419 | Te | N | Se | 1 | 1 | H | H | $n\text{-}C_{14}H_{29}$ | $n\text{-}C_{14}H_{29}$ |
| 420 | Te | N | Se | 0 | 0 | 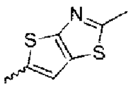 | 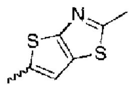 | - | - |

| Specific Example | X | Y | Z | m | n | $R^1$ | $R^2$ | $R^3$ | $R^4$ |
|---|---|---|---|---|---|---|---|---|---|
| 421 | Te | N | Se | 0 | 0 | $CH_3$ | $CH_3$ | - | - |
| 422 | Te | N | Se | 1 | 1 | 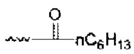 | $N(C_4H_9)_2$ | $OCH_3$ | $OCH_3$ |
| 423 | Te | N | Se | 0 | 0 | 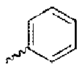 | $n\text{-}C_5H_{11}$ | - | - |
| 424 | Te | N | $N(CH_3)$ | 2 | 2 | $CH_3$ | $CH_3$ | $CH_3$ | $CH_3$ |
| 425 | Te | N | NH | 0 | 0 | $C_2H_5$ | $C_2H_5$ | - | - |
| 426 | Te | N | $N(CH_3)$ | 0 | 0 | $n\text{-}C_{14}H_{29}$ | $n\text{-}C_{14}H_{29}$ | - | - |
| 427 | Te | N | $N(CH_3)$ | 0 | 0 | 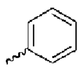 | 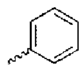 | - | - |
| 428 | Te | N | $N(CH_3)$ | 0 | 0 | 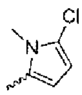 | 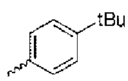 | - | - |
| 429 | Te | N | $N(CH_3)$ | 1 | 1 | H | H | $n\text{-}C_{14}H_{29}$ | $n\text{-}C_{14}H_{29}$ |
| 430 | Te | N | $N(n\text{-}C_9H_{19})$ | 0 | 0 | 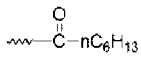 | 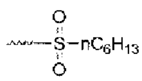 | - | - |

| Specific Example | X | Y | Z | m | n | $R^1$ | $R^2$ | $R^3$ | $R^4$ |
|---|---|---|---|---|---|---|---|---|---|
| 431 | Te | N | $N(CH_3)$ | 0 | 0 | $CH_3$ | $CH_3$ | - | - |
| 432 | Te | N | N(i-Pr) | 1 | 1 | 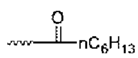 | $N(C_4H_9)_2$ | $OCH_3$ | $OCH_3$ |
| 433 | Te | N | $N(CH_3)$ | 0 | 0 | 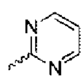 | $n\text{-}C_5H_{11}$ | - | - |
| 434 | Te | N | $N(CH_3)$ | 2 | 2 | $CH_3$ | $CH_3$ | $CH_3$ | $CH_3$ |

(continued)

| Specific Example | X | Y | Z | m | n | $R^1$ | $R^2$ | $R^3$ | $R^4$ |
|---|---|---|---|---|---|---|---|---|---|
| 435 | Te | $N(CH_3)$ | N | 0 | 0 | $CH_3$ | $CH_3$ | - | - |
| 436 | Te | NH | N | 0 | 0 | $n-C_{14}H_{29}$ | $n-C_{14}H_{29}$ | - | - |
| 437 | Te | $N(CH_3)$ | N | 0 | 0 | 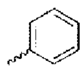 | 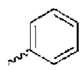 | - | - |
| 438 | Te | $N(CH_3)$ | N | 0 | 0 | 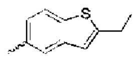 | 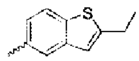 | - | - |
| 439 | Te | $N(CH_3)$ | N | 1 | 1 | $CH_3$ | $CH_3$ | $CH_3$ | $CH_3$ |
| 440 | Te | $N(CH_3)$ | N | 0 | 0 | 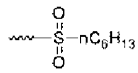 | 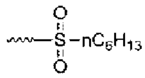 | - | - |

| Specific Example | X | Y | Z | m | n | $R^1$ | $R^2$ | $R^3$ | $R^4$ |
|---|---|---|---|---|---|---|---|---|---|
| 441 | Te | $N(n-C_9H_{19})$ | N | 0 | 0 | $CH_3$ | $CH_3$ | - | - |
| 442 | Te | $N(CH_3)$ | N | 1 | 1 | 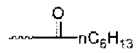 | 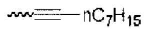 | 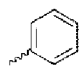 | 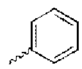 |
| 443 | Te | N(i-Pr) | N | 0 | 0 | 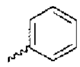 | $n-C_5H_{11}$ | - | - |
| 444 | Te | $N(CH_3)$ | N | 2 | 2 | $CH_3$ | $CH_3$ | $CH_3$ | $CH_3$ |
| 445 | Te | $N(CH_3)$ | CH | 0 | 0 | $CH_3$ | $CH_3$ | - | - |
| 446 | Te | NH | CH | 0 | 0 | $n-C_{14}H_{29}$ | $n-C_{14}H_{29}$ | - | - |
| 447 | Te | $N(CH_3)$ | CH | 0 | 0 | 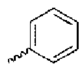 | 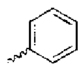 | - | - |
| 448 | Te | $N(CH_3)$ | CH | 0 | 0 | 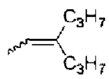 | 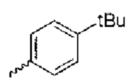 | - | - |
| 449 | Te | $N(CH_3)$ | CH | 1 | 1 | H | H | $n-C_{14}H_{29}$ | $n-C_{14}H_{29}$ |
| 450 | Te | $N(CH_3)$ | $C(n-C_9H_{19})$ | 0 | 0 | $-O-nC_6H_{13}$ | $-O-nC_6H_{13}$ | - | - |

| Specific Example | X | Y | Z | m | n | $R^1$ | $R^2$ | $R^3$ | $R^4$ |
|---|---|---|---|---|---|---|---|---|---|
| 451 | Te | $N(n-C_9H_{19})$ | $C(n-C_6H_{13})$ | 0 | 0 | $CH_3$ | $CH_3$ | - | - |
| 452 | Te | $N(CH_3)$ | CH | 1 | 1 | 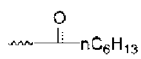 | $-N(C_4H_9)_2$ | $OCH_3$ | $OCH_3$ |
| 453 | Te | N(i-Pr) | CH | 0 | 0 | 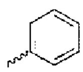 | $n-C_5H_{11}$ | - | - |
| 454 | Te | $N(CH_3)$ | CH | 2 | 2 | $CH_3$ | $CH_3$ | $CH_3$ | $CH_3$ |

(continued)

| Specific Example | X | Y | Z | m | n | $R^1$ | $R^2$ | $R^3$ | $R^4$ |
|---|---|---|---|---|---|---|---|---|---|
| 455 | $N(CH_3)$ | CH | Se | 0 | 0 | $CH_3$ | $CH_3$ | - | - |
| 456 | NH | CH | Se | 0 | 0 | $n\text{-}C_{14}H_{29}$ | $n\text{-}C_{14}H_{29}$ | - | - |
| 457 | $N(CH_3)$ | CH | Se | 0 | 0 | 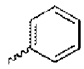 | 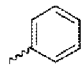 | - | - |
| 458 | $N(CH_3)$ | CH | Se | 0 | 0 | 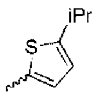 | 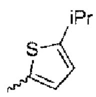 | - | - |
| 459 | $N(CH_3)$ | CH | Se | 1 | 1 | $CH_3$ | $CH_3$ | $CH_3$ | $CH_3$ |
| 460 | $N(CH_3)$ | $C(n\text{-}C_8H_{17})$ | Se | 0 | 0 | H | H | - | - |

| Specific Example | X | Y | Z | m | n | $R^1$ | $R^2$ | $R^3$ | $R^4$ |
|---|---|---|---|---|---|---|---|---|---|
| 461 | $N(n\text{-}C_9H_{19})$ | $C(n\text{-}C_6H_{13})$ | Se | 0 | 0 | $CH_3$ | $CH_3$ | - | - |
| 462 | $N(CH_3)$ | CH | Se | 1 | 1 | 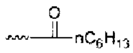 | $N(C_4H_9)_2$ | $OCH_3$ | $OCH_3$ |
| 463 | N(i-Pr) | CH | Se | 0 | 0 | 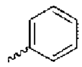 | $n\text{-}C_5H_{11}$ | - | - |
| 464 | $N(CH_3)$ | CH | Se | 2 | 2 | $CH_3$ | $CH_3$ | $CH_3$ | $CH_3$ |
| 465 | $N(CH_3)$ | O | N | 0 | 0 | $CH_3$ | $CH_3$ | - | - |
| 466 | NH | O | N | 0 | 0 | $n\text{-}C_{14}H_{29}$ | $n\text{-}C_{14}H_{29}$ | - | - |
| 467 | $N(CH_3)$ | O | N | 0 | 0 | 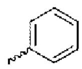 | 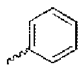 | - | - |
| 468 | $N(CH_3)$ | O | N | 0 | 0 | 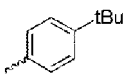 | 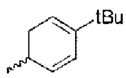 | - | - |
| 469 | $N(CH_3)$ | O | N | 1 | 1 | $CH_3$ | $CH_3$ | $CH_3$ | $CH_3$ |
| 470 | $N(CH_3)$ | O | N | 0 | 0 | 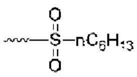 | 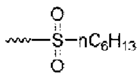 | - | - |

| Specific Example | X | Y | Z | m | n | $R^1$ | $R^2$ | $R^3$ | $R^4$ |
|---|---|---|---|---|---|---|---|---|---|
| 471 | $N(n\text{-}C_9H_{19})$ | O | N | 0 | 0 | $CH_3$ | 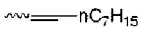 | - | - |
| 472 | $N(CH_3)$ | O | N | 1 | 1 | 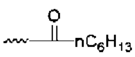 | 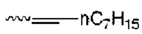 | $SCH_3$ | $SCH_3$ |

(continued)

| Specific Example | X | Y | Z | m | n | $R^1$ | $R^2$ | $R^3$ | $R^4$ |
|---|---|---|---|---|---|---|---|---|---|
| 473 | N(i-Pr) | O | N | 0 | 0 | 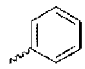 | $n\text{-}C_5H_{11}$ | - | - |
| 474 | $N(CH_3)$ | O | N | 2 | 2 | $CH_3$ | $CH_3$ | $CH_3$ | $CH_3$ |
| 475 | $N(CH_3)$ | S | N | 0 | 0 | $CH_3$ | $CH_3$ | - | - |
| 476 | NH | S | N | 0 | 0 | $n\text{-}C_9H_{19}$ | $n\text{-}C_9H_{19}$ | - | - |
| 477 | $N(CH_3)$ | S | N | 0 | 0 | 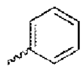 | 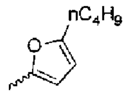 | - | - |
| 478 | $N(CH_3)$ | S | N | 0 | 0 | 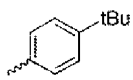 | 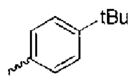 | - | - |
| 479 | $N(CH_3)$ | S | N | 1 | 1 | $CH_3$ | $CH_3$ | $CH_3$ | $CH_3$ |
| 480 | $N(CH_3)$ | S | N | 0 | 0 | $S-nC_6H_{13}$ | $S-nC_6H_{13}$ | - | - |

| Specific Example | X | Y | Z | m | n | $R^1$ | $R^2$ | $R^3$ | $R^4$ |
|---|---|---|---|---|---|---|---|---|---|
| 481 | $N(n\text{-}C_9H_{19})$ | S | N | 0 | 0 | $CH_3$ | $CH_3$ | - | - |
| 482 | $N(CH_3)$ | S | N | 1 | 1 | 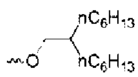 | 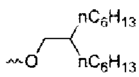 | $SCH_3$ | $SCH_3$ |
| 483 | N(i-Pr) | S | N | 0 | 0 | 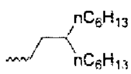 | $n\text{-}C_5H_{11}$ | - | - |
| 484 | $N(CH_3)$ | S | N | 2 | 2 | $CH_3$ | $CH_3$ | $CH_3$ | $CH_3$ |
| 485 | $N(CH_3)$ | Se | CH | 0 | 0 | $CH_3$ | $CH_3$ | - | - |
| 486 | NH | Se | CH | 0 | 0 | $n\text{-}C_{14}H_{29}$ | $n\text{-}C_{14}H_{29}$ | - | - |
| 487 | $N(CH_3)$ | Se | CH | 0 | 0 | 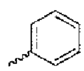 | 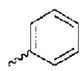 | - | - |
| 488 | $N(CH_3)$ | Se | CH | 0 | 0 | 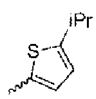 | 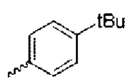 | - | - |
| 489 | $N(CH_3)$ | Se | CH | 1 | 1 | H | H | $n\text{-}C_{14}H_{29}$ | $n\text{-}C_{14}H_{29}$ |

(continued)

| Specific Example | X | Y | Z | m | n | $R^1$ | $R^2$ | $R^3$ | $R^4$ |
|---|---|---|---|---|---|---|---|---|---|
| 490 | $N(CH_3)$ | Se | C(n-$C_9H_{19}$) | 0 | 0 | 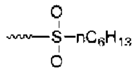 | 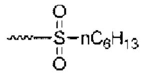 | - | - |

| Specific Example | X | Y | Z | m | n | $R^1$ | $R^2$ | $R^3$ | $R^4$ |
|---|---|---|---|---|---|---|---|---|---|
| 491 | N(n-$C_9H_{19}$) | Se | C(n-$C_6H_{13}$) | 0 | 0 | $CH_3$ | $CH_3$ | - | - |
| 492 | $N(CH_3)$ | Se | CH | 1 | 1 | 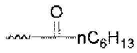 | $N(C_4H_9)_2$ | $OCH_3$ | $OCH_3$ |
| 493 | NH | Se | CH | 0 | 0 | 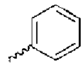 | n-$C_5H_{11}$ | - | - |
| 494 | $N(CH_3)$ | Se | CH | 2 | 2 | $CH_3$ | $CH_3$ | $CH_3$ | $CH_3$ |
| 495 | $N(CH_3)$ | Se | N | 0 | 0 | $CH_3$ | $CH_3$ | - | - |
| 496 | $N(CH_3)$ | Se | N | 0 | 0 | n-$C_{14}H_{29}$ | n-$C_{14}H_{29}$ | - | - |
| 497 | $N(CH_3)$ | Se | N | 0 | 0 | 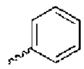 | 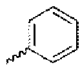 | - | - |
| 498 | N(n-$C_9H_{19}$) | Se | N | 0 | 0 | 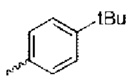 | 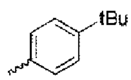 | - | - |
| 499 | $N(CH_3)$ | Se | N | 1 | 1 | $CH_3$ | $CH_3$ | $CH_3$ | $CH_3$ |
| 500 | N(i-Pr) | Se | N | 0 | 0 | 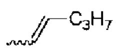 | 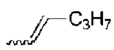 | - | - |

| Specific Example | X | Y | Z | m | n | $R^1$ | $R^2$ | $R^3$ | $R^4$ |
|---|---|---|---|---|---|---|---|---|---|
| 501 | $N(CH_3)$ | Se | N | 0 | 0 | $CH_3$ | $CH_3$ | - | - |
| 502 | $N(CH_3)$ | Se | N | 1 | 1 | 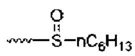 | 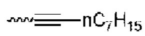 | $SCH_3$ | $SCH_3$ |
| 503 | NH | Se | N | 0 | 0 | 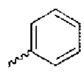 | n-$C_5H_{11}$ | - | - |
| 504 | $N(CH_3)$ | Se | N | 2 | 2 | $CH_3$ | $CH_3$ | $CH_3$ | $CH_3$ |
| 505 | $N(CH_3)$ | CH | O | 0 | 0 | $CH_3$ | $CH_3$ | - | - |
| 506 | $N(CH_3)$ | CH | O | 0 | 0 | n-$C_{14}H_{29}$ | n-$C_{14}H_{29}$ | - | - |
| 507 | $N(CH_3)$ | CH | O | 0 | 0 | 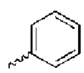 | 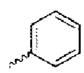 | - | - |
| 508 | NH | CH | O | 0 | 0 | 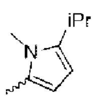 | 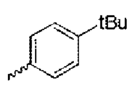 | - | - |

(continued)

| Specific Example | X | Y | Z | m | n | $R^1$ | $R^2$ | $R^3$ | $R^4$ |
|---|---|---|---|---|---|---|---|---|---|
| 509 | $N(CH_3)$ | CH | S | 1 | 1 | $CH_3$ | $CH_3$ | $n\text{-}C_{14}H_{29}$ | $n\text{-}C_{14}H_{29}$ |
| 510 | $N(CH_3)$ | $C(n\text{-}C_9H_{19})$ | S | 0 | 0 | 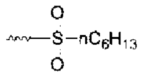 | 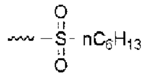 | - | - |

| Specific Example | X | Y | Z | m | n | $R^1$ | $R^2$ | $R^3$ | $R^4$ |
|---|---|---|---|---|---|---|---|---|---|
| 511 | $N(CH_3)$ | $C(n\text{-}C_6H_{13})$ | S | 0 | 0 | $CH_3$ | $CH_3$ | - | - |
| 512 | $N(CH_3)$ | CH | S | 1 | 1 | 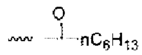 | $N(C_4H_9)_2$ | $OCH_3$ | $OCH_3$ |
| 513 | $N(n\text{-}C_9H_{19})$ | CH | $N(CH_3)$ | 0 | 0 | 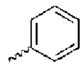 | $n\text{-}C_5H_{11}$ | - | - |
| 514 | $N(CH_3)$ | CH | $N(n\text{-}C_3H_7)$ | 2 | 2 | $CH_3$ | $CH_3$ | $CH_3$ | $CH_3$ |
| 515 | N(i-Pr) | O | CH | 0 | 0 | $CH_3$ | $CH_3$ | - | - |
| 516 | $N(CH_3)$ | O | CH | 0 | 0 | $n\text{-}C_{14}H_{29}$ | $n\text{-}C_{14}H_{29}$ | - | - |
| 517 | $N(CH_3)$ | O | $C(n\text{-}C_9H_{19})$ | 0 | 0 | 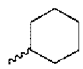 | 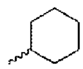 | - | - |
| 518 | NH | O | $C(n\text{-}C_6H_{13})$ | 0 | 0 | 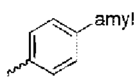 | 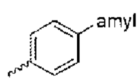 | - | - |
| 519 | $N(CH_3)$ | O | CH | 1 | 1 | $CH_3$ | $CH_3$ | $CH_3$ | $CH_3$ |
| 520 | $N(CH_3)$ | O | CH | 0 | 0 | 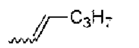 | 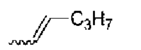 | - | - |

| Specific Example | X | Y | Z | m | n | $R^1$ | $R^2$ | $R^3$ | $R^4$ |
|---|---|---|---|---|---|---|---|---|---|
| 521 | $N(CH_3)$ | S | $C(n\text{-}C_9H_{19})$ | 0 | 0 | $CH_3$ | $CH_3$ | - | - |
| 522 | $N(CH_3)$ | S | $C(n\text{-}C_6H_{13})$ | 1 | 1 | 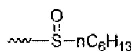 | 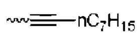 | $SCH_3$ | $SCH_3$ |
| 523 | $N(n\text{-}C_9H_{19})$ | S | CH | 0 | 0 | 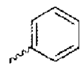 | $n\text{-}C_5H_{11}$ | - | - |
| 524 | $N(CH_3)$ | S | CH | 2 | 2 | $CH_3$ | $CH_3$ | $CH_3$ | $CH_3$ |
| 525 | N(i-Pr) | N | S | 0 | 0 | $CH_3$ | $CH_3$ | - | - |
| 526 | $N(CH_3)$ | N | S | 0 | 0 | $n\text{-}C_{14}H_{29}$ | $n\text{-}C_{14}H_{29}$ | - | - |
| 527 | $N(CH_3)$ | N | S | 0 | 0 | 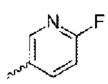 | 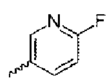 | - | - |

(continued)

| Specific Example | X | Y | Z | m | n | $R^1$ | $R^2$ | $R^3$ | $R^4$ |
|---|---|---|---|---|---|---|---|---|---|
| 528 | NH | N | S | 0 | 0 | 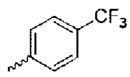 | 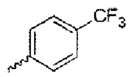 | - | - |
| 529 | $N(CH_3)$ | N | S | 1 | 1 | $CH_3$ | $CH_3$ | $CH_3$ | $CH_3$ |
| 530 | $N(CH_3)$ | N | S | 0 | 0 | 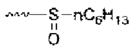 | 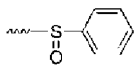 | - | - |

| Specific Example | X | Y | Z | m | n | $R^1$ | $R^2$ | $R^3$ | $R^4$ |
|---|---|---|---|---|---|---|---|---|---|
| 531 | $N(CH_3)$ | N | S | 0 | 0 | $CH_3$ | $CH_3$ | - | - |
| 532 | $N(CH_3)$ | N | S | 1 | 1 | 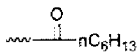 | ≡ $nC_7H_{15}$ | $SCH_3$ | $SCH_3$ |
| 533 | $N(n-C_9H_{19})$ | N | S | 0 | 0 | 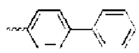 | $n-C_5H_{11}$ | - | - |
| 534 | $N(CH_3)$ | N | S | 2 | 2 | $CH_3$ | $CH_3$ | $CH_3$ | $CH_3$ |
| 535 | N(i-Pr) | N | O | 0 | 0 | $CH_3$ | $CH_3$ | - | - |
| 536 | $N(CH_3)$ | N | O | 0 | 0 | $n-C_9H_{19}$ | $n-C_9H_{19}$ | - | - |
| 537 | $N(CH_3)$ | N | O | 0 | 0 | 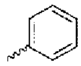 | 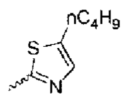 | - | - |
| 538 | NH | N | O | 0 | 0 | 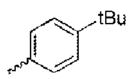 | 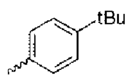 | - | - |
| 539 | $N(CH_3)$ | N | O | 1 | 1 | $CH_3$ | $CH_3$ | $CH_3$ | $CH_3$ |
| 540 | $N(CH_3)$ | N | O | 0 | 0 | $S-nC_6H_{13}$ | $S-nC_6H_{13}$ | - | - |

| Specific Example | X | Y | Z | m | n | $R^1$ | $R^2$ | $R^3$ | $R^4$ |
|---|---|---|---|---|---|---|---|---|---|
| 541 | $N(CH_3)$ | N | O | 0 | 0 | $CH_3$ | $CH_3$ | - | - |
| 542 | $N(CH_3)$ | N | O | 1 | 2 | 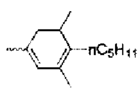 | 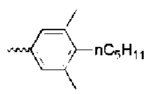 | $SCH_3$ | $SCH_3$ |
| 543 | $N(n-C_9H_{19})$ | N | O | 0 | 0 | 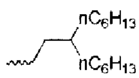 | $n-C_5H_{11}$ | - | - |
| 544 | $N(CH_3)$ | N | Se | 0 | 0 | $CH_3$ | $CH_3$ | - | - |
| 545 | N(i-Pr) | N | Se | 0 | 0 | $n-C_{14}H_{29}$ | $n-C_{14}H_{29}$ | - | - |
| 546 | $N(CH_3)$ | N | Se | 0 | 0 | 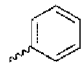 | 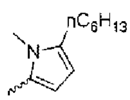 | - | - |

(continued)

| Specific Example | X | Y | Z | m | n | $R^1$ | $R^2$ | $R^3$ | $R^4$ |
|---|---|---|---|---|---|---|---|---|---|
| 547 | $N(CH_3)$ | N | Se | 0 | 0 | 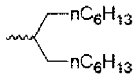 | 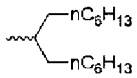 | - | - |
| 548 | NH | N | Se | 1 | 1 | H | H | $n\text{-}C_{14}H_{29}$ | $n\text{-}C_{14}H_{29}$ |
| 549 | $N(CH_3)$ | N | Se | 0 | 0 | 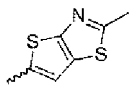 | 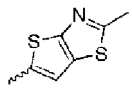 | - | - |
| 550 | $N(CH_3)$ | N | Se | 0 | 0 | $CH_3$ | $CH_3$ | - | - |

| Specific Example | X | Y | Z | m | n | $R^1$ | $R^2$ | $R^3$ | $R^4$ |
|---|---|---|---|---|---|---|---|---|---|
| 551 | $N(CH_3)$ | N | Se | 1 | 1 | 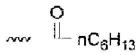 | $N(C_4H_9)_2$ | $OCH_3$ | $OCH_3$ |
| 552 | $N(CH_3)$ | N | Se | 0 | 0 | 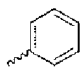 | $n\text{-}C_5H_{11}$ | - | - |
| 553 | $N(n\text{-}C_9H_{19})$ | N | $N(CH_3)$ | 2 | 2 | $CH_3$ | $CH_3$ | $CH_3$ | $CH_3$ |
| 554 | $N(CH_3)$ | N | NH | 0 | 0 | $C_2H_5$ | $C_2H_5$ | - | - |
| 555 | N(i-Pr) | N | $N(CH_3)$ | 0 | 0 | $n\text{-}C_{14}H_{29}$ | $n\text{-}C_{14}H_{29}$ | - | - |
| 556 | $N(CH_3)$ | N | $N(CH_3)$ | 0 | 0 | 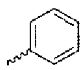 | 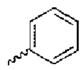 | - | - |
| 557 | $N(CH_3)$ | N | $N(CH_3)$ | 0 | 0 | 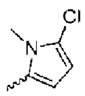 | 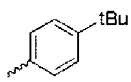 | - | - |
| 558 | NH | N | $N(CH_3)$ | 1 | 1 | H | H | $n\text{-}C_{14}H_{29}$ | $n\text{-}C_{14}H_{29}$ |
| 559 | $N(CH_3)$ | N | $N(n\text{-}C_9H_{19})$ | 0 | 0 | 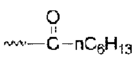 | 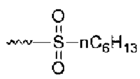 | - | - |
| 560 | $N(CH_3)$ | N | $N(CH_3)$ | 0 | 0 | $CH_3$ | $CH_3$ | - | - |

| Specific Example | X | Y | Z | m | n | $R^1$ | $R^2$ | $R^3$ | $R^4$ |
|---|---|---|---|---|---|---|---|---|---|
| 561 | $N(CH_3)$ | N | N(i-Pr) | 1 | 1 | 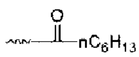 | $N(C_4H_9)_2$ | $OCH_3$ | $OCH_3$ |
| 562 | $N(CH_3)$ | N | $N(CH_3)$ | 0 | 0 | 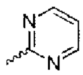 | $n\text{-}C_5H_{11}$ | - | - |
| 563 | $N(n\text{-}C_9H_{19})$ | N | $N(CH_3)$ | 2 | 2 | $CH_3$ | $CH_3$ | $CH_3$ | $CH_3$ |
| 564 | $N(CH_3)$ | $N(CH_3)$ | N | 0 | 0 | $CH_3$ | $CH_3$ | - | - |

(continued)

| Specific Example | X | Y | Z | m | n | $R^1$ | $R^2$ | $R^3$ | $R^4$ |
|---|---|---|---|---|---|---|---|---|---|
| 565 | $N(CH_3)$ | NH | N | 0 | 0 | $n\text{-}C_{14}H_{29}$ | $n\text{-}C_{14}H_{29}$ | - | - |
| 566 | NH | $N(CH_3)$ | N | 0 | 0 | 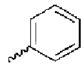 | 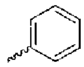 | - | - |
| 567 | $N(CH_3)$ | $N(CH_3)$ | N | 0 | 0 | 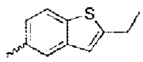 | 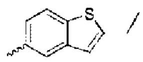 | - | - |
| 568 | $N(CH_3)$ | $N(CH_3)$ | N | 1 | 1 | $CH_3$ | $CH_3$ | $CH_3$ | $CH_3$ |
| 569 | $N(CH_3)$ | $N(CH_3)$ | N | 0 | 0 | 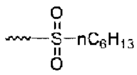 | 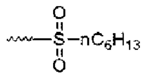 | - | - |
| 570 | $N(CH_3)$ | $N(n\text{-}C_9H_{19})$ | N | 0 | 0 | $CH_3$ | $CH_3$ | - | - |

| Specific Example | X | Y | Z | m | n | $R^1$ | $R^2$ | $R^3$ | $R^4$ |
|---|---|---|---|---|---|---|---|---|---|
| 571 | $N(n-C_9H_{19})$ | $N(CH_3)$ | N | 1 | 1 | 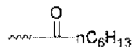 | 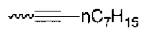 | 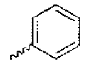 | 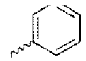 |
| 572 | $N(CH_3)$ | N(i-Pr) | N | 0 | 0 | 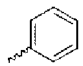 | $n-C_5H_{11}$ | - | - |
| 573 | N(i-Pr) | $N(CH_3)$ | N | 2 | 2 | $CH_3$ | $CH_3$ | $CH_3$ | $CH_3$ |
| 574 | $N(CH_3)$ | $N(CH_3)$ | CH | 0 | 0 | $CH_3$ | $CH_3$ | - | - |
| 575 | $N(CH_3)$ | NH | CH | 0 | 0 | $n-C_{14}H_{29}$ | $n-C_{14}H_{29}$ | - | - |
| 576 | NH | $N(CH_3)$ | CH | 0 | 0 | 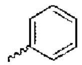 | 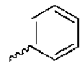 | - | - |
| 577 | $N(CH_3)$ | $N(CH_3)$ | CH | 0 | 0 | 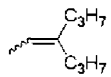 | 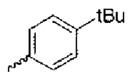 | - | - |
| 578 | $N(CH_3)$ | $N(CH_3)$ | CH | 1 | 1 | H | H | $n-C_{14}H_{29}$ | $n-C_{14}H_{29}$ |
| 579 | $N(CH_3)$ | $N(CH_3)$ | $C(n-C_9H_{19})$ | 0 | 0 | $-O-nC_6H_{13}$ | $-O-nC_6H_{13}$ | - | - |
| 580 | $N(CH_3)$ | $N(n-C_9H_{19})$ | $C(n-O_6H_{13})$ | 0 | 0 | $CH_3$ | $CH_3$ | - | - |

| Specific Example | X | Y | Z | m | n | $R^1$ | $R^2$ | $R^3$ | $R^4$ |
|---|---|---|---|---|---|---|---|---|---|
| 581 | $N(n-C_9H_{19})$ | $N(CH_3)$ | CH | 1 | 1 | 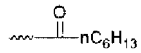 | $N(C_4H_9)_2$ | $OCH_3$ | $OCH_3$ |
| 582 | $N(CH_3)$ | N(i-Pr) | CH | 0 | 0 | 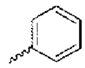 | $n-C_5H_{11}$ | - | - |
| 583 | N(i-Pr) | $N(CH_3)$ | CH | 2 | 2 | $CH_3$ | $CH_3$ | $CH_3$ | $CH_3$ |
| 584 | S | CH | S | 0 | 0 | $CH_3$ | $CH_3$ | - | - |
| 585 | S | CH | S | 0 | 0 | $n-C_9H_{19}$ | $n-C_9H_{19}$ | - | - |
| 586 | S | CH | S | 0 | 0 | $n-C_3H_7$ | $n-C_3H_7$ | - | - |
| 587 | S | CH | S | 0 | 0 | $n-C_4H_9$ | $n-C_4H_9$ | - | - |
| 588 | S | CH | S | 0 | 0 | $n-C_5H_{11}$ | $n-C_5H_{11}$ | - | - |
| 589 | S | CH | S | 0 | 0 | $n-C_8H_{17}$ | $n-C_8H_{17}$ | - | - |
| 590 | S | CH | S | 0 | 0 | $n-C_{10}H_{21}$ | $n-C_{10}H_{21}$ | - | - |

| Specific Example | X | Y | Z | m | n | R1 | R2 | R3 | R4 |
|---|---|---|---|---|---|---|---|---|---|
| 591 | S | CH | S | 0 | 0 | $n-C_{13}H_{27}$ | $n-C_{13}H_{27}$ | - | - |
| 592 | S | CH | S | 0 | 0 | $n-C_4H_9$ | $n-C_5H_{11}$ | - | - |
| 593 | S | CH | S | 0 | 0 | $n-C_4H_9$ | $n-C_8H_{17}$ | - | - |
| 594 | S | CH | S | 0 | 0 | $n-C_4H_9$ | $n-C_{10}H_{21}$ | - | - |
| 595 | S | CH | S | 0 | 0 | $n-C_5H_{11}$ | $n-C_8H_{17}$ | - | - |
| 596 | S | CH | S | 0 | 0 | $n-C_8H_{17}$ | $n-C_{10}H_{21}$ | - | - |
| 597 | S | CH | S | 0 | 0 | $n-C_{10}H_{21}$ | $n-C_3H_7$ | - | - |
| 598 | S | CH | S | 0 | 0 | 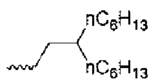 | 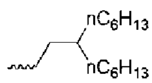 | - | - |
| 599 | S | CH | S | 0 | 0 | i-Pr | i-Pr | - | - |
| 600 | S | CH | S | 0 | 0 | t-Bu | $n-C_3H_7$ | - | - |
| 601 | S | CH | S | 0 | 0 | 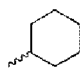 | $n-C_3H_7$ | - | - |
| 602 | O | CH | Se | 1 | 1 | $n-C_{10}H_{21}$ | $n-C_{10}H_{21}$ | F | F |
| 603 | O | CH | Se | 1 | 1 | $n-C_{10}H_{21}$ | $n-C_{10}H_{21}$ | Cl | Cl |

| Specific Example | X | Y | Z | m | n | R1 | R2 | R3 | R4 |
|---|---|---|---|---|---|---|---|---|---|
| 604 | O | N | N($CH_3$) | 2 | 2 | $CH_3$ | $CH_3$ | $CH_3$ | $CH_3$ |
| 605 | O | M | NH | 0 | 0 | $C_2H_5$ | $C_2H_5$ | - | - |
| 606 | O | N | N($CH_3$) | 0 | 0 | n-$C_{14}H_{29}$ | n-$C_{14}H_{29}$ | - | - |
| 607 | O | N | N($CH_3$) | 0 | 0 | 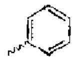 | 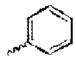 | - | - |
| 608 | O | N | N($CH_3$) | 0 | 0 | 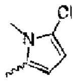 | 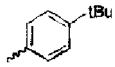 | - | - |
| 609 | O | N | N($CH_3$) | 1 | 1 | H | H | n-$C_{14}H_{29}$ | n-$C_{14}H_{29}$ |
| 610 | O | N | N(n-$C_9H_{19}$) | 0 | 0 | 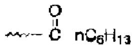 | 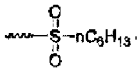 | - | - |
| 611 | O | N | N($CH_3$) | 0 | 0 | $CH_3$ | $CH_3$ | - | - |
| 612 | O | N | N(iPr) | 1 | 1 | 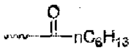 | N($C_4H_9$)$_2$ | $OCH_3$ | $OCH_3$ |
| 613 | O | N | N($CH_3$) | 0 | 0 | 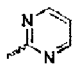 | n-$C_5H_{11}$ | - | - |
| 614 | O | N | N($CH_3$) | 2 | 2 | $CH_3$ | $CH_3$ | $CH_3$ | $CH_3$ |
| 615 | O | N($CH_3$) | N | 0 | 0 | $CH_3$ | $CH_3$ | - | - |
| 616 | O | NH | N | 0 | 0 | n-$C_{14}H_{25}$ | n-$C_{14}H_{29}$ | - | - |
| 817 | O | N($CH_3$) | N | 0 | 0 | 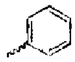 | 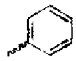 | - | - |
| 618 | O | N($CH_3$) | N | 0 | 0 | 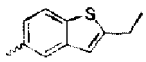 | 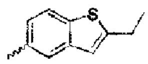 | - | - |
| 619 | O | N($CH_3$) | N | 1 | 1 | $CH_3$ | $CH_3$ | $CH_3$ | $CH_3$ |
| 620 | O | N($CH_3$) | N | 0 | 0 | 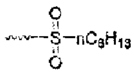 | 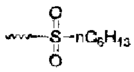 | - | - |
| 621 | O | N(n-$C_9H_{19}$) | N | 0 | 0 | $CH_3$ | $CH_3$ | - | - |
| 622 | O | N($CH_3$) | N | 1 | 1 | 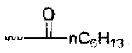 | 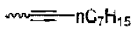 | 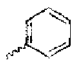 | 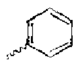 |
| 623 | O | N(iPr) | N | 0 | 0 | 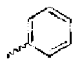 | n-$C_5H_{11}$ | - | - |
| 624 | O | N($CH_3$) | N | 2 | 2 | $CH_3$ | $CH_3$ | $CH_3$ | $CH_3$ |

| Specific Example | X | Y | Z | m | n | R1 | R2 | R3 | R4 |
|---|---|---|---|---|---|---|---|---|---|
| 625 | S | N($CH_3$) | N | 0 | 0 | $CH_3$ | $CH_3$ | - | - |

(continued)

| Specific Example | X | Y | Z | m | n | $R^1$ | $R^2$ | $R^3$ | $R^4$ |
|---|---|---|---|---|---|---|---|---|---|
| 626 | S | NH | N | 0 | 0 | 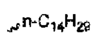 | 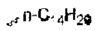 | - | - |
| 627 | S | $N(CH_3)$ | N | 0 | 0 | 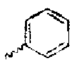 | 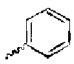 | - | - |
| 628 | S | $N(CH_3)$ | N | 0 | 0 | 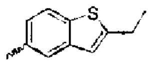 | 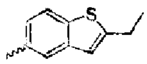 | - | - |
| 629 | S | $N(CH_3)$ | N | 1 | 1 | 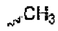 | 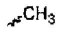 | 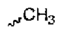 | 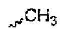 |
| 630 | S | $N(CH_3)$ | N | 0 | 0 | 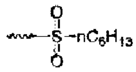 | 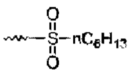 | - | - |
| 631 | S | $N(n\text{-}C_9H_{19})$ | N | 0 | 0 | 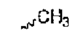 | 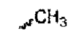 | - | - |
| 632 | S | $N(CH_3)$ | N | 1 | 1 | 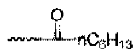 | 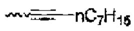 | 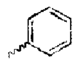 | 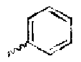 |
| 633 | S | N(iPr) | N | 0 | 0 | 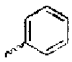 | 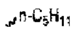 | - | - |
| 634 | S | $N(CH_3)$ | N | 2 | 2 | 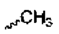 | 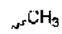 | 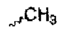 | 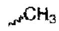 |

As the fused heterocyclic compound included in the composition according to the embodiment of the present invention, one kind may be used alone, or two or more kinds may be used.

The compound represented by Formula (1) can be synthesized using a typical method. The synthesis of the compound can be found in, for example, Examples of JP2015-195362A.

The content of the fused heterocyclic compound in the composition according to the embodiment of the present invention (in a case where the composition according to the embodiment of the present invention includes two or more fused heterocyclic compounds, the total content thereof; hereinafter, the same shall be applied) is preferably 0.01 to 2 mass% and more preferably 0.02 to 1 mass% with respect to the total mass of the composition according to the embodiment of the present invention. In a case where the content is 0.01 mass% or higher, an organic semiconductor element having a high carrier mobility can be obtained. On the other hand, in a case where the content is lower than 2 mass%, the composition according to the embodiment of the present invention can be suitably applied to a printing method, in particular, an ink jet printing or flexographic printing method.

The content of the fused heterocyclic compound in the composition according to the embodiment of the present invention is preferably 0.1 to 40 mass%, more preferably 0.5 to 30 mass%, and still more preferably 1 to 15 mass% with respect to the total mass of the solid content.

In addition, the content of the fused heterocyclic compound is preferably 0.1 to 50 mass% and more preferably 0.5 to 20 mass% with respect to the total mass of the solid content excluding the binder polymer described below.

Further, the content of the fused heterocyclic compound with respect to the total mass of the organic semiconductors included in the composition according to the embodiment of the present invention is appropriately set in a range satisfying the above-described content. From the viewpoint of carrier mobility, the content of the fused heterocyclic compound is preferably higher than 0 mass% and 20 mass% or lower and more preferably 0.5 to 15 mass%.

In a case where the composition according to the embodiment of the present invention includes an organic semiconductor other than the low molecular weight organic semiconductor compound and the fused heterocyclic compound described below, the content of the fused heterocyclic compound is preferably 0.01 mass% or higher, more preferably 0.1 mass% or higher, and still more preferably 0.5 mass% or higher with respect to the total mass of the organic semiconductors.

In the composition according to the embodiment of the present invention, a ratio between the contents of the low molecular weight organic semiconductor compound and the fused heterocyclic compound is not particularly limited as long as the contents of the low molecular weight organic semiconductor compound and the fused heterocyclic compound in the composition according to the embodiment of the present invention satisfy the above-described ranges. For example, a mass ratio low molecular weight organic semiconductor compound:fused heterocyclic compound is preferably 70:30 to 99:1, more preferably 80:20 to 97:3, and still more preferably 85:15 to 95:5.

<Organic Semiconductor other than Low Molecular Weight Organic Semiconductor Compound and Fused Heterocyclic Compound>

The composition according to the embodiment of the present invention may also include an organic semiconductor other than the low molecular weight organic semiconductor compound and the fused heterocyclic compound. This organic semiconductor is not particularly limited as long as it is used in an organic semiconductor element.
The content of the organic semiconductor in the composition according to the embodiment of the present invention is not particularly limited as long as the contents of the low molecular weight organic semiconductor compound and the fused heterocyclic compound satisfy the above-described ranges in a case where the composition according to the embodiment of the present invention includes an organic semiconductor other than the low molecular weight organic semiconductor compound and the fused heterocyclic compound described below, and is appropriately determined.

<Solvent>

It is preferable that the composition according to the embodiment of the present invention includes a solvent.
The solvent is not particularly limited as long as the organic semiconductors are soluble therein at appropriate concentrations. Examples of the solvent include the following solvents.
A hydrocarbon compound such as hexane, octane, decane, toluene, xylene, mesitylene, ethylbenzene, amylbenzene, decaline, 1-methoxytoluene, 1-methylnaphthalene, 1-ethylnaphthalene, 2-ethylnaphthalene, 2-isopropylnaphthalene, 1,6-dimethylnaphthalene, or tetralin, a ketone compound such as acetone, methyl ethyl ketone, methyl isobutyl ketone, cyclohexanone, acetophenone, propiophenone, or butyrophenone, a halogenated hydrocarbon compound such as dichloromethane, chloroform, tetrachloromethane, dichloroethane, trichloroethane, tetrachloroethane, chlorobenzene, 1,2-dichlorobenzene, 1,2,4-trichlorobenzene, chlorotoluene, 1-chloronaphthalene, or 1-fluoronaphthalene, a heterocyclic compound such as pyridine, picoline, quinoline, thiophene, 3-butylthiophene, or thieno[2,3-b]thiophene, a halogenated heterocyclic compound such as 2-chlorothiophene, 3-chlorothiophene, 2,5-dichlorothiophene, 3,4-dichlorothiophene, 2-bromothiophene, 3-bromothiophene, 2,3-dibromothiophene, 2,4-dibromothiophene, 2,5-dibromothiophene, 3,4-dibromothiophene, or 3,4-dichloro-1,2,5-thiadiazole, an ester compound such as ethyl acetate, butyl acetate, amyl acetate, 2-ethylhexyl acetate, $\gamma$-butyrolactone, or phenyl acetate, an alcohol compound such as methanol, propanol, butanol, pentanol, hexanol, cyclohexanol, methyl cellosolve, ethyl cellosolve, or ethylene glycol, an ether compound such as dibutyl ether, tetrahydrofuran, dioxane, dimethoxyethane, anisole, ethoxybenzene, propoxybenzene, isopropoxybenzene, butoxybenzene, 2-methylanisole, 3-methylanisole, 4-methylanisole, 4-ethylanisole, dimethylanisole (any one of 2,3-, 2,4-, 2,5-, 2,6-, 3,4-, 3,5-, or 3,6-), or 1,4-benzodioxane, an amide compound or an imide compound such as N,N-dimethylformamide, N,N-dimethylacetamide, 1-methyl-2-pyrrolidone, 1-methyl-2-imidazolidinone, or 1,3-dimethyl-2-imidazolidinone, a sulfoxide compound such as dimethyl sulfoxide, a phosphate compound such as trimethyl phosphate, a nitrile compound such as acetonitrile or benzonitrile, a nitro compound such as nitromethane or nitrobenzene, and/or water can be used.
As the solvent, one kind may be used alone, or a combination of plural kinds may be used. It is preferable that an appropriate solvent is selected according to a printing method. In particular, one kind or two or more kinds selected from the hydrocarbon compound, the halogenated hydrocarbon compound, the heterocyclic compound, the halogenated heterocyclic compound, or the ether compound are preferable, one kind or two or more kinds selected from toluene, xylene, mesitylene, amylbenzene, tetralin, acetophenone, propiophenone, butyrophenone, chlorobenzene, dichlorobenzene, anisole, ethoxybenzene, propoxybenzene, isopropoxybenzene, butoxybenzene, 2-methylanisole, 3-methylanisole, 4-methylanisole, 1-methylnaphthalene, 1-ethylnaphthalene, 2-ethylnaphthalene, 2-isopropylnaphthalene, 1,6-dimethylnaphthalene, 1-chloronaphthalene, 1-fluoronaphthalene, 3-chlorothiophene, or 2,5-dibromothiophene are more preferable, and one kind or two or more kinds selected from amylbenzene, toluene, xylene, tetralin, acetophenone, propiophenone, butyrophenone, anisole, ethoxybenzene, propoxybenzene, butoxybenzene, 2-methylanisole, 3-methylanisole, 4-methylanisole, 1-methylnaphthalene, 1-ethylnaphthalene, 2-ethylnaphthalene, 2-isopropylnaphthalene, 1,6-dimethylnaphthalene, 1-chloronaphthalene, 1-fluoronaphthalene, 3-chlorothiophene, or 2,5-dibromothiophene are still more preferable.
It is preferable that the composition according to the embodiment of the present invention includes at least one solvent having a boiling point of 180°C or higher among the above-described solvents. In the present invention, the boiling point refers to a boiling point at 1 atm. From the viewpoint of carrier mobility, the boiling point of the solvent is preferably 180°C to 280°C and more preferably 200°C to 260°C.

It is preferable that the composition according to the embodiment of the present invention includes at least one solvent having a boiling point of 180°C and having a SP value of 17.0 to 23.0 $MPa^{1/2}$. From the viewpoint of carrier mobility, the SP value of the solvent is more preferably 18.0 to 22.0 $MPa^{1/2}$ and still more preferably 19.0 to 21.0 $MPa^{1/2}$. By adjusting the solvent having a boiling point and a SP value in the above-described range, the organic semiconductors can be dissolved with a high solubility, and the composition having an appropriate concentration can be prepared. In addition, an organic semiconductor element having excellent carrier mobility and hysteresis can be manufactured.

In the present invention, the SP value refers to a solubility parameter value. In the present invention, the SP value refers to a Hansen solubility parameter obtained from an expression described in "Hansen Solubility Parameter: A User's Handbook, Second Edition, C. M. Hansen (2007), Taylor and Francis Group, LLC (HSPiP Manual). Specifically, the SP value can be calculated from the following expression using "Hansen Solubility Parameter HSPiP Third Edition" (Software Version 4.0.05).

$$(\text{SP value})^2=(\delta Hd)^2+(\delta Hp)^2+(\delta Hh)^2$$

Hd: Dispersion contribution
Hp: Polar contribution
Hh: Hydrogen bond contribution

The solvent having a boiling point of 180°C or higher and a SP value of 17.0 to 23.0 $MPa^{1/2}$ is not particularly limited, and examples thereof include amylbenzene (boiling point: 205°C and SP value: 17.5 $MPa^{1/2}$), tetralin (boiling point: 208°C and SP value: 19.6 $MPa^{1/2}$), 2-isopropylnaphthalene (boiling point: 261°C and SP value: 19.0 $MPa^{1/2}$), 1-methylnaphthalene (boiling point: 204°C and SP value: 20.0 $MPa^{1/2}$), 1-ethylnaphthalene (boiling point: 260°C and SP value: 19.6 $MPa^{1/2}$), 2-ethylnaphthalene (boiling point: 251°C and SP value: 19.6 $MPa^{1/2}$), 1,6-dimethylnaphthalene (boiling point: 265°C and SP value: 19.4 $MPa^{1/2}$), 1-chloronaphthalene (boiling point: 259°C and SP value: 20.8 $MPa^{1/2}$), and 1-fluoronaphthalene (boiling point: 215°C and SP value: 20.3 $MPa^{1/2}$).

It is more preferable that the solvent included in the composition according to the embodiment of the present invention has a naphthalene skeleton, and it is still more preferable that the solvent having a boiling point of 180°C and having a SP value of 17.0 to 23.0 $MPa^{1/2}$ has a naphthalene skeleton. The solvent having a naphthalene skeleton is not particularly limited, and examples thereof include the above-described examples. With the composition according to the embodiment of the present invention that includes at least one solvent having a naphthalene skeleton, an organic semiconductor element having reduced hysteresis can be manufactured.

In the composition according to the embodiment of the present invention, the content of the solvent is preferably 60 mass% or higher, more preferably 80 mass% or higher, and still more preferably 90 mass% or higher. In the composition according to the embodiment of the present invention, the content of the solvent is lower than 100 mass%, and a part or all of the remainder excluding the solvent are formed by the organic semiconductors and additives described below.

In a case where the composition according to the embodiment of the present invention includes the solvent having a naphthalene skeleton, the content of the solvent in the composition according to the embodiment of the present invention is not particularly limited as long as it satisfies the above-described content of the solvent, and is preferably 50 mass% and, from the viewpoint of carrier mobility and hysteresis, is more preferably 70 mass% or higher and still more preferably 90 mass% or higher.

<Additives>

The composition according to the embodiment of the present invention may include various additives which are typically used in an organic semiconductor film of an organic semiconductor element. Examples of the additives include a binder polymer.

(Binder Polymer)

As the binder polymer, any binder polymer which is typically used in an organic semiconductor film can be used without any particular limitation.

Examples of the binder polymer include an insulating polymer such as polystyrene, poly($\alpha$-methylstyrene), polyvinyl cinnamate, poly(4-divinylbenzene), poly(4-vinyl phenol), poly(4-methylstyrene), polycarbonate, polyarylate, polyester, polyamide, polyimide, polyurethane, polysiloxane, polysulfone, polymethyl methacrylate, polymethyl acrylate, cellulose, polyethylene, or polypropylene and a copolymer thereof, a rubber, and a thermoplastic elastomer.

The weight-average molecular weight of the binder polymer is not particularly limited and is preferably 1000 to

10000000, more preferably 3000 to 5000000, and still more preferably 5000 to 3000000. A measurement method will be described below.

As the binder polymer, one kind may be included, or two or more kinds may be included.

The content of the binder polymer in the composition according to the embodiment of the present invention is preferably 5 mass% or lower, more preferably 2 mass% or lower, and still more preferably 1 mass% or lower.

In addition, the content of the binder polymer is preferably 1 to 80 mass%, more preferably 5 to 50 mass%, and still more preferably 10 to 42 mass% with respect to the total mass of the solid content in the composition according to the embodiment of the present invention.

(Additives other than Binder Polymer)

As the additives (other additives) other than the binder polymer, any additives which are typically used in the composition according to the embodiment of the present invention can be used without any particular limitation.

The content of the other additives in the composition according to the embodiment of the present invention is preferably 10 mass% or lower, more preferably 5 mass% or lower, and still more preferably 1 mass% or lower.

In addition, the content of the other additives is preferably 50 mass% or lower, more preferably 30 mass% or lower, and still more preferably 10 mass% or lower with respect to the total mass of the solid content in the composition according to the embodiment of the present invention.

In a case where the content is in the above-described range, film forming properties are excellent. For example, in a case where an organic semiconductor film of an organic semiconductor element is formed using the composition according to the embodiment of the present invention in which the content of the other additives is in the above-described range, film forming properties are excellent, and the carrier mobility and heat resistance of the organic semiconductor element are further improved.

<Physical Properties or Characteristics of Composition according to Present Invention>

The viscosity of the composition is not particularly limited and, from the viewpoint of obtaining excellent coating properties in addition to the effects of the present invention, is preferably 1 to 50 mPa·s, 1.5 to 20 mPa·s, and still more preferably 2 to 10 mPa·s. In the present invention, the viscosity is a value measured 25°C using a vibration viscometer (VM-10A, manufactured by Sekonic Corporation).

<Preparation Method>

A preparation method of the composition according to the embodiment of the present invention is not particularly limited, and a typical preparation method can be adopted. For example, the composition according to the embodiment of the present invention can be prepared by mixing predetermined amounts of the respective components with the solvent and appropriately stirring the components.

[Organic Semiconductor Film]

An organic semiconductor film according to the embodiment of the present invention will be described.

The organic semiconductor film according to the embodiment of the present invention includes the low molecular weight organic semiconductor compound and the fused heterocyclic compound described above. In a case where the organic semiconductor film including the low molecular weight organic semiconductor compound and the fused heterocyclic compound is used for an organic semiconductor element, in particular, an organic thin film transistor, a high carrier mobility and small hysteresis are exhibited. The reason for this is not clear but is presumed to be as follows.

That is, it is presumed that, by using the low molecular weight organic semiconductor compound and the fused heterocyclic compound in combination, an organic semiconductor in which crystal grains of the fused heterocyclic compound are arranged between crystal grains of the low molecular weight organic semiconductor compound is formed. As a result, in the organic semiconductor film, disconnection of a carrier path is prevented, and a high carrier mobility is exhibited. In addition, the low molecular weight organic semiconductor compound and the fused heterocyclic compound are likely to embrace the solvent in the process of forming the organic semiconductor film, and it is presumed that an organic semiconductor film including no solvent or an organic semiconductor film having a small amount of residual solvent is formed. As a result, the organic semiconductor film exhibits small hysteresis.

In addition to the low molecular weight organic semiconductor compound and the fused heterocyclic compound, the organic semiconductor film according to the embodiment of the present invention may further include an organic semiconductor other than the low molecular weight organic semiconductor compound and the fused heterocyclic compound or the above-described additives.

As each of the low molecular weight organic semiconductor compound and the fused heterocyclic compound included in the organic semiconductor film according to the embodiment of the present invention, one kind may be used, or two or more kinds may be used.

The content of each of the low molecular weight organic semiconductor compound and the fused heterocyclic compound in the organic semiconductor film is the same as the content thereof with respect to the total mass of the solid content in the composition according to the embodiment of the present invention, and preferable ranges thereof are also the same.

As the organic semiconductor other than the low molecular weight organic semiconductor compound and the fused heterocyclic compound included in the organic semiconductor film, one kind may be used, or two or more kinds may be used. The content is as described above.

As each of the additives included in the organic semiconductor film, one kind may be used, or two or more kinds may be used.

The content of each of the additives in the organic semiconductor film is the same as the content thereof with respect to the total mass of the solid content in the composition according to the embodiment of the present invention, and preferable ranges thereof are also the same.

The thickness of the organic semiconductor film cannot be uniquely determined according to the organic semiconductor element to be applied. For example, in a case where the organic semiconductor film is applied to an organic thin film transistor, the thickness of the organic semiconductor film is preferably 10 to 500 nm and more preferably 10 to 200 nm.

It is preferable that the organic semiconductor film is formed with a method described below using the above-described composition according to the embodiment of the present invention.

The organic semiconductor film according to the embodiment of the present invention is applied to preferably an organic semiconductor element and more preferably an organic thin film transistor.

[Organic Semiconductor Element]

Next, an organic semiconductor element according to the embodiment of the present invention will be described.

The organic semiconductor element according to the embodiment of the present invention includes the organic semiconductor film according to the embodiment of the present invention.

The organic semiconductor element according to the embodiment of the present invention is not particularly limited and is preferably used as a non-light-emitting organic semiconductor device. The non-light-emitting organic semiconductor device is not particularly limited as long as it is a device that does not emit light, and examples thereof include an organic thin film transistor that controls a current amount or a voltage amount, an organic photoelectric conversion element (for example, a solid image pickup element for an optical sensor or a solar cell for energy conversion) that converts light energy into electric power, an organic thermoelectric conversion element that converts thermal energy into electric power, a gas sensor, an organic rectifying element, an organic inverter, and an information recording element. In the non-light-emitting organic semiconductor device, it is preferable that the organic semiconductor film functions as an electronic element.

In particular, it is preferable that the organic semiconductor element according to the embodiment of the present invention is an organic thin film transistor.

<Organic Thin Film Transistor>

An organic thin film transistor as a preferable aspect of the organic semiconductor element according to the embodiment of the present invention will be described.

An organic thin film transistor (also referred to as "organic TFT") that is obtained using the method according to the embodiment of the present invention includes the above-described organic semiconductor film according to the embodiment of the present invention, and may further include a source electrode, a drain electrode, and a gate electrode.

The organic TFT according to the present invention includes, on a substrate, a gate electrode, an organic semiconductor layer, a gate insulating layer that is provided between the gate electrode and the organic semiconductor layer, and a source electrode and a drain electrode that are provided adjacent to the organic semiconductor layer and are linked to each other through the organic semiconductor layer. In the organic TFT, the organic semiconductor layer and the gate insulating layer are provided adjacent to each other.

A structure of the organic TFT according to the embodiment of the present invention is not particularly limited as long as it includes the respective layers. For example, any structure such as a bottom contact type (a bottom gate-bottom contact type and a top gate-bottom contact type) or a top contact type (a bottom gate-top contact type and a top gate-top contact type) may be adopted. It is more preferable that the organic TFT according to the present invention is a bottom gate-bottom contact type or bottom gate-top contact type (collectively referred to as "bottom gate type").

Hereinafter, an example of the organic TFT according to the present invention will be described with reference to the accompanying drawings.

(Bottom Gate-Bottom Contact type Organic Thin Film Transistor)

Fig. 1 is a schematic cross-sectional view showing a bottom gate-bottom contact type organic TFT 100 as an example of the organic semiconductor element according to the embodiment of the present invention.

As shown in Fig. 1, the organic TFT 100 includes a substrate (base material) 10, a gate electrode 20, a gate insulating layer 30, a source electrode 40 and a drain electrode 42, an organic semiconductor layer 50, and a sealing layer 60 in this order.

Hereinafter, the substrate (base material), the gate electrode, the gate insulating layer (film), the source electrode, the drain electrode, the organic semiconductor layer, and the sealing layer, and preparation methods thereof will be described in detail.

- Substrate -

The substrate functions to support the gate electrode, the source electrode, the drain electrode, and the like described below.

The kind of the substrate is not particularly limited, and examples thereof include a plastic substrate, a silicon substrate, a glass substrate, and a ceramic substrate. In particular, from the viewpoints of versatility, applicability to each device and costs, a silicon substrate, a glass substrate, or a plastic substrate is preferable.

The thickness of the substrate is not particularly limited and is, for example, preferably 10 mm or less, more preferably 2 mm or less, and still more preferably 1.5 mm or less. On the other hand, the thickness of the substrate is preferably 0.01 mm or more and more preferably 0.05 mm or more.

- Gate Electrode -

As the gate electrode, a typical electrode that is used as a gate electrode of an organic TFT can be used without any particular limitation.

A material (electrode material) which forms the gate electrode is not particularly limited, and examples thereof include: a metal such as gold, silver, aluminum, copper, chromium, nickel, cobalt, titanium, platinum, magnesium, calcium, barium, or sodium; a conductive oxide such as $InO_2$, $SnO_2$, or indium tin oxide (ITO); a conductive polymer such as polyaniline, polypyrrole, polythiophene, polyacetylene, or polydiacetylene; a semiconductor such as silicon, germanium, or gallium-arsenic; and a carbon material such as fullerene, carbon nanotube, or graphite. Among these, the metal is preferable, and silver or aluminum is more preferable.

The thickness of the gate electrode is not particularly limited and is preferably 20 to 200 nm.

The gate electrode may function as the substrate such as a silicon substrate. In this case, the substrate is not necessarily provided.

A method of forming the gate electrode is not particularly limited, and examples thereof include a method of performing vacuum deposition (hereinafter, simply referred to as "deposition") or sputtering on the substrate using the above-described electrode material and a method of applying or printing an electrode-forming composition including the above-described electrode material. In addition, in a case where an electrode is patterned, examples of a patterning method include a printing method such as ink jet printing, screen printing, offset printing, or relief printing (flexographic printing); a photolithography method, and a mask deposition method.

- Gate Insulating Layer -

The gate insulating layer is not particularly limited as long as it is an insulating layer provided between the gate electrode and the organic semiconductor layer, and may have a single-layer structure or a multi-layer structure.

It is preferable that the gate insulating layer is formed of an insulating material, and preferable examples of the insulating material include an organic material such as an organic polymer and an inorganic material such as an inorganic oxide. From the viewpoint of handleability and the like, in a case where a plastic substrate or a glass substrate is used, it is preferable that an organic material is used.

The organic polymer, the inorganic oxide, or the like is not particularly limited as long as it has insulating characteristics, and an organic polymer or an inorganic oxide with which a thin film, for example, a thin film having a thickness of 1 $\mu$m or less can be formed is preferable.

As the organic polymer or the inorganic oxide, one kind may be used alone, and two or more kinds may be used in combination. In addition, the gate insulating layer may be a hybrid layer formed of a mixture of the organic

polymer and the inorganic oxide described below.

The organic polymer is not particularly limited, and examples thereof include: a poly(meth)acrylate such as polyvinyl phenol, polystyrene (PS), or polymethyl methacrylate; a cyclic fluoroalkyl polymer such as polyvinyl alcohol, polyvinyl chloride (PVC), polyvinylidene fluoride (PVDF), polytetrafluoroethylene (PTFE), or CYTOP (registered trade name); a polyorganosiloxane such as polycycloolefin, polyester, polyethersulfone, polyether ketone, polyimide, poly(meth)acrylic acid, polybenzoxazole, an epoxy resin, or polydimethylsiloxane (PDMS); polysilsesquioxane; and butadiene rubber. In addition to the above-described examples, a thermosetting resin such as a phenolic resin, a novolac resin, a cinnamate resin, an acrylic resin, or a polyparaxylylene resin may also be used.

The organic polymer can also be used in combination with a compound having a reactive substituent such as an alkoxysilyl group, a vinyl group, an acryloyloxy group, an epoxy group, or a methylol group.

In a case where the gate insulating layer is formed of the organic polymer, it is preferable that the organic polymer is crosslinked and cured, for example, in order to improve solvent resistance or insulation resistance of the gate insulating layer. It is preferable that crosslinking is performed by generating an acid or a radical using either or both light and heat.

In a case where the organic polymer is crosslinked by a radical, as a radical generator that generates a radical using light or heat, for example, a thermal polymerization initiator (H1) and a photopolymerization initiator (H2) described in paragraphs "0182" to "0186" of JP2013-214649A, a photoradical generator described in paragraphs "0046" to "0051" of JP2011-186069A, or a photoradical polymerization initiator described in paragraphs "0042" to "0056" of JP2010-285518A can be preferably used, the contents of which are preferably incorporated herein by reference.

In addition, "a compound (G) having a number-average molecular weight (Mn) of 140 to 5000, having a crosslinking functional group, and not having a fluorine atom" which is described in paragraphs "0167" to "0177" of JP2013-214649A can also be preferably used, the contents of which are incorporated herein by reference.

In a case where the organic polymer is crosslinked by an acid, as a photoacid generator that generates an acid using light, for example, a photocationic polymerization initiator described in paragraphs "0033" and "0034" of JP2010-285518A or an acid generator, in particular, a sulfonium salt or an iodonium salt described in paragraphs "0120" to "0136" of JP2012-163946A can be preferably used, the contents of which are preferably incorporated herein by reference.

As a thermal acid generator (catalyst) that generates an acid using heat, for example, a thermal cationic polymerization initiator, in particular, an onium salt described in paragraphs "0035" to "0038" of JP2010-285518A or a catalyst, in particular, a sulfonic acid or a sulfonic acid amine salt described in paragraphs "0034" and "0035" of JP2005-354012A can be preferably used, the contents of which are preferably incorporated herein by reference.

In addition, a crosslinking agent, in particular, a bifunctional or higher epoxy compound or oxetane compound described in paragraphs "0032" and "0033" of JP2005-354012A, a crosslinking agent, in particular, a compound having two or more crosslinking groups at least one of which is a methylol group or an NH group described in paragraphs "0046" to "0062" of JP2006-303465A, or a compound having two or more hydroxymethyl groups or alkoxymethyl groups in a molecule described in paragraphs "0137" to "0145" of JP2012-163946A is also preferably used, the contents of which are preferably incorporated herein by reference.

A method of forming the gate insulating layer using the organic polymer is not particularly limited, and examples thereof include a method of applying a coating solution including the organic polymer and optionally curing the applied coating film.

The solvent used in the coating solution is not particularly limited as long as the organic polymer is soluble or dispersible therein, and can be appropriately selected from typically used solvents according to the kind and the like of the organic polymer.

A coating method is not particularly limited, and examples thereof include the above-described printing methods. Among these, a wet coating method such as a microgravure coating method, a dip coating method, a screen coating printing method, a die coating method, or a spin coating method is preferable.

Coating conditions are also not particularly limited and can be appropriately set.

A method and conditions for curing are not particularly limited as long as they are a method and conditions in which the organic polymer can be crosslinked. For example, the crosslinking method can be appropriately set according to the kind and the like of a photoacid generator, a thermal acid generator, or the like to be used.

The inorganic oxide is not particularly limited, and examples thereof include: an oxide such as silicon oxide, silicon nitride (SiNy), hafnium oxide, titanium oxide, tantalum oxide, aluminum oxide, niobium oxide, zirconium oxide, copper oxide, or nickel oxide; a compound having a perovskite structure such as $SrTiO_3$, $CaTiO_3$, $BaTiO_3$, $MgTiO_3$, or $SrNb_2O_6$; and a composite oxide or a mixture thereof.

Examples of the silicon oxide include silicon oxide (SiOx), boron phosphorus silicon glass (BPSG), phosphorus silicon glass (PSG), borosilicate glass (BSG), arsenic silicate glass (AsSG), lead silicate glass (PbSG), silicon nitride oxide (SiON), spin-on-glass (SOG), and a low dielectric constant $SiO_2$ material (for example, polyarylether, a cycloperfluorocarbon polymer, benzocyclobutene, a cyclic fluororesin, polytetrafluoroethylene, fluorinated aryl ether, fluorinated

polyimide, amorphous carbon, or organic SOG).

A method of forming the gate insulating layer using the inorganic oxide is not particularly limited. For example, a vacuum film formation method such as a vacuum deposition method, a sputtering method, or an ion plating or chemical vapor deposition (CVD) method can be used. In addition, the film formation may be assisted with plasma using predetermined gas, an ion gun, or a radical gun.

In addition, the gate insulating layer can also be formed by causing a precursor corresponding to each of metal oxides, specifically, a metal halide such as a chloride or a bromide, a metal alkoxide, a metal hydroxide, or the like is to react with an acid such as hydrochloric acid, sulfuric acid, or nitric acid or a base such as sodium hydroxide or potassium hydroxide in alcohol or water for hydrolysis. In a case where the solution-based process is used, the wet coating method can be used.

In a case where the gate insulating layer is formed using the inorganic oxide, in addition to the above-described method, a combination of any one of a lift-off method, a sol-gel method, an electrodeposition method, or a shadow mask method with a patterning method can also be optionally used.

The gate insulating layer may undergo a surface treatment such as a corona treatment, a plasma treatment, or an ultraviolet (UV)/ozone treatment.

In this case, it is preferable that a surface roughness of the gate insulating layer is not high. For example, it is preferable that an arithmetic average roughness Ra or a root-mean-square roughness $R_q$ (both of which are defined according to JIS B0601:2013) of the treated gate insulating layer surface is 0.5 nm or lower.

The thickness of the gate insulating layer is not particularly limited and is preferably 100 to 1000 nm.

- Source Electrode and Drain Electrode -

In the organic TFT according to the embodiment of the present invention, the source electrode is an electrode into which charges flow from the outside through a wiring. In addition, the drain electrode is an electrode from which charges flow to the outside through a wiring.

As a material which forms the source electrode and the drain electrode, the same electrode material as that of the gate electrode can be used. Among these, a metal is preferable, and gold or silver is more preferable. In addition, it is preferable that a charge injection layer is provided between the metal and the organic semiconductor so as to promote charge injection from the source electrode into the organic semiconductor and to improve mobility.

The thickness of each of the source electrode and the drain electrode is not particularly limited and is preferably 1 nm or more and more preferably 10 nm or more. In addition, the thickness of each of the source electrode and the drain electrode is preferably 500 nm or less and more preferably 300 nm or less.

An interval (gate length) between the source electrode and the drain electrode can be appropriately determined and is, for example, preferably 200 μm or less and more preferably 100 μm or less. In addition, the gate width can be appropriately determined and is, for example, preferably 5000 μm or less and more preferably 1000 μm or less.

A method of forming the source electrode and the drain electrode is not particularly limited, and examples thereof include a method of performing vacuum deposition or sputtering using the electrode material on the substrate on which the gate electrode and the gate insulating layer are formed and a method of applying or printing an electrode-forming composition to or on the substrate. In a case where the source electrode and the drain electrode are patterned, a patterning method thereof is the same as that of the gate electrode.

- Organic Semiconductor Layer -

As the organic semiconductor layer in the organic TFT, the organic semiconductor film according to the embodiment of the present invention is used.

The organic semiconductor film can be formed by applying the above-described composition according to the embodiment of the present invention to a substrate. Specifically, the organic semiconductor film can be formed by applying the composition according to the embodiment of the present invention to the substrate and drying the applied composition.

In the present invention, the application of the composition according to the embodiment of the present invention to the substrate includes an aspect in which the composition according to the embodiment of the present invention is directly applied to the substrate and an aspect in which the composition according to the embodiment of the present invention is applied over the substrate through another layer provided on the substrate.

The substrate is not particularly limited as long as the composition according to the embodiment of the present invention can be applied thereto, and examples thereof include a substrate of the organic TFT and a plate-shaped body of the organic TFT other than the substrate. In a case where the substrate of the organic TFT is used, examples of the substrate include various substrates described above as the examples of the substrate 10. Examples of another layer include the gate insulating layer, the source electrode, and the drain electrode during the manufacturing of the organic

TFT. On the other hand, in a case where the organic semiconductor film is formed on the substrate of the organic TFT, a plate-shaped body of the organic TFT other than the substrate is used. Examples of this substrate include various plate-shaped bodies such as a separating plate or a glass plate.

A method of applying the composition according to the embodiment of the present invention is not particularly limited, and a typical method can be used. Examples of the method include various printing methods such as a bar coating method, a spin coating method, a knife coating method, a doctor blade method, an ink jet printing method, a flexographic printing method, a gravure printing method, or a screen printing method. Further, as the method of applying the composition according to the embodiment of the present invention, for example, a method (so-called gap casting method) of forming an organic semiconductor film described in JP2013-207085A or a method (so-called edge casting method or continuous edge casting method) of forming an organic semiconductor thin film described in WO2014/175351A can be preferably used.

In particular, as described above, the composition according to the embodiment of the present invention can be applied using a printing method. Examples of the printing method include the above-described methods. Among these, an ink jet printing method is preferable.

Conditions for ink jet printing are not particularly limited as long as the composition according to the embodiment of the present invention can be printed, and are preferably conditions of head: 1 to 10 pL, jetting frequency: 1 to 10 Hz, and inter-dot pitch: 10 to 30 $\mu$m.

As drying conditions for drying the composition according to the embodiment of the present invention, appropriate conditions can be selected according to the kinds of the respective components included in the composition according to the embodiment of the present invention. Natural drying may be performed, but heating is preferable from the viewpoint of improving productivity. Heating conditions cannot be uniquely determined. For example, the heating temperature is preferably 30°C to 250°C, more preferably 40°C to 200°C, and still more preferably 50°C to 150°C, and the heating time is preferably 10 to 300 minutes and more preferably 20 to 180 minutes.

(Sealing Layer)

In the organic TFT according to the present invention, it is preferable that the sealing layer is provided on the outermost layer from the viewpoint of durability. For the sealing layer, a sealing agent (sealing layer-forming composition) that is typically used in the organic TFT can be used.

The thickness of the sealing layer is not particularly limited and is preferably 0.1 to 10 $\mu$m.

In the present invention, in a case where a printing method is adopted as a method of forming each of the layers or the electrodes (including a case where the composition according to the embodiment of the present invention is applied using a printing method), a mask can be used. The mask is not particularly limited as long as it has an opening that is suitable for the shape, pattern, and dimension of the layer or the electrode to be formed, and a mask which is typically used in each printing method can be used.

For example, as a mask used in a case where the source electrode and the drain electrode are formed using a printing method, a metal mask 151 shown in Fig. 3 or a mask including a plurality of formation patterns of an opening shown in Fig. 3 can be used. In the metal mask 151, openings 153 and 154 having dimensions shown in Fig. 3 are provided in parallel at a predetermined interval in a flat mask main body.

<Bottom Gate-Top Contact Type Organic Thin Film Transistor>

Fig. 2 is a schematic cross-sectional view showing a bottom gate-top contact type organic TFT 200 as an example of the organic semiconductor element according to the embodiment of the present invention.

As shown in Fig. 2, the organic TFT 200 includes the substrate 10, the gate electrode 20, the gate insulating layer 30, the organic semiconductor layer 50, the source electrode 40 and the drain electrode 42, and the sealing layer 60 in this order.

The organic TFT 200 is the same as the organic TFT 100 except for the layer configuration (stack aspect). Accordingly, the details of the substrate, the gate electrode, the gate insulating layer, the source electrode, the drain electrode, the organic semiconductor layer, and the sealing layer are the same as those of the bottom gate-bottom contact type organic TFT, and thus the description thereof will not be repeated.

Examples

The present invention will be described in more detail using Examples, but the present invention is not limited to the following Examples.

[Synthesis Examples]

Organic semiconductors used in Examples will be shown below.

In the following organic semiconductors, iPr represents isopropyl, and Bu represents butyl.

| Compound No. | Fused Heterocyclic Compound | Compound No. | Fused Heterocyclic Compound |
|---|---|---|---|
| 1 | 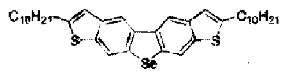 | 11 | 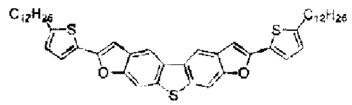 |
| 2 | 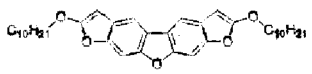 | 12 | 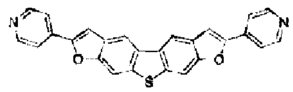 |
| 3 | 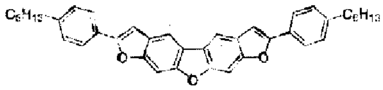 | 13 | 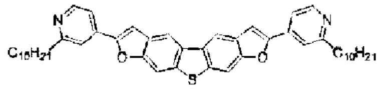 |
| 4 | 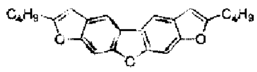 | 14 | 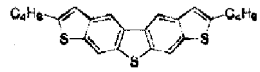 |
| 5 | 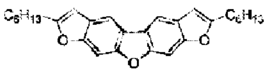 | 15 | 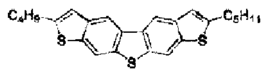 |
| 6 | 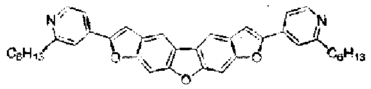 | 18 | 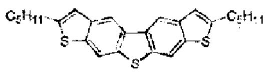 |
| 7 | 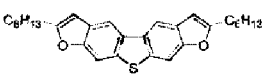 | 17 | 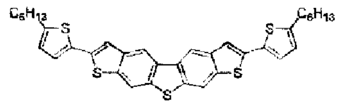 |
| 8 | 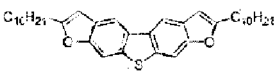 | 18 | 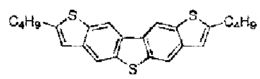 |
| 9 | 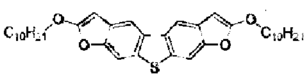 | 19 | 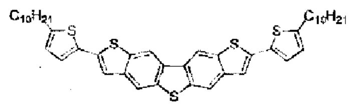 |
| 10 | 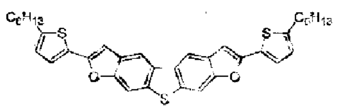 | 20 | 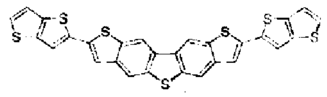 |

| Compound No. | Fused Heterocyclic Compound | Compound No. | Fused Heterocyclic Compound |
|---|---|---|---|
| 21 | 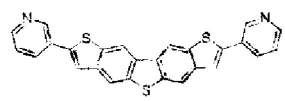 | 31 | 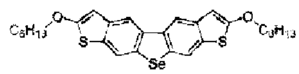 |
| 22 | 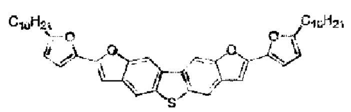 | 32 | 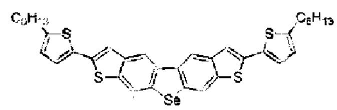 |

(continued)

| Compound No. | Fused Heterocyclic Compound | Compound No. | Fused Heterocyclic Compound |
|---|---|---|---|
| 23 | 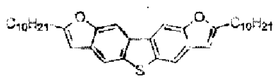 | 33 | 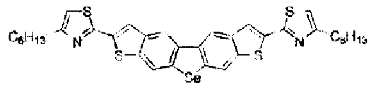 |
| 24 | 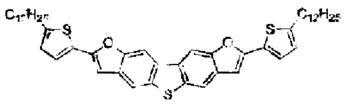 | 34 | 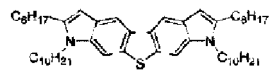 |
| 25 | 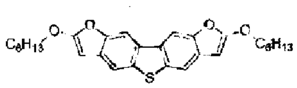 | 35 | 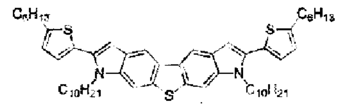 |
| 26 | 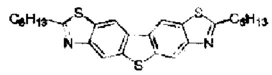 | 36 | 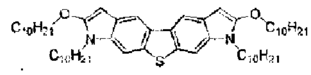 |
| 27 | 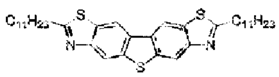 | 37 | 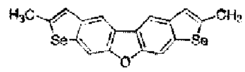 |
| 28 | 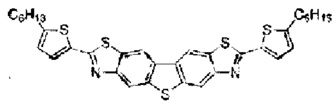 | 38 | 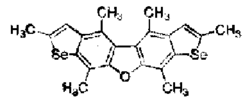 |
| 29 | 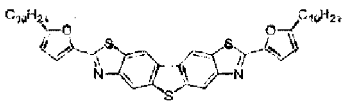 | 39 | 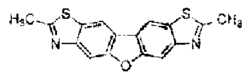 |
| 30 | 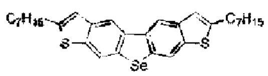 | 40 | 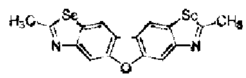 |

| Compound No. | Fused Heterocyclic Compound | Compound No. | Fused Heterocyclic Compound |
|---|---|---|---|
| 41 | 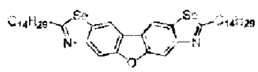 | 51 | 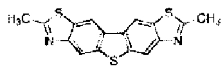 |
| 42 | 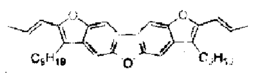 | 52 | 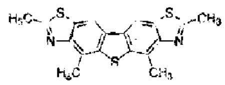 |
| 43 | 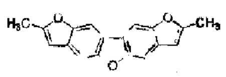 | 53 | 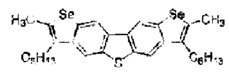 |
| 44 | 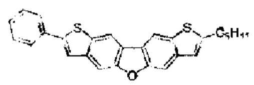 | 54 | 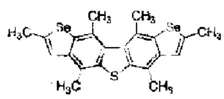 |
| 45 | 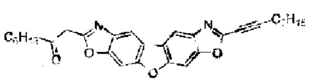 | 45 | 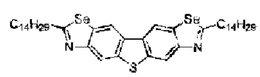 |

(continued)

| Compound No. | Fused Heterocyclic Compound | Compound No. | Fused Heterocyclic Compound |
|---|---|---|---|
| 46 | 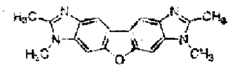 | 56 | 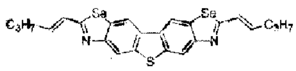 |
| 47 | 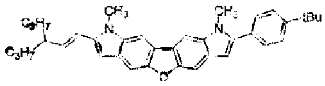 | 57 | 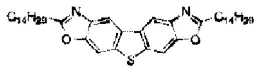 |
| 48 | 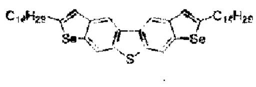 | 58 | 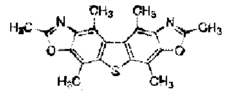 |
| 49 | 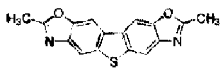 | 59 | 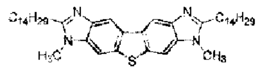 |
| 50 | 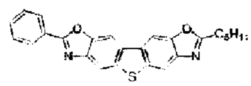 | 60 | 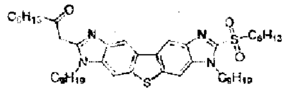 |

| Compound No. | Fused Heterocyclic Compound | Compound No. | Fused Heterocyclic Compound |
|---|---|---|---|
| 61 | 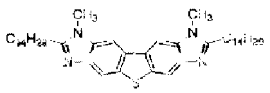 | 71 | 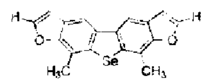 |
| 62 | 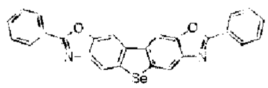 | 72 | 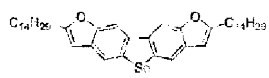 |
| 63 | 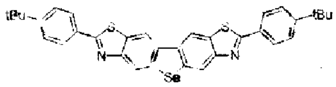 | 73 | 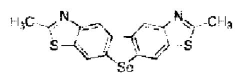 |
| 64 | 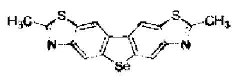 | 74 | 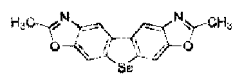 |
| 65 | 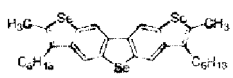 | 75 | 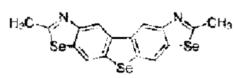 |
| 66 | 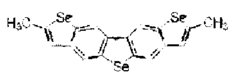 | 76 | 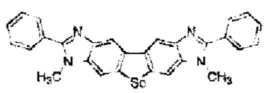 |
| 67 | 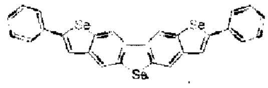 | 77 | 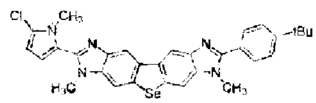 |
| 68 | 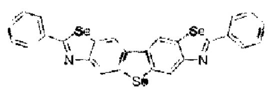 | 78 | 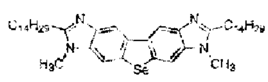 |

(continued)

| Compound No. | Fused Heterocyclic Compound | Compound No. | Fused Heterocyclic Compound |
|---|---|---|---|
| 69 | 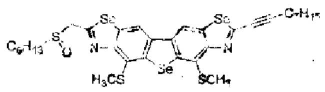 | 79 | 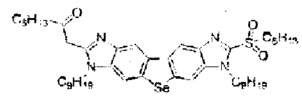 |
| 70 | 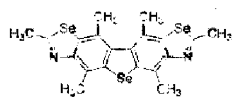 | 80 | 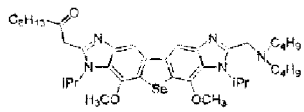 |

| Compound No. | Fused Heterocyclic Compound | Compound No. | Fused Heterocyclic Compound |
|---|---|---|---|
| 81 | 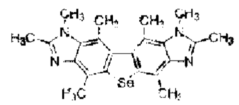 | 91 | 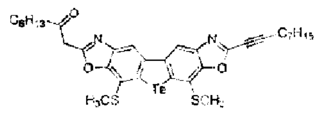 |
| 82 | 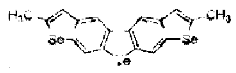 | 92 | 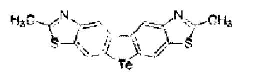 |
| 83 | 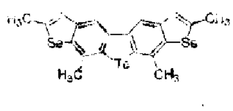 | 93 | 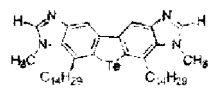 |
| 84 | 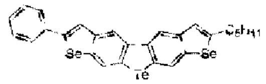 | 94 | 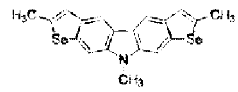 |
| 85 | 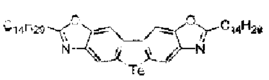 | 95 | 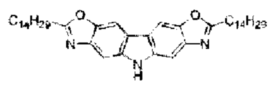 |
| 86 | 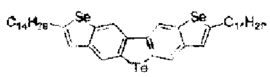 | 96 | 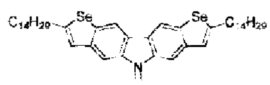 |
| 87 | 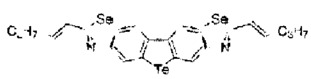 | 97 | 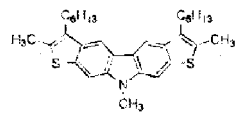 |
| 88 | 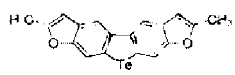 | 98 | 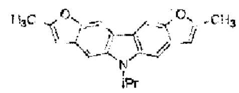 |
| 89 | 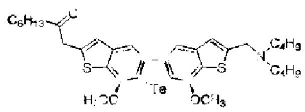 | 99 | 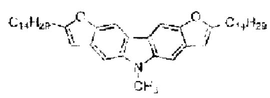 |
| 90 | 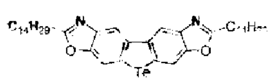 | 100 | 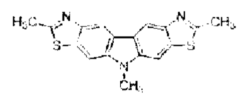 |

| Compound No. | Fused Heterocyclic Compound | Compound No. | Fused Heterocyclic Compound |
|---|---|---|---|
| 101 | 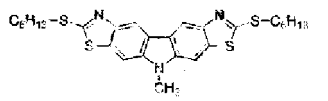 | 107 | 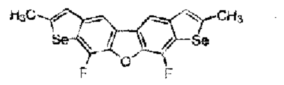 |
| 102 | 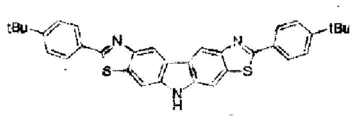 | 108 | 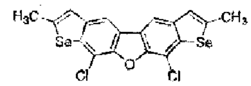 |
| 103 | 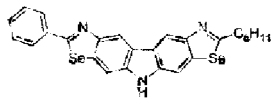 | 109 | 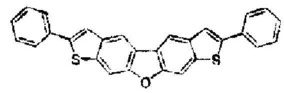 |
| 104 | 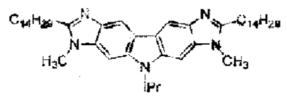 | 110 | 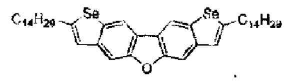 |
| 105 | 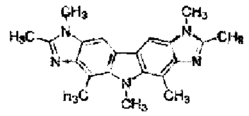 | 111 | 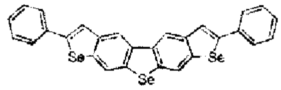 |
| 106 | 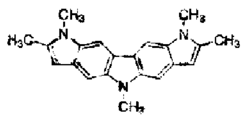 | 112 | 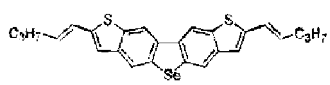 |

<Synthesis Example 1: Synthesis of Compound 1>

A compound 1 was synthesized according to the following scheme.

In the following reaction scheme, Bu represents butyl, Et represents ethyl, THF represents tetrahydrofuran, DMF represents N,N-dimethylformamide, TMP represents tetramethylpiperidine, and dppf represents 1,1'-bis(diphenylphosphino)ferrocene.

-Synthesis of Intermediate 1a-

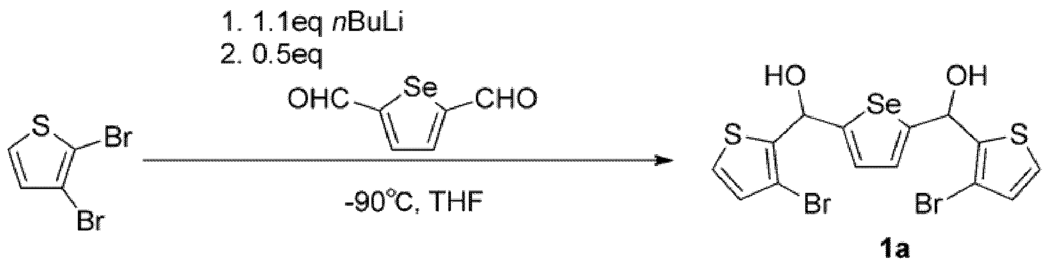

**1a**

A 2,3-dibromothiophene n-butyllithium solution (15.9 g, 65.8 mmol) was dissolved in 120 ml of diethyl ether, and n-butyllithium (1.6 M solution) was added dropwise to the solution while stirring the solution at -90°C. After 30 minutes, a solution in which 2,5-selenophene dicarboxaldehyde (6.00 g, 32.1 mmol) was dissolved in 50 ml of tetrahydrofuran was added dropwise, was stirred at -78°C for 20 minutes, and then was heated to room temperature. The reaction solution was quenched with water, and the organic layer was extracted with diethyl ether and was dried with magnesium sulfate. After concentration with an evaporator, an intermediate 1a (12.9 g) as a brown oily target material was obtained. A coarse body of the obtained target material was used for the next reaction without being purified.

$^1$H-NMR (400 MHz, $CDCl_3$) $\delta$: 7.28 (d, J=5.2 Hz, 2H), 7.04 (d, J=5.2 Hz, 2H), 6.93 (d, J=5.2 Hz, 2H), 6.31 (s, 2H)

-Synthesis of Intermediate 2a-

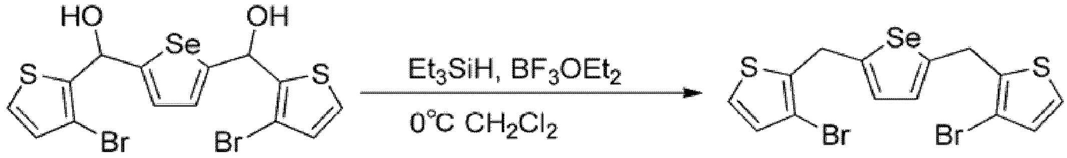


**1a** **2a**

The intermediate 1a (12.9 g) and triethylsilane (15.4 ml, 96.2 mmol) were dissolved in 70 ml of dichloromethane, and the solution was cooled to 0°C. Next, boron trifluoride etherate (11.9 ml, 96.2 mmol) was added dropwise to the solution and was stirred for 30 minutes. Next, the solution was quenched with water, the organic layer was extracted with ethyl acetate and was dried with magnesium sulfate. After concentration, the coarse body was purified by column chromatography (hexane:ethyl acetate=95:5). As a result, an intermediate 2a (9.20 g, 19.1 mmol, 60% yield for 2 steps) as a yellow oily target material was obtained.

$^{1}$H-NMR (400 MHz, $CDCl_3$) $\delta$: 7.16 (d, J=5.2 Hz, 2H), 6.92 (d, J=5.2 Hz, 2H), 6.86 (s, 2H), 4.28 (s, 4H)

-Synthesis of Intermediate 3a-

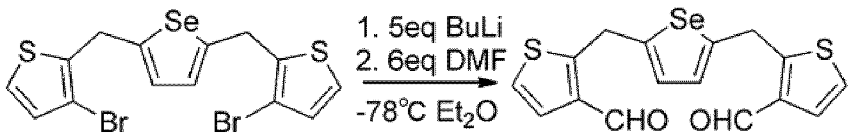


**2a** **3a**

N-butyllithium (1.6 M solution) (58.5 ml, 93.5 mmol) was cooled to -78°C, a solution in which the intermediate 2a (9.00 g, 18.7 mmol) was dissolved in 240 ml of diethyl ether was added dropwise thereto, and the solution was stirred for 30 minutes. Next, N,N-dimethylformamide (8.7 ml, 112 mmol) was added dropwise. The solution was stirred at -78°C for 20 minutes, was heated to room temperature, and was quenched with water. Next, the organic layer was extracted with diethyl ether and was dried with magnesium sulfate. After concentration, an intermediate 3a (6.50 g) as a red oily target material was obtained. A coarse body of the obtained target material was used for the next reaction without being purified.

$^{1}$H-NMR (400 MHz, $CDCl_3$) $\delta$: 10.0 (s, 2H), 7.40 (d, J=4.8 Hz, 2H), 7.15 (d, J=4.8 Hz, 2H), 6.88 (s, 2H), 4.68 (s, 4H)

-Synthesis of Intermediate 4a-

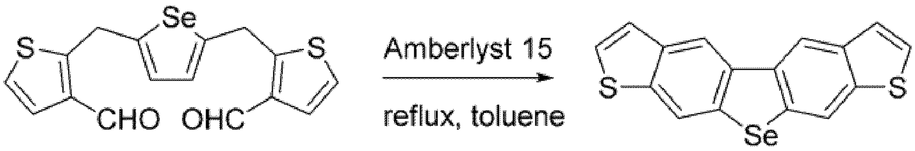


**3a** **4a**

The intermediate 3a (6.50 g) was dissolved in 350 ml of toluene, AMBERLYST (registered trade name) 15 hydrogen form dry (15.0 g) was added thereto, and the solution was heated to reflux for 2 hours. The reaction solution was separated by filtration, and the filtrate was concentrated recrystallized with toluene/methanol, and purified by column chromatography (toluene). As a result, an intermediate 4a (2.35 g, 6.84 mmol, 36% yield for 2 steps) as a white solid target material was obtained.

$^1$H-NMR (400 MHz, $CDCl_3$) δ: 8.63 (s, 2H), 8.31 (d, J=0.8 Hz, 2H), 7.46 (m, 4H)

-Synthesis of Intermediate 5a-

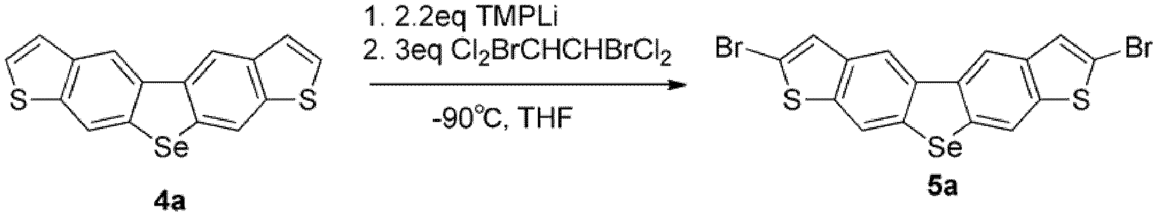


4a

5a

A mixed solution of the intermediate 4a (2.00 g, 5.83 mmol) and 58 ml of tetrahydrofuran was stirred at -90°C, 20 ml of a tetrahydrofuran solution of lithiumtetramethylpiperidine (12.8 mmol) was added dropwise, and the solution was stirred for 30 minutes. A solution in which dibromotetrachloroethane (5.69 g, 17.5 mmol) was dissolved in 20 ml of tetrahydrofuran was added dropwise to the reaction solution, and the solution was stirred at -78°C for 20 minutes and was heated to room temperature. The reaction solution was quenched with water, and the organic layer was extracted with dichloromethane and was dried with magnesium sulfate. After concentration, the solution was recrystallized with tetrahydrofuran/methanol. As a result, an intermediate 5a (2.21 g, 4.41 mmol, 76% yield) as a white solid target material was obtained.

$^1$H-NMR (400 MHz, $CDCl_3$) δ: 8.46 (s, 2H), 8.16 (s, 2H), 7.45 (s, 2H)

(Synthesis of Compound 1)

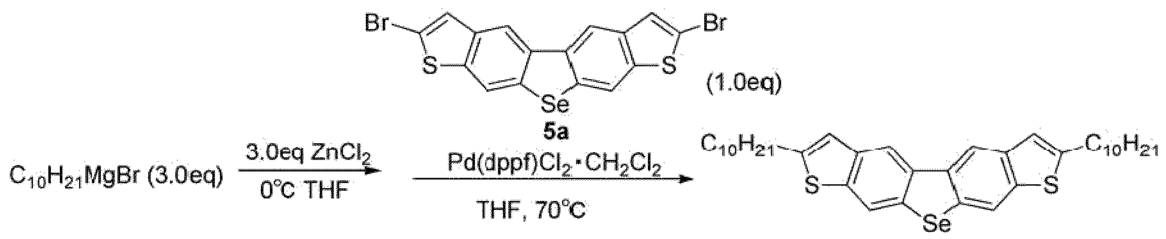


Compound 1

A zinc chloride (II) solution (1.0 mol/L, tetrahydrofuran solution, 1.50 ml) was added at 0°C to an n-decyl magnesium bromide solution (1.0 mol/L, in diethylether, 1.50 ml, 1.50 mmol) used as a reactant. Next, the solution was stirred for 15 minutes, and the intermediate 5a (250 mg, 0.45 mmol) and a 1,1'-bis(diphenylphosphino) ferrocene dichloro palladium (II) dichloromethane adduct (20.2 mg, 0.025 mmol) were added thereto. The reaction solution was stirred at 70°C for 1 hour, was concentrated, and was purified by column chromatography (hexane/chloroform=95/5). As a result, a compound 1 (102 mg, 0.16 mmol, 33% yield) as a white solid target material was obtained.

$^1$H-NMR (400 MHz, $CDCl_3$) δ: 8.43 (s, 2H), 8.17 (s, 2H), 7.10 (s, 2H), 2.93 (t, J=7.6 Hz, 4H), 1.78 (quint, J=6.4 Hz, 4H), 1.46-1.27 (m, 28H), 0.88 (t, J=6.8 Hz, 6H)

<Synthesis Examples 2 to 112: Synthesis of Compounds 2 to 112>

The compounds 2 to 112 shown above were synthesized under the same conditions of the compound 1 according to Examples of JP2015-195362A.

C8-BTBT, TIPS-PEN, DNTT, TES-ADT, and P3HT were commercially available products (manufactured by Sigma-Aldrich Corporation). The number-average molecular weight (Mn) of P3HT was 15000 to 45000.

As a solvent, a commercially available product was used.

[Preparation Examples of Organic Semiconductor Film-Forming Composition]

Organic semiconductors shown in Tables 1-1 to 1-5 (collectively referred to as "Table 1") were weighed and put into a vial such that a ratio between the contents of the low molecular weight organic semiconductor compound and the fused heterocyclic compound was a value shown in Table 1. The solvent was added such that the total concentration of the organic semiconductor was 0.7 mass% as shown in Table 1. Further, polystyrene (molecular weight: 35000) as a binder polymer was added such that the concentration was 0.5 mass%. The components were mixed with each other and stirred for 10 minutes using a Mix rotor (manufactured by AS ONE Corporation) and were filtered through a 0.5 μm membrane filter. This way, organic semiconductor film-forming compositions T1 to T126 according to the present invention and organic semiconductor film-forming composition cTI to cT7 for comparison were prepared.

Regarding each of the organic semiconductor film-forming compositions, the contents of the low molecular weight organic semiconductor compound and the fused heterocyclic compound are as follows.

Contents in organic semiconductor film-forming composition: low molecular weight organic semiconductor compound=0.63 mass%, fused heterocyclic compound=0.07 mass%

Contents with respect to total mass of solid content: low molecular weight organic semiconductor compound=52.5 mass%, fused heterocyclic compound=5.8 mass%, binder polymer=41.7 mass%

Contents with respect to total mass of solid content excluding binder polymer: low molecular weight organic semiconductor compound=90 mass%, fused heterocyclic compound=10 mass%

Contents with respect to total mass of organic semiconductors: low molecular weight organic semiconductor compound=90 mass%, fused heterocyclic compound=10 mass%

In all the prepared organic semiconductor film-forming compositions T1 to T126, the viscosity measured using the measurement method was in a range of 1 to 50 mPa·s.

<Measurement of Weight-Average Molecular Weight>

In the present invention, the weight-average molecular weight of the polymer is measured by gel permeation chromatography (GPC) in terms of standard polystyrene. Specifically, for example, HLC-8121GPC (manufactured by Tosoh Corporation) is used as a GPC, two columns TSKgel $GMH_{HR}$-H (20) HT (manufactured by Tosoh Corporation, 7.8 mm ID×30 cm) are used as columns, and 1,2,4-trichlorobenzene is used as an eluent. In addition, the measurement can be used using an infrared (IR) detector under conditions of sample concentration: 0.02 mass%, flow rate: 1.0 mL/min, sample injection volume: 300 μL, and measurement temperature: 160°C.

In addition, a calibration curve can be obtained from 12 samples of "Standard samples, TSK standard, polystyrene": "F-128", "F-80", "F-40", "F-20", "F-10", "F-4", "F-2", "F-1", "A-5000", "A-2500", "A-1000", and "A-500" (manufactured by Tosoh Corporation).

[Example 1: Manufacturing of Organic Thin Film Transistor]

The bottom gate-bottom contact type organic thin film transistor 100 shown in Fig. 1 was manufactured.

<Formation of Gate Electrode>

Silver Nano Ink (H-1, manufactured by Mitsubishi Materials Corporation) was applied to a wiring pattern having a width of 100 μm and a thickness of 100 nm on a non-alkaline glass plate (5 cm×5 cm, thickness: 0.7 mm) 10 by ink jet printing using DMP2831 (1 picoliter head). Next, the applied ink was fired on a hot plate at 200°C for 90 minutes. As a result, the gate electrode 20 and the wiring were formed.

<Formation of Gate Insulating Layer>

5 parts by mass of polyvinyl phenol (weight-average molecular weight: 25000, manufactured by Sigma-Aldrich Corporation), 5 parts by mass of melamine, and 90 parts by mass of polyethylene glycol monomethyl ether acetate were mixed with each other and stirred, and the mixture was filtered through a 0.2 μm membrane filter. As a result, a gate insulating layer-forming solution was prepared. The obtained gate insulating layer-forming solution was added dropwise to the glass substrate on which the gate electrode was prepared, was applied by spin coating (1000 rpm, 120 seconds), and was heated at 150°C for 30 minutes. The gate insulating layer 30 (thickness: 1 μm) was formed.

<Formation of Source Electrode and Drain Electrode>

Next, a metal mask including a plurality of formation patterns of openings shown in Fig. 3 was placed at the center of the substrate on which the gate insulating layer was formed, and was irradiated with ultraviolet (UV) ozone for 30 minutes. As a result, the surface of the gate insulating layer exposed through the openings was reformed to be hydrophilic. A source-drain electrode pattern having a gate length of 50 μm and a gate width of 320 μm was formed in the vicinity of the surface-reformed portion by ink jet printing using DMP2831 (1 picoliter head). The obtained substrate was fired on a hot plate at 200°C for 90 minutes in a nitrogen gas atmosphere (in a glove box, an environment in which the oxygen concentration was 20 ppm or lower). As a result, copper electrodes (a source electrode 41a and a drain electrode 41b) having a thickness of 200 nm were formed.

<Formation of Organic Semiconductor Layer>

The organic semiconductor film-forming composition prepared in [Preparation Example of Organic Semiconductor Film-Forming Composition] was applied using an ink jet method to the substrate on which the source electrode and the drain electrode were formed. By using DPP2831(manufactured by Fuji Film Global Graphic Systems Co., Ltd.) and a 10 pL head as an ink jet device, a solid film was formed under conditions of jetting frequency: 2 Hz and inter-dot pitch: 20 μm. Next, the applied organic semiconductor film-forming composition was dried at 70°C for 1 hour to form an organic semiconductor layer (thickness: 10 nm).

This way, 100 specimens were manufactured for each of the organic thin film transistors T1 to T126 according to the present invention and the organic thin film transistors cTl to cT7 for comparison.

<Evaluation of Organic Thin Film Transistor>

Regarding each of the manufactured organic TFTs, the following performance evaluation was performed in air using a semiconductor characteristic evaluation device: B2900A (trade name, manufactured by Agilent Technologies Inc.). The results are shown in Table 1.

[Test Example 1: Evaluation of Carrier Mobility μ]

Regarding the organic TFT of each sample number, the carrier mobilities of the manufactured 100 specimens were measured. Specifically, a voltage of -70 V was applied between the source electrode and the drain electrode of each of the organic TFTs, a gate voltage was caused to vary in a range of +60 V to -60 V, and a carrier mobility μ was calculated using the following expression indicating a drain current $I_d$. Regarding each of the organic TFTs, the average value of the carrier mobilities of the 100 specimens was obtained as a carrier mobility $\mu^{AV}$ of the organic TFT of each sample number.

$$I_d=(w/2L)\mu C_i(V_g-V_{th})^2$$

In the expression, L represents the gate length, w represents the gate width, μ represents the carrier mobility, $C_i$ represents the volume of the gate insulating layer per unit area, $V_g$ represents the gate voltage, and $V_{th}$ represents a threshold voltage.

The obtained carrier mobility $\mu^{AV}$ was evaluated based on the following evaluation standards. The higher the carrier mobility $\mu^{AV}$, the better. In this test, "3" or higher is preferable.

5: the carrier mobility $\mu^{AV}$ was 0.4 $cm^2$/Vs or higher
4: the carrier mobility $\mu^{AV}$ was 0.2 $cm^2$/Vs or higher and lower than 0.4 $cm^2$/Vs
3: the carrier mobility $\mu^{AV}$ was 0.1 $cm^2$/Vs or higher and lower than 0.2 $cm^2$/Vs
2: the carrier mobility $\mu^{AV}$ was 0.01 $cm^2$/Vs or higher and lower than 0.1 $cm^2$/Vs
1: the carrier mobility $\mu^{AV}$ was lower than 0.01 $cm^2$/Vs

[Test Example 2: Evaluation of Hysteresis]

Regarding the organic TFT of each sample number, the hysteresis values of the manufactured 100 specimens were measured. Specifically, the performance was evaluated using the same method as in Test Example 1, and an absolute value ($|Vth^F-Vth^A|$) of a difference between a threshold voltage ($Vth^F$) in a case where the gate voltage was

swept from +40 V to -40 V and a threshold voltage ($Vth^R$) in a case where the gate voltage was swept from -40 V to +40 V was defined as hysteresis.

Regarding each of the organic TFTs, the average value of the hysteresis values of the 100 specimens was obtained as the average hysteresis of the organic TFT of each sample number.

The obtained average hysteresis was evaluated based on the evaluation standards. The smaller the average hysteresis, the better. In this test, "3" or higher is preferable.

5: the average hysteresis was lower than 3 V
4: the average hysteresis was 3 V or higher and lower than 6 V
3: the average hysteresis was 6 V or higher and lower than 10 V
2: the average hysteresis was 10 V or higher and lower than 15 V
1: the average hysteresis was 15 V or higher

In Table 1, "Content Ratio" shows the mass ratio low molecular weight organic semiconductor compound:fused heterocyclic compound. In addition, "Content **" shows the total content (unit: mass%) of the organic semiconductors in the composition. In Table 1, the unit of the boiling point is °C, and the unit of the SP value is $MPa^{1/2}$.

In Table 1, in a case where the fused heterocyclic compound or the solvent 2 was not used, "-" is shown in the corresponding column.

[Table 1-1]

| Sample No. | Organic Semiconductor | | | | Solvent 1 | | | | Solvent 2 | | | | Evaluation | |
|---|---|---|---|---|---|---|---|---|---|---|---|---|---|---|
| | Low Molecular Weight Organic Semiconductor Compound | Fused Hete-rocyclic Com-pound | Content Ratio* | Content** | Kind of Solvent | Boiling Point | SP Value | Content** | Kind of Solvent | Boiling Point | SP Value | Content** | Carrier Mobility | Hysteresis |
| T1 | C8-BTBT | Compound 1 | 90:10 | 0.7 | Tetralin | 208 | 19.6 | 98.8 | - | - | - | - | 4 | 4 |
| T2 | C8-BTBT | Compound 2 | 90:10 | 0.7 | Tetralin | 208 | 19.6 | 98.8 | - | - | - | - | 3 | 4 |
| T3 | C8-BTBT | Compound 3 | 90:10 | 0.7 | Tetralin | 208 | 19.6 | 98.8 | - | - | - | - | 4 | 4 |
| T4 | C8-BTBT | Compound 4 | 90:10 | 0.7 | Tetralin | 208 | 19.6 | 98.8 | - | - | - | - | 4 | 4 |
| T5 | C8-BTBT | Compound 5 | 90:10 | 0.7 | Tetralin | 208 | 19.6 | 98.8 | - | - | - | - | 4 | 4 |
| T6 | C8-BTBT | Compound 6 | 90:10 | 0.7 | Tetralin | 208 | 19.6 | 98.8 | - | - | - | - | 4 | 4 |
| T7 | C8-BTBT | Compound 7 | 90:10 | 0.7 | Tetralin | 208 | 19.6 | 98.8 | - | - | - | - | 4 | 4 |
| T8 | C8-BTBT | Compound 8 | 90:10 | 0.7 | Tetralin | 208 | 19.6 | 98.8 | - | - | - | - | 4 | 4 |
| T9 | C8-BTBT | Compound 9 | 90:10 | 0.7 | Tetralin | 208 | 19.6 | 98.8 | - | - | - | - | 3 | 4 |
| T10 | C8-BTBT | Compound 10 | 90:10 | 0.7 | Tetralin | 208 | 19.6 | 98.8 | - | - | - | - | 4 | 4 |
| T11 | C8-BTBT | Compound 11 | 90:10 | 0.7 | Tetralin | 208 | 19.6 | 98.8 | - | - | - | - | 4 | 4 |
| T12 | C8-BTBT | Compound 12 | 90:10 | 0.7 | Tetralin | 208 | 19.6 | 98.8 | - | - | - | - | 4 | 4 |
| T13 | C8-BTBT | Compound 13 | 90:10 | 0.7 | Tetralin | 208 | 19.6 | 98.8 | - | - | - | - | 4 | 4 |
| T14 | C8-BTBT | Compound 14 | 90:10 | 0.7 | Tetralin | 208 | 19.6 | 98.8 | - | - | - | - | 4 | 4 |
| T15 | C8-BTBT | Compound 15 | 90:10 | 0.7 | Tetralin | 208 | 19.6 | 98.8 | - | - | - | - | 4 | 4 |
| T16 | C8-BTBT | Compound 16 | 90:10 | 0.7 | Tetralin | 208 | 19.6 | 98.8 | - | - | - | - | 4 | 4 |
| T17 | C8-BTBT | Compound 17 | 90:10 | 0.7 | Tetralin | 208 | 19.6 | 98.8 | - | - | - | - | 4 | 4 |
| T18 | C8-BTBT | Compound 18 | 90:10 | 0.7 | Tetralin | 208 | 19.6 | 98.8 | - | - | - | - | 4 | 4 |
| T19 | C8-BTBT | Compound 19 | 90:10 | 0.7 | Tetralin | 208 | 19.6 | 98.8 | - | - | - | - | 3 | 4 |
| T20 | C8-BTBT | Compound 20 | 90:10 | 0.7 | Tetralin | 208 | 19.6 | 98.8 | - | - | - | - | 3 | 4 |
| T21 | C8-BTBT | Compound 21 | 90:10 | 0.7 | Tetralin | 208 | 19.6 | 98.8 | - | - | - | - | 3 | 4 |
| T22 | C8-BTBT | Compound 22 | 90:10 | 0.7 | Tetralin | 208 | 19.6 | 98.8 | - | - | - | - | 3 | 4 |

(continued)

| Sample No. | Organic Semiconductor | | | | Solvent 1 | | | | Solvent 2 | | | | Evaluation | |
|---|---|---|---|---|---|---|---|---|---|---|---|---|---|---|
| | Low Molecular Weight Organic Semiconductor Compound | Fused Heterocyclic Compound | Content Ratio* | Content** | Kind of Solvent | Boiling Point | SP Value | Content** | Kind of Solvent | Boiling Point | SP Value | Content** | Carrier Mobility | Hysteresis |
| T23 | C8-BTBT | Compound 23 | 90:10 | 0.7 | Tetralin | 208 | 19.6 | 98.8 | - | - | - | - | 3 | 4 |
| T24 | C8-BTBT | Compound 24 | 90:10 | 0.7 | Tetralin | 208 | 19.6 | 98.8 | - | - | - | - | 3 | 4 |
| T25 | C8-BTBT | Compound 25 | 90:10 | 0.7 | Tetralin | 208 | 19.6 | 98.8 | - | - | - | - | 3 | 4 |
| T26 | C8-BTBT | Compound 26 | 90:10 | 0.7 | Tetralin | 208 | 19.6 | 98.8 | - | - | - | - | 3 | 4 |
| T27 | C8-BTBT | Compound 27 | 90:10 | 07 | Tetralin | 208 | 19.6 | 98.8 | - | - | - | - | 4 | 4 |
| T28 | C8-BTBT | Compound 28 | 90:10 | 0.7 | Tetralin | 208 | 19.6 | 98.8 | - | - | - | - | 3 | 4 |
| T29 | C8-BTBT | Compound 29 | 90:10 | 0.7 | Tetralin | 208 | 19.6 | 98.8 | - | - | - | - | 4 | 4 |
| T30 | C8-BTBT | Compound 30 | 90:10 | 0.7 | Tetralin | 208 | 19.6 | 98.8 | - | - | - | - | 4 | 4 |

[Table 1-2]

| Sample No. | Organic Semiconductor | | | | Solvent 1 | | | | Solvent 2 | | | | Evaluation | |
|---|---|---|---|---|---|---|---|---|---|---|---|---|---|---|
| | Low Molecular Weight Organic Semiconductor Compound | Fused Heterocyclic Compound | Content Ratio* | Content** | Kind of Solvent | Boiling Point | SP Value | Content** | Kind of Solvent | Boiling Point | SP Value | Content** | Carrier Mobility | Hysteresis |
| T31 | C8-BTBT | Compound 31 | 90:10 | 0.7 | Tetralin | 208 | 19.6 | 98.8 | - | - | - | - | 3 | 4 |
| T32 | C8-BTBT | Compound 32 | 90:10 | 0.7 | Tetralin | 208 | 19.6 | 98.8 | - | - | - | - | 3 | 4 |
| T33 | C8-BTBT | Compound 33 | 90:10 | 0.7 | Tetralin | 208 | 19.6 | 98.8 | - | - | - | - | 3 | 4 |
| T34 | C8-BTBT | Compound 34 | 90:10 | 0.7 | Tetralin | 208 | 19.6 | 98.8 | - | - | - | - | 3 | 4 |
| T35 | C8-BTBT | Compound 35 | 90:10 | 0.7 | Tetralin | 208 | 19.6 | 98.8 | - | - | - | - | 3 | 4 |
| T36 | C8-BTBT | Compound 36 | 90:10 | 0.7 | Tetralin | 208 | 19.6 | 98.8 | - | - | - | - | 3 | 4 |
| T37 | C8-BTBT | Compound 37 | 90:10 | 0.7 | Tetralin | 208 | 19.6 | 98.8 | - | - | - | - | 3 | 4 |
| T38 | C8-BTBT | Compound 38 | 90:10 | 0.7 | Tetralin | 208 | 19.6 | 98.8 | - | - | - | - | 3 | 4 |
| T39 | C8-BTBT | Compound 39 | 90:10 | 0.7 | Tetralin | 208 | 19.6 | 98.8 | - | - | - | - | 3 | 4 |
| T40 | C8-BTBT | Compound 40 | 90:10 | 0.7 | Tetralin | 208 | 19.6 | 98.8 | - | - | - | - | 4 | 4 |
| T41 | C8-BTBT | Compound 41 | 90:10 | 0.7 | Tetralin | 208 | 19.6 | 98.8 | - | - | - | - | 3 | 4 |
| T42 | C8-BTBT | Compound 42 | 90:10 | 0.7 | Tetralin | 208 | 19.6 | 98.8 | - | - | - | - | 3 | 4 |
| T43 | C8-BTBT | Compound 43 | 90:10 | 0.7 | Tetralin | 208 | 19.6 | 98.8 | - | - | - | - | 3 | 4 |
| T44 | C8-BTBT | Compound 44 | 90:10 | 0.7 | Tetralin | 208 | 19.6 | 98.8 | - | - | - | - | 3 | 4 |
| T45 | C8-BTBT | Compound 45 | 90:10 | 0.7 | Tetralin | 208 | 19.6 | 98.8 | - | - | - | - | 4 | 4 |
| T46 | C8-BTBT | Compound 46 | 90:10 | 0.7 | Tetralin | 208 | 19.6 | 98.8 | - | - | - | - | 4 | 4 |
| T47 | C8-BTBT | Compound 47 | 90:10 | 0.7 | Tetralin | 208 | 19.6 | 98.8 | - | - | - | - | 3 | 4 |
| T48 | C8-BTBT | Compound 48 | 90:10 | 0.7 | Tetralin | 208 | 19.6 | 98.8 | - | - | - | - | 3 | 4 |
| T49 | C8-BTBT | Compound 49 | 90:10 | 0.7 | Tetralin | 208 | 19.6 | 98.8 | - | - | - | - | 3 | 4 |
| T50 | C8-BTBT | Compound 50 | 90:10 | 0.7 | Tetralin | 208 | 19.6 | 98.8 | - | - | - | - | 3 | 4 |
| T51 | C8-BTBT | Compound 51 | 90:10 | 0.7 | Tetralin | 208 | 19.6 | 98.8 | - | - | - | - | 3 | 4 |
| T52 | C8-BTBT | Compound 52 | 90:10 | 0.7 | Tetralin | 208 | 19.6 | 98.8 | - | - | - | - | 3 | 4 |

(continued)

| Sample No. | Organic Semiconductor | | | | Solvent 1 | | | | Solvent 2 | | | | Evaluation | |
|---|---|---|---|---|---|---|---|---|---|---|---|---|---|---|
| | Low Molecular Weight Organic Semiconductor Compound | Fused Hete-rocyclic Com-pound | Content Ratio* | Content** | Kind of Solvent | Boiling Point | SP Value | Content** | Kind of Solvent | Boiling Point | SP Value | Content** | Carrier Mobility | Hysteresis |
| T53 | C8-BTBT | Compound 53 | 90:10 | 0.7 | Tetralin | 208 | 19.6 | 98.8 | - | - | - | - | 3 | 4 |
| T54 | C8-BTBT | Compound 54 | 90:10 | 0.7 | Tetralin | 208 | 19.6 | 98.8 | - | - | - | - | 3 | 4 |
| T55 | C8-BTBT | Compound 55 | 90:10 | 0.7 | Tetralin | 208 | 19.6 | 98.8 | - | - | - | - | 3 | 4 |
| T56 | C8-BTBT | Compound 56 | 90:10 | 0.7 | Tetralin | 208 | 19.6 | 98.8 | - | - | - | - | 3 | 4 |
| T57 | C8-BTBT | Compound 57 | 90:10 | 0.7 | Tetralin | 208 | 19.6 | 98.8 | - | - | - | - | 3 | 4 |
| T58 | C8-BTBT | Compound 58 | 90:10 | 0.7 | Tetralin | 208 | 19.6 | 98.8 | - | - | - | - | 3 | 4 |
| T59 | C8-BTBT | Compound 59 | 90:10 | 0.7 | Tetralin | 208 | 19.6 | 98.8 | - | - | - | - | 3 | 4 |
| T60 | C8-BTBT | Compound 60 | 90:10 | 0.7 | Tetralin | 208 | 19.6 | 98.8 | - | - | - | - | 3 | 4 |

[Table 1-3]

| Sample No. | Organic Semiconductor | | | | Solvent 1 | | | | Solvent 2 | | | | Evaluation | |
|---|---|---|---|---|---|---|---|---|---|---|---|---|---|---|
| | Low Molecular Weight Organic Semiconductor Compound | Fused Hete-rocyclic Com-pound | Content Ratio* | Content** | Kind of Solvent | Boiling Point | SP Value | Content** | Kind of Solvent | Boiling Point | SP Value | Content** | Carrier Mobility | Hysteresis |
| T61 | C8-BTBT | Compound 61 | 90:10 | 0.7 | Tetralin | 208 | 19.6 | 98.8 | - | - | - | - | 3 | 4 |
| T62 | C8-BTBT | Compound 62 | 90:10 | 0.7 | Tetralin | 208 | 19.6 | 98.8 | - | - | - | - | 3 | 4 |
| T63 | C8-BTBT | Compound 63 | 90:10 | 0.7 | Tetralin | 208 | 19.6 | 98.8 | - | - | - | - | 4 | 4 |
| T64 | C8-BTBT | Compound 64 | 90:10 | 0.7 | Tetralin | 208 | 19.6 | 98.8 | - | - | - | - | 4 | 4 |
| T65 | C8-BTBT | Compound 65 | 90:10 | 0.7 | Tetralin | 208 | 19.6 | 98.8 | - | - | - | - | 4 | 4 |
| T66 | C8-BTBT | Compound 66 | 90:10 | 0.7 | Tetralin | 208 | 19.6 | 98.8 | - | - | - | - | 3 | 4 |
| T67 | C8-BTBT | Compound 67 | 90:10 | 0.7 | Tetralin | 208 | 19.6 | 98.8 | - | - | - | - | 3 | 4 |
| T68 | C8-BTBT | Compound 68 | 90:10 | 0.7 | Tetralin | 208 | 19.6 | 98.8 | - | - | - | - | 3 | 4 |
| T69 | C8-BTBT | Compound 69 | 90:10 | 0.7 | Tetralin | 208 | 19.6 | 98.8 | - | - | - | - | 4 | 4 |
| T70 | C8-BTBT | Compound 70 | 90:10 | 0.7 | Tetralin | 208 | 19.6 | 98.8 | - | - | - | - | 3 | 4 |
| T71 | C8-BTBT | Compound 71 | 90:10 | 0.7 | Tetralin | 208 | 19.6 | 98.8 | - | - | - | - | 3 | 4 |
| T72 | C8-BTBT | Compound 72 | 90:10 | 0.7 | Tetralin | 208 | 19.6 | 98.8 | - | - | - | - | 4 | 4 |
| T73 | C8-BTBT | Compound 73 | 90:10 | 0.7 | Tetralin | 208 | 19.6 | 98.8 | - | - | - | - | 3 | 4 |
| T74 | C8-BTBT | Compound 74 | 90:10 | 0.7 | Tetralin | 208 | 19.6 | 98.8 | - | - | - | - | 3 | 4 |
| T75 | C8-BTBT | Compound 75 | 90:10 | 0.7 | Tetralin | 208 | 19.6 | 98.8 | - | - | - | - | 3 | 4 |
| T76 | C8-BTBT | Compound 76 | 90:10 | 0.7 | Tetralin | 208 | 19.6 | 98.8 | - | - | - | - | 3 | 4 |
| T77 | C8-BTBT | Compound 77 | 90:10 | 0.7 | Tetralin | 208 | 19.6 | 98.8 | - | - | - | - | 3 | 4 |
| T78 | C8-BTBT | Compound 78 | 90:10 | 0.7 | Tetralin | 208 | 19.6 | 98.8 | - | - | - | - | 3 | 4 |
| T79 | C8-BTBT | Compound 79 | 90:10 | 0.7 | Tetralin | 208 | 19.6 | 98.8 | - | - | - | - | 3 | 4 |
| T80 | C8-BTBT | Compound 80 | 90:10 | 0.7 | Tetralin | 208 | 19.6 | 98.8 | - | - | - | - | 3 | 4 |
| T81 | C8-BTBT | Compound 81 | 90:10 | 0.7 | Tetralin | 208 | 19.6 | 98.8 | - | - | - | - | 3 | 4 |
| T82 | C8-BTBT | Compound 82 | 90:10 | 0.7 | Tetralin | 208 | 19.6 | 98.8 | - | - | - | - | 3 | 4 |

(continued)

| Sample No. | Organic Semiconductor | | | | Solvent 1 | | | | Solvent 2 | | | | Evaluation | |
|---|---|---|---|---|---|---|---|---|---|---|---|---|---|---|
| | Low Molecular Weight Organic Semiconductor Compound | Fused Hete-rocyclic Com-pound | Content Ratio* | Content** | Kind of Solvent | Boiling Point | SP Value | Content** | Kind of Solvent | Boiling Point | SP Value | Content** | Carrier Mobility | Hysteresis |
| T83 | C8-BTBT | Compound 83 | 90:10 | 0.7 | Tetralin | 208 | 19.6 | 98.8 | - | - | - | - | 3 | 4 |
| T84 | C8-BTBT | Compound 84 | 90:10 | 0.7 | Tetralin | 208 | 19.6 | 98.8 | - | - | - | - | 3 | 4 |
| T85 | C8-BTBT | Compound 85 | 90:10 | 0.7 | Tetralin | 208 | 19.6 | 98.8 | - | - | - | - | 3 | 4 |
| T86 | C8-BTBT | Compound 86 | 90:10 | 0.7 | Tetralin | 208 | 19.6 | 98.8 | - | - | - | - | 3 | 4 |
| T87 | C8-BTBT | Compound 87 | 90:10 | 0.7 | Tetralin | 208 | 19.6 | 98.8 | - | - | - | - | 3 | 4 |
| T88 | C8-BTBT | Compound 88 | 90:10 | 0.7 | Tetralin | 208 | 19.6 | 98.8 | - | - | - | - | 3 | 4 |
| T89 | C8-BTBT | Compound 89 | 90:10 | 0.7 | Tetralin | 208 | 19.6 | 98.8 | - | - | - | - | 3 | 4 |
| T90 | C8-BTBT | Compound 90 | 90:10 | 0.7 | Tetralin | 208 | 19.6 | 98.8 | - | - | - | - | 3 | 4 |

[Table 1-4]

| Sample No. | Organic Semiconductor | | | | Solvent 1 | | | | Solvent 2 | | | | Evaluation | |
|---|---|---|---|---|---|---|---|---|---|---|---|---|---|---|
| | Low Molecular Weight Organic Semiconductor Compound | Fused Heterocyclic Compound | Content Ratio* | Content** | Kind of Solvent | Boiling Point | SP Value | Content** | Kind of Solvent | Boiling Point | SP Value | Content** | Carrier Mobility | Hysteresis |
| T91 | C8-BTBT | Compound 91 | 90:10 | 0.7 | Tetralin | 208 | 19.6 | 98.8 | - | - | - | - | 3 | 4 |
| T92 | C8-BTBT | Compound 92 | 90:10 | 0.7 | Tetralin | 208 | 19.6 | 98.8 | - | - | - | - | 3 | 4 |
| T93 | C8-BTBT | Compound 93 | 90:10 | 0.7 | Tetralin | 208 | 19.6 | 98.8 | - | - | - | - | 3 | 4 |
| T94 | C8-BTBT | Compound 94 | 90:10 | 0.7 | Tetralin | 208 | 19.6 | 98.8 | - | - | - | - | 3 | 4 |
| T95 | C8-BTBT | Compound 95 | 90:10 | 0.7 | Tetralin | 208 | 19.6 | 98.8 | - | - | - | - | 3 | 4 |
| T96 | C8-BTBT | Compound 96 | 90:10 | 0.7 | Tetralin | 208 | 19.6 | 98.8 | - | - | - | - | 3 | 4 |
| T97 | C8-BTBT | Compound 97 | 90:10 | 0.7 | Tetralin | 208 | 19.6 | 98.8 | - | - | - | - | 3 | 4 |
| T98 | C8-BTBT | Compound 98 | 90:10 | 0.7 | Tetralin | 208 | 19.6 | 98.8 | - | - | - | - | 3 | 4 |
| T99 | C8-BTBT | Compound 99 | 90:10 | 0.7 | Tetralin | 208 | 19.6 | 98.8 | - | - | - | - | 3 | 4 |
| T100 | C8-BTBT | Compound 100 | 90:10 | 0.7 | Tetralin | 208 | 19.6 | 98.8 | - | - | - | - | 3 | 4 |
| T101 | C8-BTBT | Compound 101 | 90:10 | 0.7 | Tetralin | 208 | 19.6 | 98.8 | - | - | - | - | 3 | 4 |
| T102 | C8-BTBT | Compound 102 | 90:10 | 0.7 | Tetralin | 208 | 19.6 | 98.8 | - | - | - | - | 3 | 4 |

(continued)

| Sample No. | Organic Semiconductor | | | | Solvent 1 | | | | Solvent 2 | | | | Evaluation | |
|---|---|---|---|---|---|---|---|---|---|---|---|---|---|---|
| | Low Molecular Weight Organic Semiconductor Compound | Fused Heterocyclic Compound | Content Ratio* | Content** | Kind of Solvent | Boiling Point | SP Value | Content** | Kind of Solvent | Boiling Point | SP Value | Content** | Carrier Mobility | Hysteresis |
| T103 | C8-BTBT | Compound 103 | 90:10 | 0.7 | Tetralin | 208 | 19.6 | 98.8 | - | - | - | - | 3 | 4 |
| T104 | C8-BTBT | Compound 104 | 90:10 | 0.7 | Tetralin | 208 | 19.6 | 98.8 | - | - | - | - | 4 | 4 |
| T105 | C8-BTBT | Compound 105 | 90:10 | 0.7 | Tetralin | 208 | 19.6 | 98.8 | - | - | - | - | 4 | 4 |
| T106 | C8-BTBT | Compound 106 | 90:10 | 0.7 | Tetralin | 208 | 19.6 | 98.8 | - | - | - | - | 4 | 4 |
| T107 | C8-BTBT | Compound 15 | 60:40 | 0.7 | Tetralin | 208 | 19.6 | 98.8 | - | - | - | - | 3 | 4 |
| T108 | C8-BTBT | Compound 15 | 85:15 | 0.7 | Tetralin | 208 | 19.6 | 98.8 | - | - | - | - | 4 | 4 |
| T109 | TIPS-PEN | Compound 15 | 90:10 | 0.7 | Tetralin | 208 | 19.6 | 98.8 | - | - | - | - | 3 | 4 |
| T110 | DNTT | Compound 15 | 90:10 | 0.7 | Tetralin | 208 | 19.6 | 98.8 | - | - | - | - | 4 | 4 |
| T111 | TES-ADT | Compound 15 | 90:10 | 0.7 | Tetralin | 208 | 19.6 | 98.8 | - | - | - | - | 3 | 4 |
| T112 | Compound 14 | Compound 15 | 90:10 | 0.7 | Tetralin | 208 | 19.6 | 98.8 | - | - | - | - | 5 | 4 |
| T113 | C8-BTBT | Compound 15 | 90:10 | 0.7 | 1-Chloronaphthalene | 259 | 20.8 | 98.8 | - | - | - | - | 4 | 5 |
| T114 | C8-BTBT | Compound 15 | 90:10 | 0.7 | 1 -Fluoronaphthalene | 215 | 20.3 | 98.8 | - | - | - | - | 4 | 5 |

(continued)

| Sample No. | Organic Semiconductor | | | | Solvent 1 | | | | Solvent 2 | | | | Evaluation | |
|---|---|---|---|---|---|---|---|---|---|---|---|---|---|---|
| | Low Molecular Weight Organic Semiconductor Compound | Fused Heterocyclic Compound | Content Ratio* | Content** | Kind of Solvent | Boiling Point | SP Value | Content** | Kind of Solvent | Boiling Point | SP Value | Content** | Carrier Mobility | Hysteresis |
| T115 | C8-BTBT | Compound 15 | 90:10 | 0.7 | 1-Methylnaphthalene | 204 | 20.0 | 98.8 | - | - | - | - | 4 | 5 |
| T116 | C8-BTBT | Compound 15 | 90:10 | 0.7 | 1,2-Dichlorobenzene | 181 | 20.1 | 98.8 | - | - | - | - | 3 | 4 |
| T117 | C8-BTBT | Compound 15 | 90:10 | 0.7 | 1-Chloronaphthalene | 259 | 20.8 | 59.8 | 1,2-Dichlorobenzene | 181 | 20.1 | 39.0 | 4 | 4 |
| T118 | C8-BTBT | Compound 15 | 90:10 | 0.7 | 1-Chloronaphthalene | 259 | 20.8 | 38.8 | 1,2-Dichlorobenzene | 181 | 20.1 | 60.0 | 3 | 4 |
| T119 | C8-BTBT | Compound 15 | 90:10 | 0.7 | Amylbenzene | 205 | 17.5 | 98.8 | - | - | - | - | 3 | 4 |
| T120 | Compound 14 | Compound 15 | 90:10 | 0.7 | 1-Chloronaphthalene | 208 | 19.6 | 98.8 | - | - | - | - | 5 | 5 |

[Table 1-5]

| Sample No. | Organic Semiconductor | | | | Solvent 1 | | | | Solvent 2 | | | | Evaluation | |
|---|---|---|---|---|---|---|---|---|---|---|---|---|---|---|
| | Low Molecular Weight Organic Semiconductor Compound | Fused Heterocyclic Compound | Content Ratio* | Content** | Kind of Solvent | Boiling Point | SP Value | Content** | Kind of Solvent | Boiling Point | SP Value | Content** | Carrier Mobility | Hysteresis |
| T121 | C8-BTBT | Compound 107 | 90:10 | 0.7 | Tetralin | 208 | 19.6 | 98.8 | - | - | - | - | 3 | 4 |
| T122 | C8-BTBT | Compound 108 | 90:10 | 0.7 | Tetralin | 208 | 19.6 | 98.8 | - | - | - | - | 3 | 4 |
| T123 | C8-BTBT | Compound 109 | 90:10 | 0.7 | Tetralin | 208 | 19.6 | 98.8 | - | - | - | - | 3 | 4 |
| T124 | C8-BTBT | Compound 110 | 90:10 | 0.7 | Tetralin | 208 | 19.6 | 98.8 | - | - | - | - | 3 | 4 |
| T125 | C8-BTBT | Compound 111 | 90:10 | 0.7 | Tetralin | 208 | 19.6 | 98.8 | - | - | - | - | 3 | 4 |
| T126 | C8-BTBT | Compound 112 | 90:10 | 0.7 | Tetralin | 208 | 19.6 | 98.8 | - | - | - | - | 3 | 4 |
| cT1 | C8-BTBT | - | - | 0.7 | Tetralin | 208 | 19.6 | 98.8 | - | - | - | - | 2 | 3 |
| cT2 | TIPS-PEN | - | - | 0.7 | Tetralin | 208 | 19.6 | 98.8 | - | - | - | - | 2 | 3 |
| cT3 | DNTT | - | - | 0.7 | Tetralin | 208 | 19.6 | 98.8 | - | - | - | - | 2 | 3 |
| cT4 | Compound 16 | - | - | 0.7 | Tetralin | 208 | 19.6 | 98.8 | - | - | - | - | 2 | 3 |
| cT5 | C8-BTBT | TIPS-PEN | 90:10 | 0.7 | Tetralin | 208 | 19.6 | 98.8 | - | - | - | - | 1 | 1 |
| cT6 | TIPS-PEN | C8-BTBT | 90:10 | 0.7 | Tetralin | 208 | 19.6 | 98.8 | - | - | - | - | 1 | 1 |
| cT7 | TIPS-PEN | P3HT | 90:10 | 0.7 | Tetralin | 208 | 19.6 | 98.8 | - | - | - | - | 1 | 1 |

The following can be seen from the results of Table 1.

In each of the organic thin film transistors cT1 to cT7, the organic semiconductor layer including one organic semiconductor as the low molecular weight organic semiconductor compound was formed or the fused heterocyclic compound or the organic semiconductor layer including two organic semiconductors other than the fused heterocyclic compound was formed, in which a sufficient carrier mobility and a small hysteresis were not able to be obtained at the same time. The organic thin film transistor cT7 was prepared assuming the organic semiconductor element described in JP4545373B.

In particular, in the organic thin film transistors cT-5 to cT-7 in which the organic semiconductor layer including two organic semiconductors other than the fused heterocyclic compound was formed, the carrier mobility was significantly lower and an increase in hysteresis was also larger as compared to the organic thin film transistors cT1 to cT-4 in which the organic semiconductor layer including one organic semiconductor was formed.

On the other hand, in each of the organic thin film transistors T1 to T126 according to the present invention, the organic semiconductor layer including two organic semiconductors as the low molecular weight organic semiconductor compound and the fused heterocyclic compound represented by Formula (1) was formed using an ink jet printing method. In the organic thin film transistors, a sufficient carrier mobility was exhibited, and the occurrence of hysteresis was able to be effectively reduced.

In particular, it was found that, in the organic thin film transistor in which the organic semiconductor layer including two organic semiconductors selected from the fused heterocyclic compounds represented by Formula (1) and having different substituents was formed, the effect of improving the carrier mobility was further enhanced while maintaining the effect of reducing hysteresis (sample numbers T112 and 120).

In addition, it was found that the composition according to the embodiment of the present invention including a solvent having a naphthalene skeleton and having a boiling point of 180°C or higher and a solubility parameter of 17.0 to 23.0 is suitable for manufacturing the organic thin film transistor in which the occurrence of hysteresis was effectively reduced while maintaining a high carrier mobility (sample numbers T113 to 115, 117, and 120).

The present invention has been described using the embodiments. However, unless specified otherwise, any of the details of the above description is not intended to limit the present invention and can be construed in a broad sense within a range not departing from the concept and scope of the present invention disclosed in the accompanying claims.

The present application claims priority based on JP2016-191915 filed on September 29, 2016, the entire content of which is incorporated herein by reference.

Explanation of References

| | |
|---|---|
| 10: | substrate |
| 20: | gate electrode |
| 30: | gate insulating layer |
| 40: | source electrode |
| 42: | drain electrode |
| 50: | organic semiconductor layer |
| 60: | sealing layer |
| 100, 200: | organic thin film transistor |
| 151: | metal mask |
| 153, 154: | opening |

## Claims

**1.** An organic semiconductor film-forming composition comprising:

at least two organic semiconductors,
wherein one organic semiconductor is a low molecular weight organic semiconductor compound having a molecular weight of 5000 or lower, and
another organic semiconductor is a fused heterocyclic compound represented by the following Formula (1) that has a chemical structure different from that of the low molecular weight organic semiconductor compound and has a molecular weight of 3000 or lower,

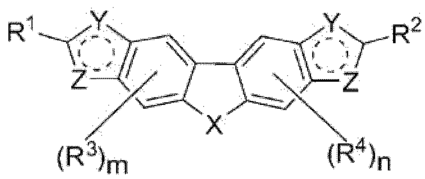

Formula (1)

in Formula (1),

X represents an oxygen atom, a sulfur atom, a selenium atom, a tellurium atom, or $NR^5$,
Y and Z each independently represent $CR^6$, an oxygen atom, a sulfur atom, a selenium atom, a nitrogen atom, or $NR^7$,
a 5-membered ring including Y and Z is an aromatic heterocycle,
$R^1$ and $R^2$ in Formula (1) are bonded to a ring-constituting atom of the 5-membered ring including Y and Z directly or indirectly through a divalent group A,
$R^3$ and $R^4$ in Formula (1) are bonded to a ring-constituting atom of a benzene ring directly or indirectly through the divalent group A,
the divalent group A is a group selected from -O-, -S-, $-NR^8-$, -CO-, -SO-, or $-SO_2-$ or is a group in which two or more selected from -O-, -S-, $-NR^8-$, -CO-, -SO-, or $-SO_2-$ are linked to each other,
$R^1$, $R^2$, $R^5$, $R^6$, $R^7$, and $R^8$ each independently represent a hydrogen atom, an alkyl group, an alkenyl group, an alkynyl group, an aryl group, or a heteroaryl group,
$R^3$ and $R^4$ each independently represent a halogen atom, an alkyl group, an alkenyl group, an alkynyl group, an aryl group, or a heteroaryl group, and
m and n each independently represent an integer of 0 to 2.

2. The organic semiconductor film-forming composition according to claim 1, wherein the 5-membered ring including Y and Z is a ring selected from a thiophene ring, a furan ring, a selenophene ring, a pyrrole ring, a thiazole ring, a selenazole ring, an imidazole ring, or an oxazole ring.

3. The organic semiconductor film-forming composition according to claim 1 or 2, wherein the number of carbon atoms in each of $R^1$, $R^2$, $R^3$, and $R^4$ is 30 or less.

4. The organic semiconductor film-forming composition according to any one of claims 1 to 3, wherein both m and n represent 0.

5. The organic semiconductor film-forming composition according to any one of claims 1 to 4, wherein $R^1$ and $R^2$ each independently represent an alkyl group having 20 or less carbon atoms, an aryl group having 20 or less carbon atoms, or a heteroaryl group having 20 or less carbon atoms.

6. The organic semiconductor film-forming composition according to any one of claims 1 to 5,

wherein $R^1$ and $R^2$ are the same as each other,
$R^3$ and $R^4$ are the same as each other, and
m and n are the same as each other.

7. The organic semiconductor film-forming composition according to claim 1, wherein the compound represented by Formula (1) is a compound represented by the following Formula (2) or (3),

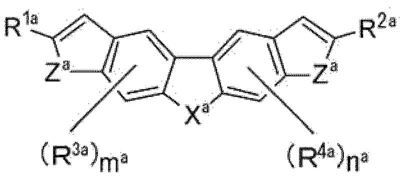

Formula (2)

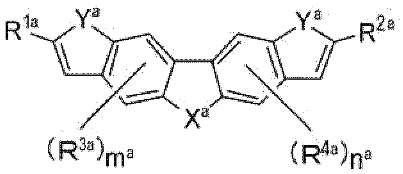

Formula (3)

in Formulae (2) and (3),

$X^a$ represents an oxygen atom, a sulfur atom, or a selenium atom,
$Y^a$ and $Z^a$ each independently represent an oxygen atom, a sulfur atom, a selenium atom, or $NR^{7a}$,
$R^{7a}$ has the same definition as $R^7$ in Formula (1),
$R^{1a}$, $R^{2a}$, $R^{3a}$, $R^{4a}$, $m^a$, and $n^a$ have the same definitions as $R^1$, $R^2$, $R^3$, $R^4$, m, and n in Formula (1), respectively, and
binding forms of $R^{1a}$, $R^{2a}$, $R^{3a}$, and $R^{4a}$ to a ring-constituting atom are also the same as binding forms of $R^1$, $R^2$, $R^3$, and $R^4$ in Formula (1) to a ring-constituting atom, respectively.

**8.** The organic semiconductor film-forming composition according to claim 7,

wherein the compound represented by Formula (2) is represented by the following Formula (4), and the compound represented by Formula (3) is represented by the following Formula (5),

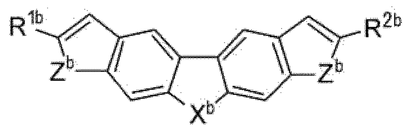

Formula (4)

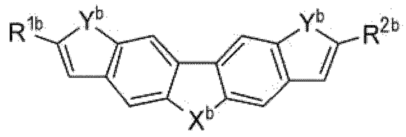

Formula (5)

in Formulae (4) and (5),

$X^b$, $Y^b$, and $Z^b$ each independently represent an oxygen atom, a sulfur atom, or a selenium atom,
$R^{1b}$ and $R^{2b}$ have the same definitions as $R^{1a}$ and $R^{2a}$ in Formula (2), respectively, and
binding forms of $R^{1b}$ and $R^{2b}$ to a ring-constituting atom are also the same as binding forms of $R^{1a}$ and $R^{2a}$ in Formula (2) to a ring-constituting atom, respectively.

**9.** The organic semiconductor film-forming composition according to claim 8, wherein $R^{1b}$ and $R^{2b}$ have an aliphatic hydrocarbon group.

**10.** The organic semiconductor film-forming composition according to claim 8 or 9, wherein $R^{1b}$ and $R^{2b}$ each independently represent an aryl group having a linear aliphatic hydrocarbon group or a heteroaryl group having a linear aliphatic hydrocarbon group.

**11.** The organic semiconductor film-forming composition according to any one of claims 1 to 10, wherein the low molecular weight organic semiconductor compound is a compound having the same mother nucleus as the fused heterocyclic compound and having a substituent different from that of the fused heterocyclic compound.

**12.** The organic semiconductor film-forming composition according to claim 11, wherein a total molecular weight of the substituent included in the low molecular weight organic semiconductor compound is lower than a total molecular weight of the substituent included in the fused heterocyclic compound.

**13.** The organic semiconductor film-forming composition according to any one of claims 1 to 12, wherein a content of the fused heterocyclic compound is 20 mass% or lower with respect to a total mass of the organic semiconductors in the organic semiconductor film-forming composition.

**14.** The organic semiconductor film-forming composition according to any one of claims 1 to 13, further comprising: at least one solvent having a boiling point of 180°C or higher and a solubility parameter of 17.0 to 23.0 $MPa^{1/2}$.

**15.** The organic semiconductor film-forming composition according to claim 14, wherein the solvent has a naphthalene skeleton.

**16.** The organic semiconductor film-forming composition according to claim 15,

wherein a content of the solvent having a naphthalene skeleton is 50 mass% or higher.

**17.** A method of forming an organic semiconductor film comprising:
a step of applying the organic semiconductor film-forming composition according to any one of claims 1 to 16 to a substrate.

**18.** An organic semiconductor film comprising:

at least two organic semiconductors,
wherein one organic semiconductor is a low molecular weight organic semiconductor compound having a molecular weight of 5000 or lower, and
another organic semiconductor is a fused heterocyclic compound represented by the following Formula (1) that has a chemical structure different from that of the low molecular weight organic semiconductor compound and has a molecular weight of 3000 or lower.

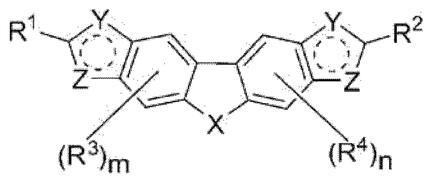
Formula (1)

in Formula (1),

X represents an oxygen atom, a sulfur atom, a selenium atom, a tellurium atom, or $NR^5$,
Y and Z each independently represent $CR^6$, an oxygen atom, a sulfur atom, a selenium atom, a nitrogen atom, or $NR^7$,
a 5-membered ring including Y and Z is an aromatic heterocycle,
$R^1$ and $R^2$ in Formula (1) are bonded to a ring-constituting atom of the 5-membered ring including Y and Z directly or indirectly through a divalent group A,
$R^3$ and $R^4$ in Formula (1) are bonded to a ring-constituting atom of a benzene ring directly or indirectly through the divalent group A,
the divalent group A is a group selected from -O-, -S-, $-NR^8-$, -CO-, -SO-, or $-SO_2-$ or is a group in which two or more selected from -O-, -S-, $-NR^8-$, -CO-, -SO-, or $-SO_2-$ are linked to each other,
$R^1$, $R^2$, $R^5$, $R^6$, $R^7$, and $R^8$ each independently represent a hydrogen atom, an alkyl group, an alkenyl group, an alkynyl group, an aryl group, or a heteroaryl group,
$R^3$ and $R^4$ each independently represent a halogen atom, an alkyl group, an alkenyl group, an alkynyl group, an aryl group, or a heteroaryl group, and
m and n each independently represent an integer of 0 to 2.

**19.** An organic semiconductor element comprising:
the organic semiconductor film according to claim 18.

FIG. 1

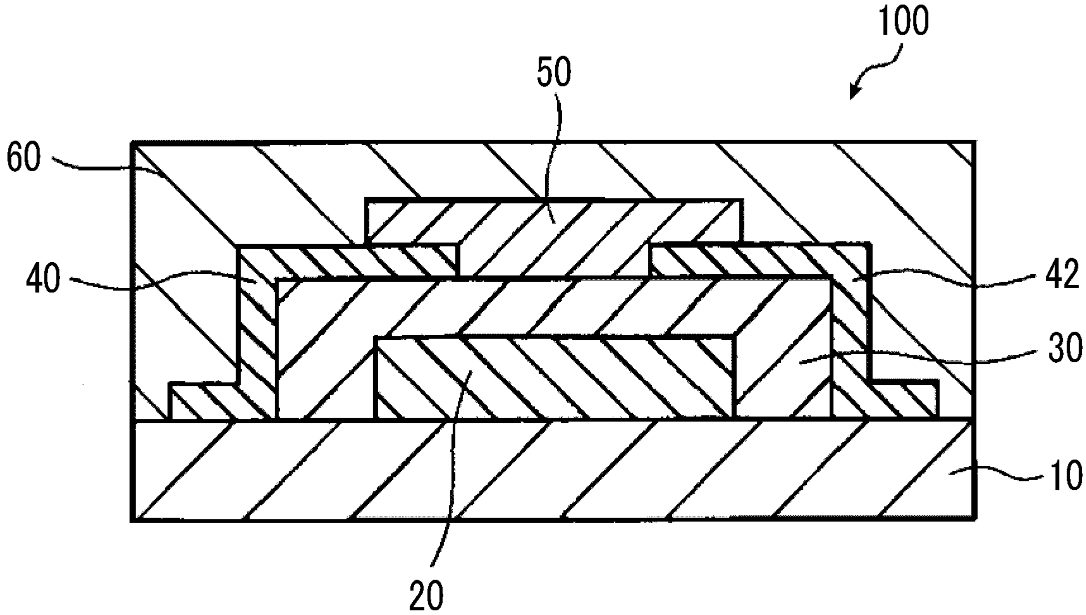


FIG. 2

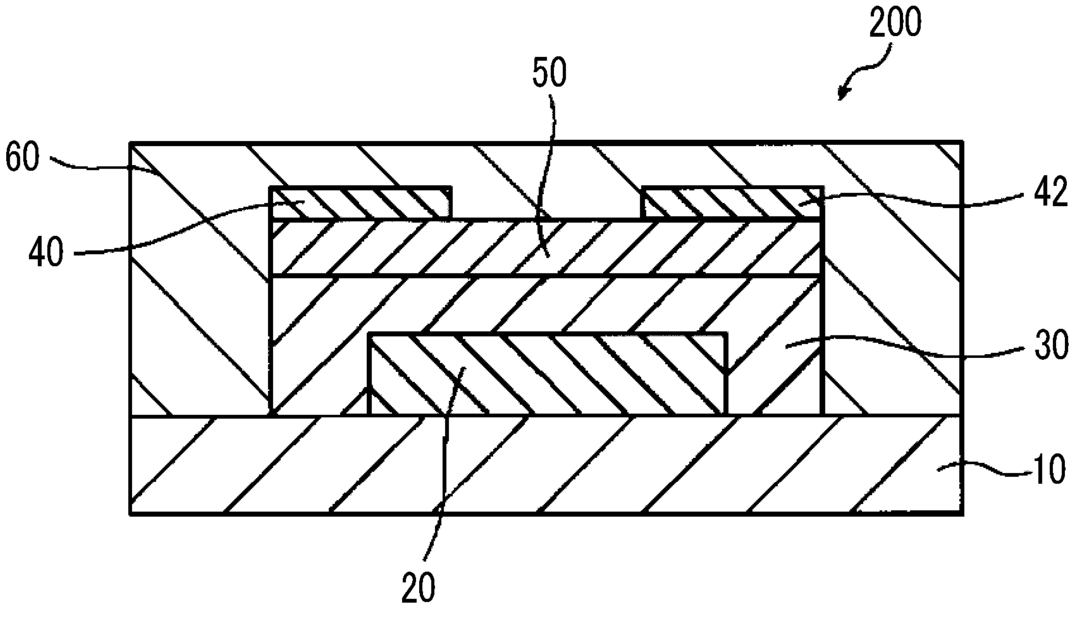

FIG. 3

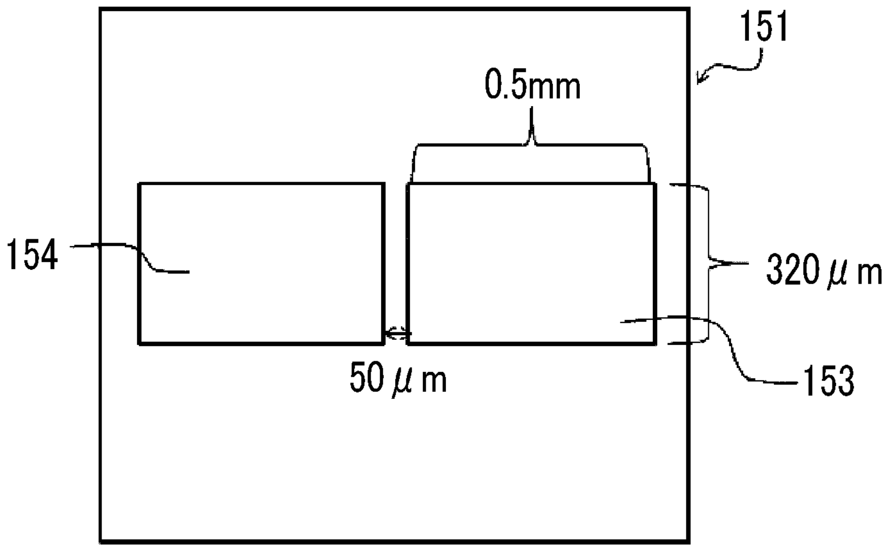

**INTERNATIONAL SEARCH REPORT**

International application No.
PCT/JP2017/033409

A. CLASSIFICATION OF SUBJECT MATTER
*H01L51/30*(2006.01)i, *H01L21/336*(2006.01)i, *H01L29/786*(2006.01)i, *H01L51/05*(2006.01)i, *H01L51/40*(2006.01)i

According to International Patent Classification (IPC) or to both national classification and IPC

B. FIELDS SEARCHED

Minimum documentation searched (classification system followed by classification symbols)
H01L51/30, H01L21/336, H01L29/786, H01L51/05, H01L51/40

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2015-195361 A (Fujifilm Corp., The University of Tokyo), 05 November 2015 (05.11.2015), paragraphs [0011] to [0125] & US 2017/0012220 A1 paragraphs [0079] to [0401] & WO 2015/147130 A1 & EP 3125298 A1 & CN 106165105 A | 1-19 |
| A | JP 2015-195362 A (Fujifilm Corp., The University of Tokyo), 05 November 2015 (05.11.2015), paragraphs [0014] to [0145] & US 2017/0018724 A1 paragraphs [0126] to [0436] & WO 2015/147129 A1 & EP 3125322 A1 & CN 106104833 A | 1-19 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 October 2017 (30.10.17) | 07 November 2017 (07.11.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2017/033409

C (Continuation). DOCUMENTS CONSIDERED TO BE RELEVANT

| Category* | Citation of document, with indication, where appropriate, of the relevant passages | Relevant to claim No. |
|---|---|---|
| A | WO 2016/143774 A1 (Fujifilm Corp.), 15 September 2016 (15.09.2016), paragraphs [0012] to [0197] & TW 201638192 A | 1-19 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015195361 A
- JP 2015195362 A
- JP 5089986 B
- JP 4545373 B
- JP 2011032268 A
- JP 2009054810 A
- JP 2011526588 A
- JP 2012209329 A
- JP 2013540697 A
- JP 2009218333 A
- US 20080142792 A
- WO 2014156773 A
- WO 2010098372 A
- JP 2010006794 A
- JP 2013214649 A
- JP 2011186069 A
- JP 2010285518 A
- JP 2012163946 A
- JP 2005354012 A
- JP 2006303465 A
- JP 2013207085 A
- WO 2014175351 A
- JP 2016191915 A


### Non-patent literature cited in the description


- *Scientific Report,* 2014, vol. 4, 5048
- *Adv. Mater.,* 2014, vol. 26, 4546
- **C. M. HANSEN.** Hansen Solubility Parameter: A User's Handbook. Taylor and Francis Group, 2007
- Hansen Solubility Parameter HSPiP